(19) Europäisches Patentamt
European Patent Office
Office européen des brevets

(11) **EP 2 004 578 B1**

(12) **EUROPÄISCHE PATENTSCHRIFT**

(45) Veröffentlichungstag und Bekanntmachung des Hinweises auf die Patenterteilung:
**03.09.2014 Patentblatt 2014/36**

(21) Anmeldenummer: **07727357.1**

(22) Anmeldetag: **26.03.2007**

(51) Int Cl.:
***C07C 5/333*** *(2006.01)*     ***C07C 11/06*** *(2006.01)*

(86) Internationale Anmeldenummer:
**PCT/EP2007/052884**

(87) Internationale Veröffentlichungsnummer:
**WO 2007/113162 (11.10.2007 Gazette 2007/41)**

(54) **VERFAHREN EINER HETEROGEN KATALYSIERTEN PARTIELLEN DEHYDRIERUNG WENIGSTENS EINES ZU DEHYDRIERENDEN KOHLENWASSERSTOFFS**

PROCESS FOR HETEROGENEOUSLY CATALYZED PARTIAL DEHYDROGENATION OF AT LEAST ONE HYDROCARBON TO BE DEHYDROGENATED

PROCÉDÉ DE DÉSHYDROGÉNATION PARTIELLE PAR CATALYSE HÉTÉROGÈNE D'AU MOINS UN HYDROCARBURE À DÉSHYDROGÉNER

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **30.03.2006 DE 102006015235
30.03.2006 US 787165 P
12.04.2006 DE 102006017623
12.04.2006 US 791207 P**

(43) Veröffentlichungstag der Anmeldung:
**24.12.2008 Patentblatt 2008/52**

(73) Patentinhaber: **BASF SE
67056 Ludwigshafen (DE)**

(72) Erfinder:
• HECHLER, Claus
  67063 Ludwigshafen (DE)
• RUPPEL, Wilhelm
  68163 Mannheim (DE)
• GERLINGER, Wolfgang
  67117 Limburgerhof (DE)
• SCHNEIDER, Wolfgang
  67098 Bad Dürkheim (DE)
• MÜLLER-ENGEL, Klaus Joachim
  76297 Stutensee (DE)

(56) Entgegenhaltungen:
**EP-B1- 0 799 169     WO-A-2004/074222**

EP 2 004 578 B1

**Beschreibung**

Vorliegende Erfindung betrifft ein Verfahren einer heterogen katalysierten partiellen Dehydrierung wenigstens eines zu dehydrierenden Kohlenwasserstoffs zu wenigstens einem dehydrierten Kohlenwasserstoff, bei dem man zum Zweck der heterogen katalysierten partiellen Dehydrierung des wenigstens einen zu dehydrierenden Kohlenwasserstoffs die Gesamtmenge eines molekularen Sauerstoff, molekularen Wasserstoff und den wenigstens einen zu dehydrierenden Kohlenwasserstoff enthaltenden Reaktionsgasgemischeingangsstroms mit der Maßgabe durch ein in einem Schacht mit vorgegebenem Querschnitt befindliches Katalysatorfestbett, das in Strömungsrichtung des Reaktionsgasgemischeingangsstroms zunächst eine Schüttung aus inerten Formkörpern und eine in Strömungsrichtung des Reaktionsgasgemischeingangsstroms sich an diese anschließende katalytisch aktive Schüttung mit wenigstens einem Katalysatorformkörper umfasst, die so beschaffen ist, dass sie im Reaktionsgasgemischeingangsstrom in Strömungsrichtung desselben wenigstens in ihrem Eingangsbereich für die Verbrennungsreaktion von molekularem Wasserstoff mit molekularem Sauerstoff zu Wasser und/oder für die Verbrennungsreaktion von im Reaktionsgasgemischeingangsstrom enthaltenem Kohlenwasserstoff mit molekularem Sauerstoff zu Kohlenoxiden und Wasser eine geringere Aktivierungsenergie bedingt, als für die Dehydrierung des wenigstens einen zu dehydrierenden Kohlenwasserstoffs zu dem wenigstens einen dehydrierten Kohlenwasserstoff, führt, dass eine Teilmenge (in der Regel wenigstens 1 mol-%, oder wenigstens 2 mol-%, oder wenigstens 3 mol-%, oder wenigstens 4 mol%, oder wenigstens 5 mol-%) des wenigstens einen zu dehydrierenden Kohlenwasserstoffs zu dem wenigstens einen dehydrierten Kohlenwasserstoff dehydriert wird, und dabei den Reaktionsgasgemischeingangsstrom im Schacht dadurch erzeugt, dass man einem im Schacht mit einem Volumenstrom V1 auf das Katalysatorfestbett zuströmenden, molekularen Wasserstoff und den wenigstens einen zu dehydrierenden Kohlenwasserstoff enthaltenden Eingangsgasstrom I vor dem Katalysatorfestbett ein molekularen Sauerstoff enthaltendes Eingangsgas II mit einem Gesamtvolumenstrom V2 zudosiert.

Der in dieser Anmeldung verwendete Begriff "dehydrierter Kohlenwasserstoff soll Kohlenwasserstoffe umfassen, deren Moleküle wenigstens zwei ("zwei" sind anwendungstechnisch bevorzugt) Wasserstoffatome weniger enthalten, als die Moleküle eines zu dehydrierenden Kohlenwasserstoffs. Im übrigen soll der Begriff Kohlenwasserstoff Stoffe umfassen, deren Moleküle nur aus den Elementen Kohlenstoff und Wasserstoff aufgebaut ist.

Damit umfassen dehydrierte Kohlenwasserstoffe insbesondere acyclische und cyclische aliphatische Kohlenwasserstoffe mit einer oder mehreren C,C-Doppelbindungen im Molekül.

Beispiele für solche aliphatische dehydrierte Kohlenwasserstoffe sind Propen, iso-Buten, Ethylen, 1-Buten, 2-Buten und Butadien. D.h., zu den dehydrierten Kohlenwasserstoffen gehören insbesondere die einfach ungesättigten linearen (n-Alkene) oder verzweigten aliphatischen Kohlenwasserstoffe (z. B. iso-Alkene), sowie die Cycloalkene. Ferner sollen die dehydrierten Kohlenwasserstoffe auch die Alkapolyene (z. B. Diene und Triene) umfassen, die mehr als eine Kohlenstoff-Kohlenstoff-Doppelbindung im Molekül enthalten. Dehydrierte Kohlenwasserstoffe sollen aber auch Kohlenwasserstoffverbindungen umfassen, die ausgehend von Alkylaromaten wie Ethylbenzol oder Isopropylbenzol durch Dehydrierung des Alkylsubstituenten erhältlich sind. Das sind z. B. Verbindungen wie Styrol oder $\alpha$-Methylstyrol.

Ganz generell bilden dehydrierte Kohlenwasserstoffe wertvolle Ausgangsverbindungen zur Synthese von z. B. funktionalisierten, radikalisch polymerisierbaren Verbindungen (z. B. Acrylsäure aus Propen oder Methacrylsäure aus iso-Buten) und deren Polymerisationsprodukten. Beispielsweise können solche funktionalisierten Verbindungen durch Partialoxidation von dehydrierten Kohlenwasserstoffen erzeugt werden. Dehydrierte Kohlenwasserstoffe eignen sich aber auch zur Herstellung von Verbindungen wie Methyl-tert.-butyl-ether (Folgeprodukt von iso-Buten, das z. B. als Kraftstoffadditiv zur Erhöhung der Oktanzahl geeignet ist). Dehydrierte Kohlenwasserstoffe können aber als solche auch selbst zur Polymerisation verwendet werden.

Als zu dehydrierende Kohlenwasserstoffe kommen in dieser Schrift insbesondere die acyclischen und cyclischen Alkane, aber auch Olefine (deren C,C-Doppelbindungszahl erhöht werden soll) in Betracht (als Beispiel sei die heterogen katalysierte partielle Dehydrierung von n-Butenen zu Butadien erwähnt).

D. h., der Begriff "zu dehydrierende Kohlenwasserstoffe" umfasst in dieser Schrift z. B. Kohlenwasserstoffe der Stöchiometrie $C_nH_{2n+2}$ mit n > 1 bis n ≤ 20, sowie der Stöchiometrie $C_nH_{2n}$ mit n > 1 bis n ≤ 20, sowie der Stöchiometrie $C_nH_{2n-2}$ mit n > 2 bis n ≤ 20, und n = ganzzahlig, insbesondere $C_2$- bis $C_{16}$-Alkane wie z. B. Ethan (zu Ethylen), Propan (zu Propylen), n-Butan, iso-Butan (zu iso-Buten), n-Pentan, iso-Pentan, n-Hexan, n-Heptan, n-Octan, n-Nonan, n-Decan, n-Undecan, n-Dodecan, n-Tridecan, n-Tetradecan, n-Pentadecan und n-Hexadecan.

Insbesondere aber gelten alle in dieser Schrift getroffenen Aussagen für $C_2$- bis $C_6$-Alkane als zu dehydrierende Kohlenwasserstoffe und ganz besonders für $C_2$- bis $C_4$-Kohlenwasserstoffe (insbesondere Alkane). D.h., zu dehydrierende Kohlenwasserstoffe sind in dieser Schrift vor allem Ethan, Propan, n-Butan und iso-Butan, aber auch 1-Buten und 2-Buten.

Unter einer heterogen katalysierten partiellen Dehydrierung eines Kohlenwasserstoffs soll in dieser Schrift eine (konventionelle) Dehydrierung verstanden werden, bei der wenigstens intermediär freier molekularer Wasserstoff gebildet wird und der Dehydrierschritt demzufolge endotherm verläuft (als Folgeschritt kann eine exotherme Wasserstoffverbrennung einbezogen sein). Im Unterschied dazu wird bei einer heterogen katalysierten partiellen Oxidehydrierung der dem zu dehydrierenden Kohlenwasserstoff zu entreißende Wasserstoff durch anwesenden Sauerstoff unmittelbar als Wasser ($H_2O$) entrissen. Der Dehydrierschritt einer heterogen katalysierten partiellen Oxidehydrierung verläuft daher grundsätzlich exotherm.

Des weiteren soll eine heterogen katalysierte partielle Dehydrierung in dieser Schrift eine Dehydrierung im Katalysatorfestbett sein.

In typischer Weise benötigt eine (konventionelle) heterogen katalysierte partielle Dehydrierung wenigstens eines zu dehydrierenden Kohlenwasserstoffs (z. B. von Propan) vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 800°C, bzw. 400 bis 700°C. Pro Molekül an z. B. zu Propylen dehydriertem Propan wird dabei ein Molekül Wasserstoff erzeugt. Hohe Temperaturen und Entfernung des Reaktionsproduktes $H_2$ begünstigen die Gleichgewichtslage im Sinne des wenigstens einen dehydrierten Kohlenwasserstoffs als Zielprodukt ebenso wie Partialdruckerniedrigung durch inerte Verdünnung. Dabei soll in dieser Schrift als Inertgas (inertes Verdünnungsgas) generell ein Reaktionsgasgemischbestandteil verstanden werden, der sich unter den Bedingungen der entsprechenden Reaktion im wesentlichen als chemisch inert verhält und - jeder inerte Reaktionsgasgemischbestanteil für sich betrachtet - zu mehr als 95 mol%, vorzugsweise zu mehr als 97 mol-% bzw. zu mehr als 99 mol-% chemisch unverändert erhalten bleibt. Beispiele für typische inerte Verdünnungsgase sind z. B. $N_2$, $H_2O$, $CO_2$, Edelgase wie He, Ne und Ar sowie Mischungen aus diesen Gasen etc..

Da bei einer heterogen katalysierten partiellen Dehydrierung wenigstens eines zu dehydrierenden Kohlenwasserstoffs (z. B. Propan) zu wenigstens einem dehydrierten Kohlenwasserstoff der Dehydrierschritt endotherm verläuft, muss die für die Einstellung der erforderlichen Reaktionstemperatur benötigte Wärme (Energie) entweder dem Reaktionsgas vorab und/oder im Verlauf der heterogen katalysierten Dehydrierung zugeführt werden.

In einfachster Weise lässt sich eine heterogen katalysierte partielle Dehydrierung in einem Schachtreaktor durchführen. Bei einem Schacht(reaktor) handelt es sich um einen Reaktionsraum, der von einer den Reaktionsraum berührenden materiellen Einhüllenden umschlossen ist, die wenigstens eine erste Öffnung zur Zufuhr eines Reaktionsgasgemischs in den Reaktionsraum und wenigstens eine zweite Öffnung zur Entnahme wenigstens eines Produktgasstroms aus dem Reaktionsraum aufweist. Im Reaktionsraum (im Schacht) befindet sich wenigstens ein Katalysatorfestbett, das vom Reaktionsgasgemisch durchströmt wird. Während der Verweilzeit im Katalysatorfestbett erfolgt die angestrebte partielle Dehydrierung des wenigstens einen zu dehydrierenden Kohlenwasserstoffs zu dem wenigstens einen dehydrierten Kohlenwasserstoff.

Angemessene Dehydrierumsätze werden bereits dann erreicht, wenn man den Schacht(reaktor) gegen seine Umgebung wärmeisoliert ((quasi)adiabat gestaltet), das Reaktionsgasgemisch auf eine Temperatur (Starttemperatur) von 350 bzw. 400 bis 800°C (häufig 500 bis 700°C, bzw. 550 bis 650°C) erhitzt und das Reaktionsgasgemisch in nur einem adiabaten Durchgang durch wenigstens ein im Reaktionsraum (im Schacht(reaktor)) befindliches Katalysatorfestbett führt.

Je nach Umsatz und gewählter inerter Verdünnung wird sich das Reaktionsgas beim einmaligen Durchgang durch das Katalysatorfestbett um etwa 30 bis 200°C abkühlen.

Eine Mitverwendung von Wasserdampf als inertem Verdünnungsgas im Reaktionsgasgemisch für eine heterogen katalysierte partielle Dehydrierung ist aus zwei Gründen vorteilhaft. Zum einen weist Wasserdampf eine vergleichsweise erhöhte molare Wärmekapazität auf, was im Rahmen einer Mitverwendung vorgenannte Abkühlung mindert.

Bei verminderter Abkühlung sind häufig auch verminderte Starttemperaturen für den angestrebten Umsatz ausreichend. Dies ist insofern vorteilhaft, als sich bei heterogen katalysierten partiellen Dehydrierungen wenigstens eines zu dehydrierenden Kohlenwasserstoffs mit zunehmender Starttemperatur über unerwünschte Nebenreaktionen in zunehmendem Umfang unter anderem in geringen Mengen schwersiedende hochmolekulare organische Verbindungen (thermische Zersetzungsprodukte), bis hin zu elementarem Kohlenstoff, bilden, die sich auf der Katalysatoroberfläche abscheiden und selbige dadurch deaktivieren. Sich dennoch auf der Katalysatoroberfläche abscheidender Kohlenstoff wird bei den erhöhten Temperaturen einer heterogen katalysierten partiellen Dehydrierung im übrigen bei einem Beisein von Wasserdampf als inertem Verdünnungsgas nach dem Prinzip der Kohlevergasung kontinuierlich wenigstens teilweise oder vollständig eliminiert.

In vorteilhafter Weise lässt sich eine heterogen katalysierte Kohlenwasserstoff-(z. B. Propan)-dehydrierung in einem (nach außen vorzugsweise adiabat gestalteten) Schachtreaktor dann betreiben (sowohl bei auf einen einmaligen Durchgang des Reaktionsgasgemischs durch den Reaktor bezogenen Dehydrierumsätzen von $\leq$ 30 mol-% als auch > 30 -mol-% (z. B. bis zu 40 mol-%, oder bis zu 50 mol-%, oder bis zu 60 mol-%)), wenn man ihn als Hordenreaktor gestaltet.

Ein solcher enthält räumlich aufeinanderfolgend mehr als ein die Dehydrierung katalysierendes Katalysatorfestbett im Schacht (umhüllten Reaktionsraum). Die Katalysatorbettenzahl kann z. B. 1 bis 20, zweckmäßig 2 bis 8, bzw. 3 bis 6 betragen. In der Regel sind die Katalysatorfestbetten dabei radial oder axial hintereinander angeordnet.

In besonders einfach realisierbarer Weise werden die Katalysatorfestbetten im Schacht entlang dessen in Strömungsrichtung des Reaktionsgases weisender Achse axial hintereinander angeordnet. Sie können aber auch in den Ringspalten von im Schacht zentrisch ineinandergestellten zylindrischen Gitterrosten angeordnet sein. Auch ist es möglich, die Ringspalte im Schacht in Segmenten übereinander anzuordnen und das Reaktionsgas nach radialem Durchtritt in einem Segment in das nächste darüber oder darunter liegende Segment zu führen.

Auf seinem Weg von einem Katalysatorfestbett zum nächsten Katalysatorfestbett kann das Reaktionsgas dann zum Beispiel durch Über- und/oder Durchleiten durch im Schacht zwischen den Festbetthorden angebrachte und mittels heißen Gasen und/oder Flüssigkeiten betriebene indirekte Wärmeaustauscher (z. B. Wärmeaustauscherrippen, oder Wärmeaustauscherplatten, oder Wärmeaustauscherrohrbündel) einer Zwischenerhitzung unterworfen werden (extern gelenkter Temperaturverlauf).

Ist der Schachtreaktor im übrigen adiabat gestaltet, ist es für auf einmaligen Durchgang des Reaktionsgases durch den Schachtreaktor bezogene Deyhdrierumsätze (z. B. Propan → Propylen-Umsätze) $\leq$ 40 mol-% (z. B. bei Verwendung der in der DE-A 10 2005 044 916 und in der DE-A 19937107 beschriebenen Katalysatoren, insbesondere den beispielhaft ausgeführten) in der Regel ausreichend, das Reaktionsgasgemisch auf eine Temperatur von 350 bzw. 400 bzw. 450 bis 550°C (bevorzugt 400 bis 500°C) vorerhitzt in den Schacht (den umhüllten, die Katalysatorfestbetten beherbergenden Reaktionsraum) zu führen und innerhalb des Schachtes, innerhalb des Hordenreaktionsraums, durch indirekten Wärmeaustausch (extern gelenkter Temperaturverlauf) wenigstens in diesem Temperaturbereich zu halten. Dies erweist sich auch für die Standzeit der Katalysatorfestbetten bis zu ihrer Regenerierung als besonders günstig.

Anwendungstechnisch geschickter ist es jedoch, die vorstehend geschilderte Zwischenerhitzung auf direktem Weg durchzuführen (intern gelenkter Temperaturverlauf). Dazu kann dem Reaktionsgasgemisch (dem Reaktionsgas) auf seinem Weg durch den Schacht z. B. nach Durchströmung des in Strömungsrichtung des Reaktionsgases ersten Katalysatorfestbetts und zwischen den in Strömungsrichtung des Reaktionsgases nachfolgenden Katalysatorfestbetten, vorteilhaft in begrenztem Umfang jeweils ein molekularen Sauerstoff enthaltendes Gas zugesetzt werden, wodurch ein molekularen Sauerstoff, molekularen Wasserstoff und wenigstens einen zu dehydrierenden Kohlenwasserstoff enthaltender Reaktionsgasgemischstrom erzeugt wird.

Gestaltet man die in Strömungsrichtung dieses Reaktionsgasgemischstroms nachfolgende katalytisch aktive Schüttung, die wenigstens einen Katalysatorformkörper umfasst (z. B. durch geeignete Auswahl der Aktivmasse), dann so, dass sie (im Reaktionsgasgemischstrom) in Strömungsrichtung des den molekularen Sauerstoff, molekularen Wasserstoff und den wenigstens einen zu dehydrierenden Kohlenwasserstoff enthaltenden Reaktionsgasgemischstroms wenigstens in ihrem Eingangsbereich für die Verbrennungsreaktion von molekularem Wasserstoff mit molekularem Sauerstoff zu Wasser und/oder für die Verbrennungsreaktion von im Reaktionsgasgemischstrom enthaltenem Kohlenwasserstoff zu Kohlenoxiden und Wasser eine geringere Aktivierungsenergie bedingt, als für die Dehydrierung des wenigstens einen zu dehydrierenden Kohlenwasserstoffs, wird so beim Durchgang des Reaktionsgasgemischs durch das Katalysatorfestbett im Katalysatorfestbett zunächst überwiegend eine begrenzte exotherme Verbrennung von im Reaktionsgasgemisch enthaltenem molekularem Wasserstoff und/oder von im Reaktionsgasgemisch enthaltenem Kohlenwasserstoff mit molekularem Sauerstoff (zu $H_2O$ bzw. $H_2O$ und Kohlenoxiden) erfolgen. Die dabei freigesetzte Reaktionswärme wärmt das Reaktionsgasgemisch auf und kann beim weiteren, überwiegend endotherm dehydrierend ausgeformten Durchgang des Reaktionsgasgemischs durch das Katalysatorfestbett, wieder verbraucht werden. Die resultierenden Verbrennungsprodukte wie $CO_2$, $H_2O$ sowie das den für die Verbrennung benötigten molekularen Sauerstoff gegebenenfalls begleitende $N_2$ bilden vorteilhafte inerte Verdünnungsgase.

Dem aus dem Katalysatorfestbett austretenden, zuvor gebildeten molekularen Wasserstoff enthaltenden, Reaktionsgasgemisch kann anschließend vorab seines Eintritts in das in Strömungsrichtung des Reaktionsgasgemischs nächste Katalysatorfestbett wieder in begrenztem Umfang ein molekularen Sauerstoff enthaltendes Gas zugesetzt werden etc..

Die Gewichtung zwischen "Wasserstoffverbrennung" und "Kohlenwasserstoffverbrennung" beim Eintritt des molekularen Sauerstoff, molekularen Wasserstoff und wenigstens einen zu dehydrierenden Kohlenwasserstoff enthaltenden Reaktionsgasgemischs in die katalytisch aktive Schüttung kann in erster Linie durch die Katalysatorwahl im Eingangsbereich (in Strömungsrichtung des Reaktionsgasgemischs) der katalytisch aktiven Schüttung beeinflusst werden. Als Katalysatoren, die vergleichsweise spezifisch (selektiv) die Verbrennung von molekularem Wasserstoff und/oder von Kohlenwasserstoff katalysieren, kommen z. B. jene der Schriften US-A 4,788,371,

US-A 4,886,928, US-A 5,430,209, US-A 5,530,171, US-A 5,527,979 und US-A 5,563,314 in Betracht.

Eine dominante "Wasserstoffverbrennung" ist gegenüber einer dominanten "Kohlenwasserstoffverbrennung" üblicherweise bevorzugt, da sie sowohl eine erhöhte Selektivität der Bildung der wenigstens einen dehydrierten Kohlenwasserstoffverbindung als auch einen erhöhten Dehydrierumsatz bezogen auf einen einmaligen Durchgang des Reaktionsgases durch den Hordenreaktor bedingt. Sie ist in der Regel dann gegeben, wenn die katalytisch aktive Schüttung als Katalysatoren nur Dehydrierkatalysatoren (insbesondere die in der DE-A 19 937 107 empfohlenen (insbesondere die beispielhaften Katalysatoren dieser DE-A) enthält, da diese in der Regel nicht nur die Dehydrierung des zu dehydrierenden Kohlenwasserstoffs (z. B. Propan) sondern auch die Verbrennung von molekularem Wasserstoff und von Kohlenwasserstoffen mit molekularem Sauerstoff zu katalysieren vermögen. Die Wasserstoffverbrennung verläuft dabei sowohl im Vergleich zur Dehydrierung des wenigstens einen zu dehydrierenden Kohlenwasserstoffs (z. B.

Propan) als auch im Vergleich zu z. B. dessen Verbrennung im Fall einer Konkurrenzsituation an diesen Katalysatoren sehr viel schneller (d.h., sie bedingen für die Verbrennung von molekularem Wasserstoff unter den gegebenen Bedingungen die mit Abstand niedrigste Aktivierungsenergie).

In Abhängigkeit vom Umfang der durchgeführten Verbrennungsreaktion (d.h., auch in Abhängigkeit von der zugeführten Menge an molekularem Sauerstoff), kann des Reaktionsgesamtverlauf beim Einmaldurchgang des Reaktionsgasgemischs durch den Hordenreaktor bezüglich der integralen Wärmetönung (d.h., bezüglich der Bruttowärmetönung) sowohl endotherm (negativ), oder autothem (im wesentlichen Null) oder exotherm (positiv) gestaltet werden.

Die Verbrennung von molekularem Wasserstoff liefert dabei etwa das Zweifache derjenigen Wärmemenge, die zu seiner Bildung im Rahmen der Dehydrierung verbraucht wird.

Selbstredend kann zwischen zwei Katalysatorfestbetten auch sowohl vom Prinzip der internen als auch vom Prinzip der externen Temperaturlenkung Gebrauch gemacht werden. Auch kann die Isothermie einer heterogen katalysierten partiellen Dehydrierung wenigstens eines Kohlenwasserstoffs dadurch weiter verbessert werden, dass man im Hordenreaktionsraum zwischen den Katalysatorfestbetten geschlossene, vor ihrer Füllung günstigerweise aber nicht notwendigerweise evakuierte, Einbauten (z. B. rohrförmige) anbringt. Derartige Einbauten können auch ins jeweilige Katalysatorfestbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen. Grundsätzlich können außerhalb der materiellen Einhüllenden des Schachtreaktors zusätzlich fluide (gasförmig und/oder flüssig) Wärmeträger geführt werden, um die Isothermie weiter zu verbessern. Der anwendungstechnische Aufwand ist jedoch erheblich, weshalb die nach außen adiabate Ausgestaltung in der Regel bevorzugt wird.

Selbstverständlich kann die beschriebene Maßnahme der internen Temperaturlenkung auch angewendet werden, um das in Strömungsrichtung des Reaktionsgasgemischs dem ersten Katalysatorfestbett zugeführte Reaktionsgasgemisch auf die erforderliche Reaktionstemperatur zu erwärmen. Zu diesem Zweck kann dem in den Schachtreaktor geführten, den wenigstens einen zu dehydrierenden Kohlenwasserstoff enthaltenden Reaktionsgasgemisch gegebenenfalls zielgerichtet vorab molekularer Wasserstoff aus einer anderen Quelle zugeführt werden. Auf eine solche Wasserstoffzugabe kann aber auch verzichtet werden. Die interne Temperaturlenkung kann dann im wesentlichen nur durch Kohlenwasserstoffverbrennung erfolgen. Häufig kann das dem ersten Katalysatorfestbett zugeführte Reaktionsgasgemisch auch auf andere Art und Weise auf Reaktionstemperatur gebracht werden. Beispielsweise können die Ausgangsgasströme, aus denen sich das dem ersten Katalysatorfestbett zugeführte Reaktionsgasgemisch konstituiert, bereits entsprechende Temperaturen aufweisen. Auch können diese Ausgangsgasströme bereits molekularen Sauerstoff enthalten, so dass sich das Problem der Zudosierung eines molekularen Sauerstoff enthaltenden Gases zu einem im Schacht auf das in Strömungsrichtung erste Katalysatorfestbett zuströmenden Gasgemisch nicht stellt.

Grundsätzlich sind Verfahren einer heterogen katalysierten partiellen Dehydrierung wenigstens eines zu dehydrierenden Kohlenwasserstoffs zu wenigstens einem dehydrierten Kohlenwasserstoff in einem zu einem Festbett-Hordenreaktor gestalteten Schachtreaktor mit intern gelenktem Temperaturverlauf bekannt (vgl. z. B. DE-A 102005061626, DE-A 102005057197, DE-A 102005052923, DE-A 102005052917, DE-A 102005022798, DE-A 102005009885, DE-A 102005010111, DE-A 102004032129, DE-A 102005013039, WO 03/076370, DE-A 10 211 275, WO 01/96270 und der in diesen Schriften zitierte Stand der Technik).

Es ist aus den vorgenannten Schriften auch bekannt, das einzelne Katalysatorfestbett des Hordenreaktors so zu gestalten, dass man die eigentliche katalytisch aktive Schüttung, die wenigstens einen katalytisch aktiven Katalysatorformkörper umfasst, in Strömungsrichtung des Reaktionsgasgemischstroms mit einer Schüttung aus inerten Formkörpern abdeckt und dem auf ein solches Katalysatorfestbett zuströmenden, molekularen Wasserstoff und den wenigstens einen zu dehydrierenden Kohlenwasserstoff enthaltenden Gasstrom das für die interne Temperaturlenkung erforderliche, molekularen Sauerstoff enthaltende Gas zuzudosieren, bevor dieser Gasstrom das Katalysatorfestbett (d. h., die die eigentliche katalytisch aktive Schüttung abdeckende Inertschüttung (Schüttung aus inerten Formkörpern)) erreicht hat.

Der gewürdigte Stand der Technik lässt jedoch völlig offen, auf weiche Art und Weise (d. h., wie) die Zudosierung des molekularen Sauerstoff enthaltenden Gasstroms großtechnisch vorzunehmen (auszuführen) ist.

Die Art und Weise der Zufuhr des molekularen Sauerstoff enthaltenden Gasstroms zum im Schacht auf das relevante Katalysatorfestbett zuströmenden, molekularen Wasserstoff und wenigstens einen zu dehydrierenden Kohlenwasserstoff enthaltenden Gasstrom ist gemäß eigenen Untersuchungsergebnissen jedoch für großtechnische Verfahrensdurchführungen aus verschiedenen Gründen relevant.

Zum einen kommt es dort, wo die Zudosierung des molekularen Sauerstoff enthaltenden Gases erfolgt, zu lokal erhöhten Sauerstoffkonzentrationen. In ungünstigen Fällen können diese so geartet sein, dass lokal zündfähige (explosionsfähige) Gasgemische entstehen, was anwendungstechnisch unerwünscht ist, weshalb deren Existenz allenfalls kurzzeitig und lokal eng begrenzt sein soll. Das nach der Zudosierung resultierende Reaktionsgasgemisch weist darüber hinaus bereits vorab der angestrebten heterogen katalysierten Verbrennung eine erhöhte Temperatur auf (die im Verlauf der heterogenen katalysierten Verbrennung zusätzlich erhöht wird). Bevor das molekularen Sauerstoff enthaltende Reaktionsgasgemisch das Katalysatorfestbett erreicht, kommt es deshalb innerhalb des Reaktionsgasgemischs normalerweise zu unerwünschten homogenen radikalischen Reaktionen (z. B. unerwünschte exotherme homogene radikalische Partialoxidationen und/oder Oxidehydrierungen von Kohlenwasserstoff; die von diesen unerwünschten Reaktionen lokal entwickelten Reaktionswärmen bewirken lokale Temperaturerhöhungen, deren Folge üblicherweise eine Zunahme an unerwünschten thermischen Zersetzungen von Kohlenwasserstoffen ist), mit einem breiten Nebenproduktspektrum, die die Zielproduktselektivität mindern und in der Regel auch die Katalysatorstandzeit verkürzen. Eine solchermaßen geminderte Zielproduktselektivität ist insbesondere dann von Nachteil, wenn das wenigstens eine Zielprodukt, der wenigstens eine dehydrierte Kohlenwasserstoff, einer Folgereaktion (z. B. einer heterogen katalysierten Partialoxidation) unterworfen werden soll. Innerhalb des Katalysatorfestbetts sind solche unerwünschten radikalischen Nebenreaktionen weitgehend unterdrückt, da die große innere Oberfläche der Festbettschüttung als Radikalfänger fungiert. Das Ausmaß solcher unerwünschter radikalischer Reaktionen nimmt mit der verfügbaren Reaktionszeit in der Regel exponentiell zu.

Aus diesem Grund wurde auch schon vorgeschlagen, die Zudosierung des molekularen Sauerstoff enthaltenden Gases ausschließlich unmittelbar in die die katalytisch aktive Schüttung abdeckende Inertschüttung hinein vorzunehmen. Eine solche Vorgehensweise ist jedoch insofern nachteilig, als bei einer Verteilung des molekularen Sauerstoff enthaltenden Gases in die Inertschüttung hinein ein zentral gespeistes Verteilungssystem (z. B. ein Leitungssystem mit Austrittsöffnungen) in der Inertschüttung lokal auf im wesentlichen zufällig verteilte Verteilungswiderstände trifft (je nachdem, in welcher Weise und ob bzw. in welchem Umfang ein inerter Formkörper die jeweilige Austrittsöffnung des Leitungssystems versperrt). Dies führt in unerwünschter Weise zu lokal gegebenenfalls vergleichsweise unterschiedlichen Zufuhrströmen an dem den molekularen Sauerstoff enthaltenden Gas. Im ungünstigsten Fall kann dies im Fall einer auf die je Zeiteinheit zugeführte Gesamtmenge an dem molekularen Sauerstoff enthaltenden Gas geregelten Zufuhr dazu führen, dass an einigen Austrittsöffnungen kein molekularen Sauerstoff enthaltendes Gas aus der Austrittsöffnung aus - sondern molekularen Wasserstoff und wenigstens einen zu dehydrierenden Kohlenwasserstoff enthaltendes Gas in die Austrittsöffnung eintritt. Außerdem können die inerten Formkörper die Strömungsrichtung der aus den Austrittsöffnungen austretenden individuellen Zudosierungsströme ungünstig beeinflussen und im Extremfall die lokale Zudosierung sogar aus dem Katalysatorfestbett herausführen.

Tritt das molekulare Sauerstoff zudosiert enthaltende Reaktionsgasgemisch in die in Strömungsrichtung hinter der Inertschüttung befindliche eigentliche katalytisch aktive Schüttung ein, bedingt eine inhomogene Verteilung des molekularen Sauerstoff im Reaktionsgasgemisch in der katalytisch aktiven Schüttung lokale katalysierte Verbrennungen von unterschiedlichem Ausmaß. Diese sind aber von entsprechend unterschiedlichen lokalen Wärmeentwicklungen begleitet. Lokal überhöhte Temperaturen bedingen normalerweise aber eine beschleunigte lokale Alterung der katalytisch aktiven Schüttung. Mit einer in Strömungsrichtung des Reaktionsgasgemischs möglichst ausgedehnten Inertschüttung lässt sich zwar eine am Eintritt des Reaktionsgasgemischs in die katalytisch aktive Schüttung in selbigem im wesentlichen homogene Verteilung des molekularen Sauerstoffs erzielen, doch geht mit einer zunehmenden Ausdehnung der Inertschüttung ein zunehmender Druckverlust des Reaktionsgasgemischs beim Durchgang durch selbige einher, was letztlich eine erhöhte Verdichterleistung erfordert. Auch können mit zunehmender Verweilzeit des Reaktionsgases in der Inertschüttung wieder unerwünschte Nebenreaktionen zum tragen kommen. Dies gilt insbesondere dann, wenn die innere Oberfläche der Inertschüttung eine vergleichsweise beschränkte ist.

Aufgabe der vorliegenden Erfindung ist daher ein wie in der Präambel dieser Schrift beschriebenes Verfahren, das den vorgenannten Gesichtspunkten möglichst umfassend und in möglichst ökonomischer, d.h., einfacher und wenig aufwendiger Weise Rechnung trägt, und eine Minimierung der Ausdehnung der relevanten Inertschüttung ermöglicht. Die Lehre der US-A 2,584,391 vermag die gestellte Aufgabe nicht zu lösen, da sie auf ein Verfahren im katalytischen Wirbelbett gerichtet ist.

Die Lehre der DE-A 102 004 024 957 vermag die gestellte Aufgabe ebenfalls nicht zu lösen. Zum einen ist sie auf eine heterogen katalysierte Oxidehydrierung gerichtet, bei der die Zufuhr des molekularen Sauerstoff enthaltenden Reaktionsgasgemischs unmittelbar in die katalytisch aktive Schüttung hinein erfolgt. Dies zudem mit Diskontinuitäten im Zufuhrsystem. Darüber hinaus bedarf sie eines vergleichsweise aufwändig zu fertigenden Rohrbündelsystems mit kostspieligen Rohrböden.

Die WO 2004/074 222 vermag bei der Lösung der gestellten Aufgabe gleichfalls nicht behilflich zu sein. So fordert diese, dass das den molekularen Sauerstoff enthaltende Gas und der den wenigstens einen zu dehydrierenden Kohlenwasserstoff enthaltende Gasstrom in streng paralleler Stromführung zueinander geführt werden sollen, wozu es einer vergleichsweise komplexen Vorrichtung bedarf. Zusätzlich bedarf eine parallele Stromzusammenführung einer vergleichsweise langen Mischstrecke.

Die DE-A 102 004 003 070 vermag zur Lösung der gestellten Aufgabe ebenfalls nicht beizutragen, da sie auf eine heterogen katalysierte Oxidehydrierung gerichtet ist, bei der das Reaktionsgasgemisch unmittelbar auf die katalytisch aktive Schüttung geführt wird. Die Lehre der EP-A 799169 vermag auch nicht zu befriedigen, da sie eine Verengung des Reaktorquerschnitts erfordert.

Als Lösung der im Stand der Technik nicht gelösten Aufgabe wird daher ein Verfahren einer heterogen katalysierten partiellen Dehydrierung wenigstens eines zu dehydrierenden Kohlenwasserstoffs zu wenigstens einem dehydrierten Kohlenwasserstoff, bei dem man zum Zweck der heterogen katalysierten partiellen Dehydrierung des wenigstens einen zu dehydrierenden Kohlenwasserstoffs die Gesamtmenge eines molekularen Sauerstoff, molekularen Wasserstoff und den wenigstens einen zu dehydrierenden Kohlenwasserstoff enthaltenden Reaktionsgasgemischeingangsstroms mit der Maßgabe durch ein in einem Schacht mit vorgegebenem Querschnitt befindliches Katalysatorfestbett, das in Strömungsrichtung des Reaktionsgasgemischeingangsstroms zunächst eine Schüttung aus inerten Formkörpern und eine in Strömungsrichtung des Reaktionsgasgemischstroms sich an diese anschließende katalytisch aktive Schüttung mit wenigstens einem Katalysatorformkörper umfasst, die so beschaffen ist, dass sie im Reaktionsgasgemischeingangsstrom in Strömungsrichtung desselben (des Reaktionsgasgemischeingangsstroms) wenigstens in ihrem Eingangsbereich für die Verbrennungsreaktion von molekularem Wasserstoff mit molekularem Sauerstoff zu Wasser und/oder für die Verbrennungsreaktion von im Reaktionsgasgemischeingangsstrom enthaltenem Kohlenwasserstoff mit molekularem Sauerstoff zu Kohlenoxiden und Wasser eine geringere Aktivierungsenergie bedingt, als für die Dehydrierung des wenigstens einen zu dehydrierenden Kohlenwasserstoffs zu dem wenigstens einen dehydrierten Kohlenwasserstoff, führt, dass wenigstens eine Teilmenge (in der Regel wenigstens 1 mol-%, oder wenigstens 2 mol-%, oder wenigstens 3 mol-%, oder wenigstens 4 mol-%, oder wenigstens 5 mol-%) des wenigstens einen zu dehydrierenden Kohlenwasserstoffs zu dem wenigstens einen dehydrierten Kohlenwasserstoff dehydriert wird, und dabei den Reaktionsgasgemischeingangsstrom im Schacht dadurch erzeugt, dass man einem im Schacht mit dem Volumenstrom V1 auf das Katalysatorfestbett zuströmenden, molekularen Wasserstoff und den wenigstens einen zu dehydrierenden Kohlenwasserstoff enthaltenden Eingangsgasstrom I vor dem Katalysatorfestbett ein molekularen Sauerstoff enthaltendes Eingangsgas II mit einem Gesamtvolumenstrom V2 zudosiert, zur Verfügung gestellt, das dadurch gekennzeichnet ist, dass das Eingangsgas II in Form von aus einer Vielzahl von in Strömungsrichtung des Eingangsgasstroms II vor dem Katalysatorfestbett befindlichen Austrittsöffnungen A eines Leitungssystems ausströmenden Eingangsgas II-strömen so zudosiert wird, dass

a) die Richtungen der Mehrzahl M aller aus den Austrittsöffnungen A in fiktiver Abwesenheit des Eingangsgasstroms I austretenden Eingangsgas II-ströme mit der Strömungsrichtung des Eingangsgasstroms I einen Winkel $\alpha$ von 90 $\pm$ 60° einschließen;

b) die Entfernung D der Mehrzahl M aller Austrittsöffnungen A vom Katalysatorfestbett, bezogen auf die Strömungsgeschwindigkeit W des Eingangsgasstroms I im Schacht, kleiner oder gleich wie die Induktionszeit J des Reaktionsgaseingangsgemischs (dies ist das Gasgemisch, das den Reaktionsgasgemischeingangsstrom konstituiert) multipliziert mit 2·W ist;

c) bei einer Projektion der Schwerpunkte der Mehrzahl M aller Austrittsöffnungen A in Strömungsrichtung des Eingangsgasstroms I in die Projektionsebene E senkrecht zur Strömungsrichtung des Eingangsgasstroms I innerhalb der Projektionsebene E für wenigstens 75 % (vorzugsweise für wenigstens 80 %, besser für wenigstens 85 %, oder für wenigstens 90 %, vorteilhaft für wenigstens 95 % oder noch besser für 100 %) der vom Eingangsgasstrom I erfassten Projektionsfläche die Anzahl ZA der in einem beliebigen $m^2$ befindlichen Austrittsöffnungsschwerpunkte $\geq$ 10 beträgt;

d) die aus den zu der Anzahl ZA von Austrittsöffnungsschwerpunkten gehörigen Austrittsöffnungen A austretenden individuellen Eingangsgas II-ströme (z. B. Volumenströme oder Massenströme) um nicht mehr als 50 % von ihrem Zahlenmittelwert abweichen (mit dem Zahlenmittelwert als Bezugsbasis);

e) innerhalb der Anzahl ZA von Austrittsöffnungen der Abstand d von einem Austrittsöffnungsschwerpunkt zum (in der Projektionsebene E) nächstliegenden Austrittsöffnungsschwerpunkt nicht mehr als $2\sqrt{1 m^2 / ZA}$ beträgt, und

f) das Verhältnis V1 : V2 $\geq$ 8 beträgt.

Grundsätzlich kann das Eingangsgas II (z. B. im Fall von Luft) beim erfindungsgemäßen Verfahren mit z. B. 20 °C (Außentemperatur) zudosiert werden.

Erfindungsgemäß zweckmäßig (dadurch wird über die Austrittsöffnungen A eine gleichmäßigere Gasdichte gewährleistet) sollte der Temperaturunterschied $\Delta T_{II}^{I}$ (als Betrag) zwischen der Temperatur des Eingangsgasstroms I und des Eingangsgases II jedoch nicht größer als 300 °C sein. Vorzugsweise ist $\Delta T_{II}^{I}$ nicht größer als 250 °C, besonders bevorzugt nicht größer als 200 °C, mit Vorteil nicht größer als 150 °C, mit besonderem Vorteil nicht größer als 100 °C, ganz besonders bevorzugt nicht größer als 75 °C, mit ganz besonderem Vorteil nicht größer als 50 °C, besser nicht größer als 30 °C und am besten $\leq$ 20 °C, oder $\leq$ 10 °C bzw. 0 °C.

Sind die beiden Temperaturen nicht identisch, weist der Eingangsstrom I in der Regel die höhere Temperatur auf.

Das Vorgenannte gilt insbesondere dann, wenn der Eingangsstrom I eine Temperatur von ≥ 350 °C (z. B. ≥ 375 °C, oder ≥ 400 °C, oder ≥ 425 °C, oder ≥ 450 °C, oder ≥ 475 °C, oder ≥ 500 °C, oder ≥ 525 °C, oder ≥ 550 °C, oder ≥ 600 °C) aufweist. In der Regel wird diese Temperatur ≤ 800 °C, häufig ≤ 700 °C betragen.

Das beim erfindungsgemäßen Verfahren im Schacht befindliche Katalysatorfestbett kann das einzige in einem Schachtreaktor befindliche Katalysatorfestbett oder eines von mehreren Katalysatorbetten in einem als Hordenreaktor ausgestalteten Schachtreaktor sein. Handelt es sich um das in einem Schachtreaktor in Strömungsrichtung des Reaktionsgases erste Katalysatorfestbett, wird der Reaktionsgasgemischeingangsstrom in der Regel noch keinen dehydrierten Kohlenwasserstoff enthalten. Handelt es sich beim Katalysatorfestbett des erfindungsgemäßen Verfahrens jedoch um ein in Strömungsrichtung des Reaktionsgases diesem ersten Katalysatorfestbett nachfolgenden Katalysatorfestbett, wird der Reaktionsgasgemischeingangsstrom (das ist der in dieses Katalysatorfestbett hineingeführte Gasstrom) normalerweise jedoch auch von dem wenigstens einen dehydrierten Kohlenwasserstoff enthalten. Auch kann das Katalysatorfestbett beim erfindungsgemäßen Verfahren das in einem Hordenreaktor in Strömungsrichtung des Reaktionsgases letzte Katalysatorfestbett sein.

D. h., grundsätzlich kann das erfindungsgemäße Verfahren auf alle in einem Hordenreaktor befindlichen Katalysatorfestbetten angewendet werden.

Das einem in Strömungsrichtung des Reaktionsgases in einem Schachtreaktor befindlichen ersten Katalysatorfestbett zugeführte Reaktionsgaseingangsgemisch kann jedoch aus unterschiedlichen Gründen bereits von dem wenigstens einen dehydrierten Kohlenwasserstoff enthalten. Dies z. B. dann, wenn gemäß der in der WO 03/076 370 bzw. DE-A 102 112 75 beschriebenen Verfahrensweise der aus dem Dehydrierreaktor austretende Produktgasstrom in zwei Teilmengen identischer Zusammensetzung aufgeteilt und eine der beiden Teilmengen als Dehydrierkreisgas in das dem Dehydrierreaktor zugeführte Reaktionsgasgemisch rückgeführt und die andere Teilmenge anderweitig weiterverwendet (z. B. zum Zweck einer heterogen katalysierten partiellen Oxidation von im Reaktionsraum gebildetem dehydriertem Kohlenwasserstoff) wird.

Das Vorstehende gilt in entsprechender Weise auch für den Eingangsgasstrom I, der gemeinsam mit dem molekularen Sauerstoff enthaltenden Eingangsgas II beim erfindungsgemäßen Verfahren den dem Katalysatorfestbett zugeführten Reaktionsgasgemischeingangsstrom konstituiert.

Als Eingangsgas II kommt für das erfindungsgemäße Verfahren prinzipiell sowohl molekularer Sauerstoff in Reinform, als auch ein Gemisch aus molekularem Sauerstoff und Inertgas (z. B. Luft) in Betracht. Als solche Inertgase können z. B. molekularer Stickstoff, Kohlendioxid, Edelgase und/oder Wasserdampf verwendet werden.

Anwendungstechnisch zweckmäßig wird das Eingangsgas II ein Gemisch aus Luft und Wasserdampf sein. Die Verwendung von Gemischen aus molekularem Sauerstoff und Inertgas als Eingangsgas II ist gegenüber einer Verwendung von reinem molekularem Sauerstoff als Eingangsgas II insofern vorteilhaft, als dadurch im Bereich der Austrittsöffnungen A, beim Eintritt des Eingangsgases II in den Eingangsgasstrom I das Explosionsverhalten günstiger gestaltet wird. Darüber hinaus ermöglicht eine Mitverwendung von Inertgas im Eingangsgas II größere Gesamtvolumenströme V2, was sich für die Stabilität (Gleichförmigkeit) der Zudosierung von Eingangsgas II in den Eingangsgasstrom I als vorteilhaft erweist. Sind jedoch erhöhte Inertgasanteile im Produktgasstrom einer heterogen katalysierten partiellen Dehydrierung wenigstens eines Kohlenwasserstoffs unerwünscht (z. B., wenn sich diese bei der Folgeverwendung des wenigstens einen dehydrierten Kohlenwasserstoffs als unter Aufwand zu fördernder Ballast erweisen), so wird man deren Anteil im Eingangsgas II minimieren.

Für das erfindungsgemäße Verfahren geeignete Eingangsgase II können z. B. folgende Gehalte aufweisen:

| | |
|---|---|
| 0 bis 80 Vol.-% | Wasserdampf, |
| 10 bis 97 Vol.-% | $N_2$, und |
| 3 bis 25 Vol.-% | $O_2$. |

Mögliche Eingangsgas II-Gehalte können damit sein:

| | |
|---|---|
| 15 bis 80 Vol.-% | $H_2O$, |
| 20 bis 85 Vol.-% | $N_2$, und |
| 5 bis 25 Vol.-% | $O_2$; |

oder

| | |
|---|---|
| 15 bis 60 Vol.-% | $H_2O$, |
| 20 bis 80 Vol.-% | $N_2$, und |
| 5 bis 20 Vol.-% | $O_2$. |

Das Verhältnis des Volumenstroms V1 (in $Nm^3/h$; $Nm^3$ sind das Volumen in $m^3$ die die entsprechende Gasmenge bei Normalbedingungen (0°C, 1 atm) einnehmen würde) zum Gesamtvolumenstrom V2 (ebenfalls in $Nm^3/h$) beträgt beim erfindungsgemäßen Verfahren $\geq 8$. Vorzugsweise beträgt es $\geq 10$, besonders bevorzugt $\geq 15$ und ganz besonders bevorzugt $\geq 20$. Häufig beträgt das Verhältnis V1 : V2 beim erfindungsgemäßen Verfahren auch $\geq 25$ oder $\geq 30$, bzw. $\geq 40$ oder $\geq 50$. In der Regel wird das Verhältnis V1 : V2 beim erfindungsgemäßen Verfahren jedoch $\leq 500$, häufig $\leq 400$ oder $\leq 300$, vielfach auch $\leq 200$ oder $\leq 100$ betragen.

In der Regel weist das Katalysatorfestbett beim erfindungsgemäßen Verfahren in Strömungsrichtung des Reaktionsgasgemischs nicht nur eine Deckschicht aus Inertmaterial, sondern auch eine Abschlussschicht aus inerten Formkörpern auf. Dies rührt daher, dass z. B. im Fall eines axialen Hordenreaktors das Katalysatorfestbett in der Regel von einem gasdurchlässigen Gitterrost getragen wird, dessen Maschenweite häufig eine größere Ausdehnung aufweist als die Katalysatorformkörper.

Handelt es sich beim erfindungsgemäßen Verfahren bei dem im Schacht befindlichen Katalysatorfestbett um das in einem Schachtreaktor in Strömungsrichtung des Eingangsgasstroms I erste (und gegebenenfalls einzige) Katalysatorfestbett, entspricht der im Schacht mit dem Volumenstrom V1 strömende Eingangsgasstrom I normalerweise dem dem Schachtreaktor zum Zweck der heterogen katalysierten Dehydrierung des wenigstens einen zu dehydrierenden Kohlenwasserstoff zugeführten Eingangsgasstrom. Dieser enthält in der Regel $\geq 5$ -Vol.-% des wenigstens einen zu dehydrierenden Kohlenwasserstoff (z. B. Propan). Daneben kann er z. B. enthalten:

a) $N_2$;
b) $N_2$ und $H_2O$;
c) $N_2$, $H_2O$ und $H_2$;
d) $N_2$, $H_2O$, $CO_2$ und $H_2$; oder
e) $N_2$, $H_2O$, CO, $CO_2$ und $H_2$.

Zusätzlich kann er auch schon begrenzte Mengen an molekularem Sauerstoff enthalten.

Handelt es sich beim Katalysatorfestbett des erfindungsgemäßen Verfahrens jedoch um ein in Strömungsrichtung des Reaktionsgases diesem ersten Katalysatorfestbett nachfolgenden Katalysatorfestbett, wird der Eingangsgasstrom I normalerweise dem aus dem in Strömungsrichtung des Reaktionsgases vorhergehenden Katalysatorfestbett austretenden Reaktionsgasgemisch entsprechen, das im Normalfall zu den vorgenannten Gasen zusätzlich wenigstens einen dehydrierten Kohlenwasserstoff enthalten wird. In der Regel wird er dann im wesentlichen keinen molekularen Sauerstoff enthalten.

Unter der Mehrzahl M aller Austrittsöffnungen A (und der aus diesen austretenden individuellen Eingangsgas II-ströme) sollen in dieser Schrift diejenigen Austrittsöffnungen A (und die aus ihnen austretenden individuellen Eingangsgas II-ströme) verstanden werden, aus denen insgesamt gerade mehr als 50 % (vorzugsweise gerade mehr als 60 bzw. 70 %, besonders bevorzugt gerade mehr als 80 bzw. 90 % und ganz besonders bevorzugt 100 %) des Gesamtvolumenstroms V2 mit der Maßgabe austreten, dass die aus ihnen austretenden individuellen Eingangsgas II-ströme innerhalb der Gesamtmenge aller individuellen Eingangsgas II-ströme keinen umfassen, der kleiner ist, als der größte der nicht zu dieser Mehrzahl M gehörigen individuellen Eingangsgas II-ströme.

Erfindungsgemäß bevorzugt sind die aus den Austrittsöffnungen A austretenden individuellen Eingangsgas II-ströme im Rahmen des,fertigungstechnisch Machbaren möglichst gleich groß.

Prinzipiell ist die Geometrie der Austrittsöffnungen A beim erfindungsgemäßen Verfahren beliebig. D. h., es kommen sowohl vieleckige (z. B. dreieckige, viereckige, fünfeckige, sechseckige etc.) als auch runde (z. B. eliptische oder kreisrunde) Austrittsöffnungen A in Betracht. Vorzugsweise ist die Geometrie aller Austrittsöffnungen A beim erfindungsgemäßen Verfahren einheitlich beschaffen (einschließlich ihrer Größe). Runde Austrittsöffnungen A ("Löcher") sind erfindungsgemäß bevorzugt. Die Längstausdehnung L (im Fall von kreisrunden Austrittsöffnungen ihr Durchmesser) einer Austrittsöffnung A (das ist die längste direkte Verbindung zweier auf der Umrisslinie der jeweiligen Austrittsöffnung A liegender Punkte) beträgt beim erfindungsgemäßen Verfahren vorteilhaft $\geq 0,1$ mm bis $\leq 5$ cm. D.h., mögliche Werte für L sind $\geq 0,2$ mm bis $\leq 4$ cm, sowie $\geq 0,3$ mm bis $\leq 3$ cm, oder $\geq 0,4$ mm bis $\leq 2$ cm, bzw. $\geq 0,5$ mm bis $\leq 1$ cm, vielfach $\geq 1$ mm bis $\leq 5$ mm.

Vorzugsweise liegen die (Querschnitts)Flächen der Austrittsöffnungen A dabei in den Bereichen $(\pi \cdot L^2)/2$, wobei für L die Grenzen der vorgenannten Bereiche einzusetzen sind.

Ist das Verhältnis R, gebildet aus der Längstausdehnung L einer Austrittsöffnung A* und der Kleinstausdehnung K dieser Austrittsöffnung A* (das ist die kürzeste durch den Schwerpunkt der Austrittsöffnung A* führende direkte Verbindung zweier auf der Umrisslinie der Austrittsöffnung A* liegender Punkte), d. h., R = L : K, $\geq 5$ (normalerweise ist R < 5 und $\geq 1$), dann soll diese Austrittsöffnung A* (z. B., wenn die Austrittsöffnung A* ein Schlitz ist) für alle Belange der vorliegenden Erfindung gedanklich durch eine Anzahl n von einheitlichen virtuellen kreisrunden Austrittsöffnungen A ersetzt werden, die sich wie folgt errechnet:

$$n = \frac{U^2}{4\Pi \cdot F}$$

mit

$\pi$ = das Verhältnis des Kreisumfangs zum Kreisdurchmesser,
U = Umfang der Austrittsöffnung A*, und
F = Austrittsfläche der Austrittsöffnung A*.

Ist n keine ganze Zahl, so wird auf die nächstliegende ganze Zahl gerundet (im Fall von "1,50" und seinen Analoga wird aufgerundet).

Der Durchmesser $d_h$ der vorgenannten virtuellen Austrittsöffnungen A errechnet sich wie folgt:

$$d_h = \frac{4 \cdot F}{U}$$

Er wird als hydraulischer Durchmesser der Austrittsöffnung A* bezeichnet.

Die Schwerpunkte dieser virtuellen kreisrunden Austrittsöffnungen A sollen sich auf einer Geraden G befinden, deren Länge der Längstausdehnung L entspricht und die so durch den Schwerpunkt der Austrittsöffnung A* führt, dass dieser Schwerpunkt die Länge der Geraden G halbiert und wenigstens die verlängerte Gerade G die Umrisslinie der Austrittsöffnung A* zweimal schneidet und die Gerade G mit der Strömungsrichtung des Eingangsstroms I einen rechten Winkel einschließt. Auf dieser Geraden G sind die Schwerpunkte der n virtuellen Austrittsöffnungen gleichmäßig zu verteilen, wobei der Schwerpunkt der Austrittsöffnung A* selbst den Mittelpunkt (das Zentrum, den Schwerpunkt) dieser Verteilung bildet.

Der Wortlaut "in fiktiver Abwesenheit" des Eingangsgasstroms I bringt zum Ausdruck, dass ein beim erfindungsgemäßen Verfahren aus einer Austrittsöffnung A austretender (individueller) Eingangsgas II-strom beim Zusammentreffen mit dem Eingangsgasstrom I in seiner Richtung verändert wird. Zur Festlegung des Winkels $\alpha$ wird deshalb die Richtung des aus der jeweiligen Austrittsöffnung A austretenden (individuellen) Eingangsgas II-ströms herangezogen, die vorliegen würde, wenn unter ansonsten unveränderten Verfahrensbedingungen der Eingangsgasstrom I unterdrückt würde.

Erfindungsgemäß vorteilhaft beträgt für die Mehrzahl M aller Austrittsöffnungen A (bzw. der aus ihnen austretenden Eingangsgas II-ströme) der Winkel $\alpha$ 90 $\pm$ 50°, vorzugsweise 90 $\pm$ 40°, besonders bevorzugt 90 $\pm$ 30°, besser 90 $\pm$ 20°, ganz besonders bevorzugt 90 $\pm$ 10°, noch besser 90 $\pm$ 5° und am besten in allen Fällen 90°. Die Richtung des aus der Austrittsöffnung austretenden (individuellen) Eingangsgas II-stroms, soll dabei die Richtung des austretenden zentralen Strahls sein.

Erfindungsgemäß sollen sich die Austrittsöffnungen A vor dem Katalysatorfestbett befinden. D.h., die Austrittsöffnungen A sollen nicht von inerten Formkörpern versperrt werden. Vielmehr soll ein vom Schwerpunkt der Austrittsöffnung A senkrecht zur Strömungsrichtung des Eingangsstroms I gezogener Strahl in zweckmäßiger Weise wenigstens auf einer Strahllänge von zwei (vorzugsweise wenigstens vier, besonders bevorzugt wenigstens sechs und ganz besonders bevorzugt wenigstens acht) hydraulischen Durchmessern $d_h$ der Austrittsöffnung A ($d_h$ = 4·F/U) und am besten auf beliebiger Strahllänge keinen inerten Formkörper berühren. Im übrigen ist es erfindungsgemäß vorteilhaft, wenn sich die Austrittsöffnungen A möglichst nahe am Katalysatorfestbett befinden.

Die Entfernung D einer Austrittsöffnung A vom Katalysatorfestbett soll in dieser Schrift die kürzeste direkte Verbindungslinie vom Schwerpunkt der Austrittsöffnung bis zu einem Berührungspunkt mit einem inerten Formkörper in der inerten Deckschüttung des Katalysatorfestbetts sein.

Erfindungsgemäß sollen die Entfernungen D der Mehrzahl M aller Austrittsöffnungen A vom Katalysatorfestbett, bezogen auf die Strömungsgeschwindigkeit W (in m/s) des Eingangsgasstroms I (= Volumenstrom des Eingangsgasstroms I, dividiert durch die von diesem Volumenstrom durchströmte engste Querschnittsfläche (dies ist die Fläche in einer senkrecht zum Eingangsgasstrom I liegenden Ebene, die vom Eingangsgasstrom I erfasst wird und durch die der Eingangsgasstrom I hindurchströmt, mit der Maßgabe, dass in vorgenannter Ebene Schwerpunkte von Austrittsöffnungen A liegen)) im Schacht, kleiner oder gleich wie die Induktionszeit J (in s) des Reaktionsgaseingangsgemischs multipliziert mit 2·W sein. D.h., für die Mehrzahl M aller Austrittsöffnungen A soll erfüllt sein: D $\leq$ 2·W . J.

Unter der Induktionszeit J soll dabei folgendes verstanden werden.

Überlässt man ein Induktionsgasgemisch, das den gleichen Gehalt an molekularem Sauerstoff und an zu dehydrierendem Kohlenwasserstoff aufweist (jeweils in Vol-%), wie der Reaktionsgasgemischeingangsstrom, und das dadurch erzeugt wird, dass man dem zu dehydrierenden Kohlenwasserstoff (oder dessen Gemisch mit molekularem Stickstoff) als Ausgangsgas 1 den molekularen Sauerstoff als Ausgangsgas 2 in Form von Luft (oder als reiner molekularer Sauerstoff bzw. als dessen Gemisch mit molekularem Stickstoff) zugibt (d. h., das Induktionsgasgemisch enthält als von molekularem Sauerstoff und zu dehydrierendem Kohlenwasserstoff verschiedene Gase lediglich molekularen Stickstoff), und das sich bei der selben Temperatur $T^R$ und dem selben Druck $P^R$ wie der Reaktionsgasgemischeingangsstrom befindet, sich selbst, so nimmt der in diesem Induktionsgasgemisch vorhandene Sauerstoffgehalt ($O_2$) als Funktion der Zeit ab. Die Induktionszeit J des Reaktionsgasgemischeingangsstroms ist dabei diejenige Zeit, innerhalb der der Sauerstoffgehalt des zugehörigen Induktionsgasgemischs auf 70 % seines Ausgangsgehaltes (als Absolutmenge) gesunken ist.

Die experimentelle Bestimmung der Induktionszeit J ist in einfacher Weise wie folgt möglich.

Eine aus Quarzglas gefertigte Y-Mischdüse (Rohrinnendurchmesser: 0,6 cm; Wanddicke: 1 mm; Winkel der Gabelung = 90°; die beiden Äste der Gabelung sind gewendelt und weisen jeder für sich eine gestreckte Länge von 2 m auf; das die Gabelung tragende Quarzrohr der Y-Mischdüse weist eine Länge von 0,5 m auf) wird in einen Ofen (z. B. ein Strahlungsofen oder ein Umluftofen) eingebracht, in welchem sie auf die Temperatur $T^R$ erwärmt wird. In den einen der beiden Äste der Gabelung wird das Ausgangsgas 1 und in den anderen der beiden Äste der Gabelung wird das Ausgangsgas 2 zugeführt. Am Ende des die Gabelung tragenden Quarzrohres der Y-Mischdüse befindet sich ein Reduzierventil, das beim Druck $P^R$ öffnet. Die Volumenströme der beiden Ausgangsgase werden relativ zueinander so bemessen, dass sich hinter der Gabelung die gewünschte Induktionsgasgemischzusammensetzung bildet. Die absoluten Volumenströme werden so eingestellt, dass der Sauerstoffgehalt des Induktionsgasgemischs beim Austritt aus dem Trägerrohr der Y-Mischdüse nur noch 70 % seines errechenbaren Ausgangsgehaltes beträgt. Aus der Strömungsgeschwindigkeit und der Länge des die Gabelung tragenden Quarzrohres ergibt sich J als der Quotient der Länge durch die Strömungsgeschwindigkeit. In der Regel wird der Volumenstrom des Induktionsgasgemischs im Trägerrohr der Y-Mischdüse dabei < 10 m$^3$/h betragen. Die Ermittlung des Sauerstoffgehaltes des Induktionsgasgemischstroms am Austritt aus dem Trägerrohr der Y-Mischdüse kann mittels einer Lambdasonde ($\lambda$-Sonde) erfolgen. Diese kann als Nernstsonde ausgeprägt sein. Dabei handelt es sich um ein keramisches Sensorelement, dessen eine Seite dem Induktionsgasgemischstrom und dessen andere Seite einer Sauerstoffreferenz ausgesetzt wird. Beispielsweise kann als solche Referenz Umgebungsluft verwendet werden. Bei den erhöhten Temperaturen $T^R$ wird die üblicherweise verwendete Yttrium-dotierte Zirkondioxid-Keramik der Sonde für negative Sauerstoff-Ionen leitend. Der Konzentrationsunterschied an molekularem Sauerstoff in den beiden Gasen erzeugt eine Ionendiffusion in der Sonde. Dadurch lässt sich zwischen innen und außen an der Sonde angebrachten Platin-Elektroden eine elektrische Spannung abnehmen, die Sondenspannung. Diese Spannung wird dabei durch die Nernst-Gleichung beschrieben und ist ein unmittelbares Maß für den Sauerstoffgehalt des Induktionsgasgemischstroms am Austritt aus dem Trägerrohr der Y-Mischdüse.

Alternativ kann eine Lambdawiderstandssonde eingesetzt werden. Hier besteht das Sensorelement in der Regel aus einer halbleitenden Titandioxidkeramik. Die Ladungsträger werden durch Sauerstofffehlstellen, die als Donatoren wirken, zur Verfügung gestellt. Bei umgebendem Sauerstoff werden die Fehlstellen besetzt und die Zahl der freien Ladungsträger reduziert. Die Sauerstoffionen tragen hier nicht wesentlich zur Leitfähigkeit bei, sondern der Sauerstoff reduziert die Zahl der freien Ladungsträger. Bei hoher Sauerstoffkonzentration hat das Sensormaterial einen großen Widerstand. Das Signal wird durch einen Spannungsteiler mit einem festen Widerstand erzeugt.

Hersteller von Lambdasonden sind z. B. die Robert Bosch GmbH, sowie die Firmen Denso und NGK.

Unter dem Schwerpunkt einer Austrittsöffnung soll dabei in dieser Schrift der gedachte Massenschwerpunkt der Austrittsöffnung verstanden werden (bei homogener Massenausfüllung).

Erfindungsgemäß bevorzugt soll für die Mehrzahl M aller Austrittsöffnungen A die Bedingung D ≤ 1 W·J, besser D ≤ 0,5 · W · J erfüllt sein. Ganz besonders bevorzugt soll für die Mehrzahl aller Austrittsöffnungen A die Bedingung D ≤ 0,2 · W · J gültig sein. Noch besser ist es, wenn für die Mehrzahl M aller Austrittsöffnungen A die Bedingung D ≤ 0,05 · W · J erfüllt ist. Ganz besonders vorteilhaft erfüllen stets alle Austrittsöffnungen A die relevante Bedingung. Ferner ist es erfindungsgemäß günstig, wenn alle Austrittsöffnungen A für D einen einheitlichen Wert aufweisen.

Es müssen aber nicht in notwendiger Weise alle Austrittsöffnungen A einen einheitlichen Wert für D aufweisen.

Projeziert man jedoch die Schwerpunkte der Mehrzahl M aller Austrittsöffnungen A in Strömungsrichtung des Eingangsgasstroms I in die Projektionsebene E senkrecht zur Strömungsrichtung des Eingangsgasstroms I, muss innerhalb der Projektionsebene E für wenigstens 75 % (vorzugsweise für wenigstens 80 %, besser für wenigstens 85 %, noch besser für wenigstens 90 %, bevorzugt für wenigstens 95 %, und besonders bevorzugt für 100 %) der vom Eingangsgasstrom I erfassten Projektionsfläche die Anzahl ZA der in einem beliebigen m$^2$ befindlichen Austrittsöffnungsschwerpunkte erfindungsgemäß ≥ 10 betragen. Erfindungsgemäß vorteilhaft beträgt vorgenanntes ZA ≥ 20 oder ≥ 30, bevorzugt ≥ 40 oder ≥ 50, besonders bevorzugt ≥ 60 oder ≥ 70, ganz besonders bevorzugt ≥ 80 oder ≥ 90 bzw. ≥ 100. Erfindungsgemäß vorteilhaft wird ZA groß gewählt (mit einem zunehmenden ZA gehen in der Regel abnehmende Längstausdehnungen L der Austrittsöffnungen A einher). Prinzipiell kann ZA beim erfindungsgemäßen Verfahren bis zu 100 000 und mehr betragen. Aus Gründen einer wirtschaftlichen Fertigung wird ZA in der Regel jedoch meistens ≤ 10000, häufig ≤ 1000 betragen. Innerhalb der Anzahl ZA von Austrittsöffnungen soll der Abstand d von einem Austrittsöffnungsschwerpunkt zum (in der Projektionsebene E) nächstliegenden Austrittöffnungsschwerpunkt nicht mehr als

$$2\sqrt{1m^2/ZA}$$ betragen.

D. h., erfindungsgemäß kann d nicht mehr als $1,75\sqrt{1m^2/ZA}$, oder nicht mehr als $1,5\sqrt{1m^2/ZA}$, oder nicht mehr als $1,25\sqrt{1m^2/ZA}$ betragen.

Selbstverständlich kann die Anzahl ZA der Austrittsöffnungsschwerpunkte über die vom Eingangsgasstrom I in der Projektionsebene E erfasste Projektionsfläche auch gleich verteilt ($d = \sqrt{1m^2/ZA}$) sein.

Ferner ist es erfindungsgemäß günstig, wenn sich keine Austrittsöffnungen einander gegenüberstehen, bei denen die aus diesen Austrittsöffnungen A austretenden Eingangsgas-II-ströme zueinander entgegengesetzt gerichtet sind.

Weiterhin ist es erfindungsgemäß wesentlich, dass die aus den zu der Anzahl ZA von Austrittsöffnungsschwerpunkten gehörigen Austrittsöffnungen A austretenden individuellen Eingangsgas II-(z. B. Volumen oder Masse-)ströme um nicht mehr als 50 % (vorzugsweise um nicht mehr als 40 %, besonders bevorzugt um nicht mehr als 30 %, ganz besonders bevorzugt um nicht mehr als 20 % bzw. um nicht mehr als 10 %, noch besser um nicht mehr als 5 % und am besten überhaupt nicht) von ihrem Zahlenmittelwert (gemittelt über die Anzahl ZA) abweichen. Dabei ist der Zahlenmittelwert die Bezugsbasis.

Erfindungsgemäß vorteilhaft weist der Schacht beim erfindungsgemäßen Verfahren eine (nicht schiefe) gerade zylindrische Geometrie auf (zwei deckungsgleiche (kongruente) Grundflächen, die in parallelen Ebenen liegen und deren Punkte durch parallele, auf den beiden Grundflächen senkrecht stehende Strecken verbunden sind). Innerhalb der zylindrischen Geometrien sind dabei der elliptische Zylinder und vor allem der Kreiszylinder bevorzugt.

Selbstredend macht vom erfindungsgemäßen Verfahren auch derjenige Gebrauch, der zusätzlich zur erfindungsgemäßen Eindüsung eines molekularen Sauerstoff enthaltenden Gases vorab des Katalysatorfestbetts noch eine Zudosierung eines molekularen Sauerstoff enthaltenden Gases in die inerte Deckschüttung hinein vornimmt.

Ein weiteres erfindungsgemäßes Erfordernis besteht darin, dass, bezogen auf den einmaligen Durchgang des Reaktionsgasgemischeingangsstroms durch das Katalysatorfestbett, eine Teilmenge, in der Regel wenigstens 1 mol-%, oder wenigstens 2 mol-%, oder wenigstens 3 mol-%, oder wenigstens 4 mol-%, oder wenigstens 5 mol-% (vorzugsweise wenigstens 7 mol-%, besonders bevorzugt wenigstens 9 mol-% und ganz besonders bevorzugt wenigstens 11 mol-%), des im Reaktionsgasgemischeingangsstrom enthaltenen wenigstens einen zu dehydrierenden Kohlenwasserstoff zu dem wenigstens einen dehydrierten Kohlenwasserstoff dehydriert wird. Normalerweise wird der vorgenannte molare Prozentsatz jedoch ≤ 20 mol-% betragen.

Generell wird der Reaktionsgasgemischeingangsstrom in der Regel wenigstens 5 Vol.-% an dem wenigstens einen zu dehydrierenden Kohlenwasserstoff enthalten. Häufig liegt dieser Volumenanteil bei entsprechend bezogenen Werten von ≥ 10 Vol.-%, oft ≥ 15 Vol.-% und meist ≥ 20 Vol.-% bzw. ≥ 25 Vol.-%, oder ≥ 30 Vol.-%. In der Regel liegt dieser Volumenanteil jedoch bei in gleicher Weise bezogenen Werten von ≤ 90 Vol.-%, meist ≤ 80 Vol.-% und oft ≤ 70 Vol.-%. Vorstehende Angaben gelten (wie alle anderen Angaben in dieser Schrift) insbesondere im Fall von Propan als zu dehydrierendem Kohlenwasserstoff und Propylen als dehydriertem Kohlenwasserstoff. Selbstredend treffen sie aber auch dann zu, wenn iso-Butan der zu dehydrierende Kohlenwasserstoff und iso-Buten der dehydrierte Kohlenwasserstoff ist.

In der Regel wird beim erfindungsgemäßen Verfahren die dem Eingangsgasstrom I zudosierte Menge an Eingangsgas II so bemessen, dass das resultierende Reaktionsgaseingangsgemisch, bezogen auf die darin enthaltene Menge an zu dehydrierendem Kohlenwasserstoff und dehydriertem Kohlenwasserstoff (z. B. Propan und Propylen), 0,01 bzw. 0,5 bis 30 Vol.-% an molekularem Sauerstoff enthält.

Erfindungsgemäß günstig beträgt der molare Gehalt des Reaktionsgasgemischeingangsstroms an molekularem Sauerstoff nicht mehr als 50 mol-% der darin enthaltenen molaren Menge an molekularem Wasserstoff.

Im Regelfall beträgt der molare Gehalt des Reaktionsgasgemischeingangsstroms an molekularem Sauerstoff, bezogen auf die darin enthaltene molare Menge an molekularem Wasserstoff, 10 bis 40 mol-%, häufig 15 bis 35 mol-% und vielfach 20 bis 30 mol-%.

Erfindungsgemäß vorteilhaft wird beim erfindungsgemäßen Verfahren beim Durchgang des Reaktionsgasgemischeingangsstroms durch das Katalysatorfestbett die Gesamtmenge (bzw. wenigstens 95 mol-%) des im Reaktionsgasgemischeingangsstrom enthaltenen molekularen Sauerstoff zur Verbrennung des im Reaktionsgasgemischeingangsstrom enthaltenen molekularen Wasserstoff verbraucht.

Anwendungstechnisch zweckmäßig wird beim erfindungsgemäßen Verfahren beim Durchgang des Reaktionsgasgemischeingangsstroms durch das Katalysatorfestbett etwa die Hälfte der Menge an molekularem Wasserstoff mit molekularem Sauerstoff verbrannt, die, im Fall eines Hordenreaktors, im zuvor durchströmten Katalysatorfestbett gebildet worden ist.

Der Vordruck des Reaktionsgasgemischeingangsstroms wird beim Eintritt desselben in das Katalysatorfestbett beim erfindungsgemäßen Verfahren in der Regel 1,5 bis 6 bar, häufig 2 bis 5 bar und vielfach 3 bis 4 bar betragen.

Die Temperatur des Reaktionsgasgemischeingangsstroms wird beim Eintritt desselben in das Katalysatorfestbett beim erfindungsgemäßen Verfahren in der Regel 300 bis 700° C, häufig 350 bis 600° C und vielfach 400 bis 500° C betragen.

Unter der Belastung eines einen Reaktionsschritt katalysierenden Katalysatorfestbetts mit Reaktionsgas soll in dieser Schrift ganz generell die Menge an Reaktionsgas in Normlitern (= Nl; das Volumen in Litern, das die entsprechende Reaktionsgasmenge bei Normalbedingungen (0° C, 1 atm) einnehmen würde) verstanden werden, die pro Stunde durch einen Liter Katalysatorfestbett geführt wird. Die Belastung kann aber auch nur auf einen Bestandteil des Reaktionsgases bezogen sein.

Dann ist es die Menge dieses Bestandteils in Nl/l•h, die pro Stunde durch einen Liter des Katalysatorfestbetts geführt wird (reine Inertmaterialschüttungen werden nicht zu einem Katalysatorfestbett gerechnet). Die Belastung kann auch nur auf die in einem Katalysatorfestbett, das den eigentlichen Katalysator mit Inertmaterial verdünnt enthält, enthaltene Menge an Katalysator bezogen sein (dies wird dann explizit vermerkt).

Grundsätzlich kann das erfindungsgemäße Verfahren bei Belastungen des Katalysatorfestbetts (bezogen auf die darin enthaltene Katalysatorgesamtmenge) sowohl mit Reaktionsgas als auch mit dem darin enthaltenen wenigstens einen zu dehydrierenden Kohlenwasserstoff (z. B. Propan) von 100 bis 10000 $h^{-1}$ (Nl/l•h, verkürzt: $h^{-1}$), häufig 300 bis 5000 $h^{-1}$, das heißt, vielfach 500 bis 3000 $h^{-1}$ (im wesentlichen unabhängig vom gewünschten Umsatz an dem wenigstens einen zu dehydrierenden Kohlenwasserstoff) betrieben werden.

In der Regel wird beim erfindungsgemäßen Verfahren der Eingangsgasstrom I das im Schacht befindliche Katalysatorfestbett im wesentlichen vertikal anströmen. D. h., der Einschlusswinkel $\beta$ zwischen der Strömungsrichtung des Eingangsgasstroms I im Schacht und einer auf der Anströmfläche des Katalysatorfestbetts abgelegten Ebene beträgt normalerweise 90 $\pm$ 30°, vorzugsweise 90 $\pm$ 20°, besonders bevorzugt 90 $\pm$ 10°, ganz besonders bevorzugt 90 $\pm$ 5° und am Besten 90°.

Normalerweise beträgt die Anströmfläche des Katalysatorfestbetts im Schacht beim erfindungsgemäßen Verfahren $\geq$ 1 $m^2$. Vielfach auch $\geq$ 2 $m^2$, oder $\geq$ 3 $m^2$, oder $\geq$ 4 $m^2$ bzw. $\geq$ 5 $m^2$. Selbstredend kann vorgenannte Anströmfläche auch $\geq$ 10 $m^2$, oder $\geq$ 20 $m^2$, oder $\geq$ 30 $m^2$ betragen. In world scale Analgen kann vorgenannte Anströmfläche aber auch $\geq$ 40 $m^2$ oder $\geq$ 50 $m^2$ betragen. Entsprechende Anströmflächen von $\geq$ 100 $m^2$ bilden eher die Ausnahme.

Als Katalysatoren kommen für die katalytisch aktive Schüttung grundsätzlich alle im Stand der Technik für heterogen katalysierte Dehydrierungen bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen (z. B. Zirkondioxid, Aluminiumoxid, Siliziumdioxid, Titandioxid, Magnesiumoxid, Lanthanoxid und/oder Ceroxid), Träger abgeschiedenen, in der Regel vergleichsweise edlen, Metall (z. B. Platin) bestehen. Unter anderem können damit alle Dehydrierkatalysatoren eingesetzt werden, die in der WO 01/96270, der EP-A 731 077, der DE-A 102 11 275, der DE-A 101 31 297, der WO 99/46039, der US-A 4,788,371, der EP-A 705 136, der WO 99/29420, der US-A 4,220,091, der US-A 5,430,220, der US-A 5,877,369, der EP-A 117 146, der DE-A 199 37 196, der DE-A 199 37 105, der US-A 3,670,044, der US-A 6,566,573 und der WO 94/29021 empfohlen werden. Dabei können diese Katalysatoren beim erfindungsgemäßen Verfahren aus den eingangs bereits angeführten Gründen die alleinigen Katalysatoren in der katalytisch aktiven Schüttung des erfindungsgemäßen Verfahrens sein. Grundsätzlich kann die katalytisch aktive Schüttung ausschließlich aus katalytisch aktiven Formkörpern bestehen. Selbstredend kann die katalytisch aktive Schüttung aber auch aus mit inerten Formkörpern verdünnten katalytisch aktiven Formkörpern bestehen. Solche inerten Formkörper könne z. B. aus gebrannten Tonen (Aluminiumsilicaten) oder Steatit (z. B. C 220 der Fa. CeramTec), oder sonstigen (vorzugsweise im wesentlichen an Poren freien) Hochtemperaturkeramikmaterialien wie Aluminiumoxiden, Siliciumdioxid, Titandioxid, Magnesiumoxid, Lanthanoxid, Ceroxid, Zinkaluminiummischoxid, Thoriumdioxid, Zirkondioxid, Siliciumcarbid oder sonstigen Silicaten wie Magnesiumsilicat und Mischungen aus vorgenannten Materialien gefertigt sein. Vorgenannte Materialien kommen auch für die inerte Deck- und gegebenenfalls Abschlussschüttung des Katalysatorfestbetts des erfindungsgemäßen Verfahrens in Betracht.

Mit Vorteil sollte die inerte Deckschüttung des Katalysatorfestbetts des erfindungsgemäßen Verfahrens so beschaffen sein, dass im Reaktionsgasgemischeingangsstrom beim Durchströmen dieser Inertschüttung $\leq$ 35 mol-% (vorzugsweise $\leq$ 30 mol-%, besonders bevorzugt $\leq$ 25 mol-%, besser $\leq$ 20 mol-%, mit besonderem Vorteil $\leq$ 15 mol-%, noch besser $\leq$ 10 mol-%, und ganz besonders bevorzugt $\leq$ 5 mol-% oder 0 mol-%) des darin enthaltenen molekularen Sauerstoff umgesetzt werden.

Im besonderen können für das erfindungsgemäße Verfahren als (z. B. alleinige) Katalysatoren für die katalytisch aktive Schüttung des Katalysatorfestbetts die Katalysatoren gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Alumiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

In einer bevorzugten Ausführungsform enthalten vorgenannte Dehydrierkatalysatoren wenigstens ein Element der VIII. Nebengruppe, mindestens ein Element der I. und II. Hauptgruppe, mindestens ein Element der III. und/oder IV. Hauptgruppe und mindestens ein Element der III. Nebengruppe einschließlich Lanthaniden und Actiniden. Als Element der VIII. Nebengruppe enthält die Aktivmasse der Dehydrierkatalysatoren bevorzugt Platin und/oder Palladium, besonders bevorzugt Platin. Als Elemente der I. und II. Hauptgruppe enthält die Aktivmasse der vorgenannten Dehydrierkatalysatoren bevorzugt Kalium und/oder Cäsium. Als Elemente der III. Nebengruppe einschließlich der Lanthaniden und Actiniden enthält die Aktivmasse der vorgenannten Dehydrierkatalysatoren bevorzugt Lanthan und/oder Cer. Als Elemente der III. und/oder IV. Hauptgruppe enthält die Aktivmasse der vorgenannten Dehydrierkatalysatoren bevorzugt ein oder mehrere Elemente aus der Gruppe bestehend aus Bor, Gallium, Silizium, Germanium, Zinn und Blei, besonders bevorzugt Zinn. Ganz besonders bevorzugt enthält die Aktivmasse der vorgenannten Dehydrierkatalysatoren jeweils wenigstens einen Vertreter der vorgenannten Elementgruppen.

Generell kann es sich bei den Dehydrierkatalysatoren um Katalysatorstränge (Durchmesser typisch 0,1 bzw. 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln.

Grundsätzlich bestehen weder hinsichtlich der Katalysatorgeometrie (insbesondere im Fall von Trägerkatalysatoren) noch hinsichtlich der Geometrie der inerten Formkörper Einschränkungen. Besonders häufige Geometrien sind Vollzylinder, Hohlzylinder (Ringe), Kugeln, Kegel, Pyramiden und Würfel sowie Stränge, Wagenräder, Sterne und Monolithe.

Die Längstausdehnung der Katalysatorformkörper sowie der Inertformkörper (längste direkte Verbindungslinie zweier auf der Formkörperoberfläche befindlicher Punkte) kann dabei 0,5 mm bis 100 mm, oft 1,5 mm bis 80 mm, und vielfach 3 mm bis 50 mm bzw. bis 20 mm betragen).

Schließlich seien auch noch die Katalysatoren der deutschen Anmeldung Nr. 10 2005 044 916 als (z. B. alleinige Katalysatoren) für die katalytisch aktive Schüttung des Katalysatorfestbetts des erfindungsgemäßen Verfahrens besonders geeignete Katalysatoren ausgeführt.

Im Rahmen des erfindungsgemäßen Verfahrens auf der Katalysatoroberfläche abgeschiedene schwersiedende hochmolekulare organische Verbindungen, bis hin zum Kohlenstoff, die die Katalysatoren deaktivieren, können dadurch beseitigt und die Katalysatoren so regeneriert werden, dass man das Katalysatorfestbett von Zeit zu Zeit bei erhöhter Temperatur mit einem Sauerstoff enthaltenden Gas durchströmt und damit die abgeschiedenen Substanzen quasi abbrennt.

Nach längerer Betriebsdauer sind die in dieser Schrift empfohlenen Katalysatoren normalerweise zum Beispiel in einfacher Weise dadurch regenerierbar, dass man bei einer Eintrittstemperatur von 300 bis 600 °C (in Extremfällen gegebenenfalls auch bis 750 °C), häufig 400 bis 450 °C, zunächst in ersten Regenerierstufen mit Stickstoff und/oder Wasserdampf (bevorzugt) verdünnte Luft durch das Katalysatorfestbett leitet. Die Katalysatorbelastung mit Regeneriergas kann (bezogen auf die regenerierte Katalysatorgesamtmenge) dabei z. B. 50 bis 10000 $h^{-1}$ und der Sauerstoffgehalt des Regeneriergases 0,5 bis 20 Vol.-% betragen.

In nachfolgenden weiteren Regenerierungsstufen kann unter ansonsten gleichen Regenerierungsbedingungen als Regeneriergas Luft verwendet werden. Anwendungstechnisch zweckmäßig empfiehlt es sich, den Katalysator vor seiner Regenerierung mit Inertgas (z. B. $N_2$, z. B. Industriestickstoff mit bis zu 1 Vol.-% $O_2$, $H_2O$ oder deren Gemische) zu spülen.

Anschließend ist es in der Regel empfehlenswert, noch mit reinem molekularem Wasserstoff oder mit durch Inertgas (vorzugsweise Wasserdampf und/oder Stickstoff) verdünntem molekularem Wasserstoff (der Wasserstoffgehalt sollte ≥ 1 Vol.-% betragen) im ansonsten gleichen Bedingungsraster zu regenerieren.

Erfindungsgemäß vorteilhaft beträgt das Verhältnis aus der Schüttlänge X (in Strömungsrichtung des Reaktionsgasgemischeingangsstroms) der inerten Deckschicht des Katalysatorfestbetts und dem Abstand d in der Projektionsebene E, d. h., X : d, ≥ 0,05, vorzugsweise ≥ 0,07 und mit besonderem Vorteil ≥ 0,1. Häufig wird das vorgenannte Verhältnis X : d beim erfindungsgemäß Verfahren ≥ 0,2, oder ≥ 0,3, oder ≥ 0,5 oder ≥ 1 betragen. Normalerweise beträgt das Verhältnis X : d beim erfindungsgemäßen Verfahren jedoch meist ≤ 10 und vielfach ≤ 5.

Günstig ist es für das erfindungsgemäße Verfahren, wenn beim Eintritt des Reaktionsgasgemischs in die in Strömungsrichtung hinter der Inertschüttung befindliche eigentliche katalytisch aktive Schüttung des Katalysatorfestbetts die Verteilung des molekularen Sauerstoffs im Reaktionsgasgemisch möglichst homogen ist. Vorzugsweise sollte der Unterschied zwischen der höchsten lokalen Volumenkonzentration an molekularem Sauerstoff und der niedrigsten lokalen Volumenkonzentration an molekularem Sauerstoff im Reaktionsgasgemisch, bezogen auf die mittlere Volumenkonzentration an molekularem Sauerstoff im Reaktionsgasgemisch, beim Eintritt des Reaktionsgasgemischs in die eigentliche katalytisch aktive Schüttung nicht mehr als 200 %, vorzugsweise nicht mehr als 150 %, besonders bevorzugt nicht mehr als 100% und ganz besonders bevorzugt nicht mehr als 50 % bzw. nicht mehr als 25 % betragen.

Typische Induktionszeiten J können beim erfindungsgemäßen Verfahren $\leq$ 2000 ms, oder $\leq$ 1000 ms, oder $\leq$ 500 ms, oder $\leq$ 100 ms, oder $\leq$ 50 ms, oder $\leq$ 20 ms, oder $\leq$ 10 ms betragen. Normalerweise liegen die Induktionszeiten J beim erfindungsgemäßen Verfahren jedoch bei Werten von $\geq$ 1 ms.

Grundsätzlich können die Gasleitungen des das Eingangsgas II führenden Leitungssystems beim erfindungsgemäßen Verfahren jeden beliebigen Querschnitt aufweisen. D. h., der Querschnitt kann z. B. rund (z. B. kreisrund, oder oval bzw. elliptisch) oder vieleckig sein (z. B. dreieckig, viereckig oder sechseckig etc.).

Anwendungstechnisch zweckmäßig ist der Querschnitt der das Eingangsgas II führenden Leitungen dort, wo sich die Austrittsöffnungen A befinden, vieleckig. Vorzugsweise dreieckig oder viereckig. Besonders bevorzugt ist der Querschnitt der das Eingangsgas II führenden Leitungen (Kanäle) beim erfindungsgemäßen Verfahren dort, wo sich die Austrittsöffnungen A befinden, quadratisch oder rechteckig (die Austrittsöffnungen A befinden sich dann möglichst nahe an der dem Katalysatorfestbett nächstliegenden Kante des Vierecks). Ursächlich ist das Vorgenannte darauf zurückzuführen, dass bei Anwendung der vorgenannten vieleckigen Querschnitte sowohl die Entfernungen D als auch die Winkel $\alpha$ für alle Austrittsöffnungen A sowohl präziser als auch leichter einheitlich zu fertigen (einstellbar) sind. Die Austrittsöffnungen A selbst sind auch bei vorgenannten eckigen Querschnitten bevorzugt (kreis)rund und vorzugsweise mit über alle Austrittsöffnungen A einheitlichem Durchmesser gefertigt. Erfindungsgemäß bevorzugt ist D dabei für alle Austrittsöffnungen A identisch. Erfindungsgemäß vorteilhaft ist zusätzlich auch $\alpha$ für alle Austrittsöffnungen A identisch und liegt mit Vorteil bei 90° $\pm$ 5° bzw. bei 90°. Desweiteren sind beim erfindungsgemäßen Verfahren in vorteilhafter Weise auch die aus den Austrittsöffnungen A austretenden Eingangsgas-II-ströme identisch und die Austrittsöffnungen A mit Bezug auf die Anströmfläche des Katalysatorfestbetts mit dem Reaktionsgasgemischeingangsstrom über die Anströmfläche vorzugsweise ideal gleich verteilt.

Ist das Katalysatorfestbett beim erfindungsgemäßen Verfahren in einem als Hordenreaktor ausgestalteten Schachtreaktor in Strömungsrichtung des Reaktionsgases das auf ein anderes Katalysatorfestbett (Vorläuferkatalysatorfestbett) in Strömungsrichtung des Reaktionsgases nachfolgende Katalysatorfestbett, ist es für das erfindungsgemäße Verfahren vorteilhaft, wenn das Vorläuferkatalysatorfestbett möglichst einheitlich, möglichst homogen (beschickt) ist (insbesondere die Schüttlänge in Strömungsrichtung des Reaktionsgases betreffend). Konsequenz einer solchen möglichst einheitlichen Gestaltung des Vorläuferkatalysatorfestbetts ist eine weitgehend einheitliche Durchströmung beim Durchgang des Reaktionsgases durch das Vorläuferkatalysatorfestbett und daraus resultierend ein über den Eingangsgasstrom I des erfindungsgemäßen Verfahrens weitestgehend einheitliches Druckniveau.

Auf diese Weise ist es möglich, das zu erreichen, was für das erfindungsgemäße Verfahren von generellem Vorteil ist. Dass nämlich der maximal auftretende Druckunterschied innerhalb der vor den Austrittsöffnungen A im Eingangsgasstrom I bestehenden lokalen Drucke kleiner oder höchstens gleich groß ist, wie die beim Austreten der individuellen Eingangsgas-II-ströme aus den Austrittsöffnungen A auftretenden Druckverluste. Betragen letztere Druckverluste z. B. 5 mbar, ist es erfindungsgemäß vorteilhaft, wenn der vorgenannte maximal auftretende Druckunterschied nicht mehr als 3 mbar beträgt.

Es sei an dieser Stelle noch festgehalten, dass beim erfindungsgemäßen Verfahren das das Eingangsgas II führende Leitungssystem vorteilhaft so gestaltet wird, dass der Druckverlust, der auf dem Weg von der Einspeisestelle des Eingangsgases II in das Leitungssystem bis zur Ankunft des Eingangsgases II an den Austrittsöffnungen A möglichst gering (insbesondere relativ zum Druckverlust beim Durchtritt durch die Austrittsöffnungen A; in der Regel sollte er nicht mehr als ein Fünftel, mit Vorteil nicht mehr als ein Zehntel des vorgenannten Druckverlustes betragen), und bei einheitlichen Austrittsöffnungen A, möglichst einheitlich ist (insbesondere dann, wenn die Zufuhr des Eingangsgases II auf eine bestimmte Gesamtzufuhrmenge geregelt erfolgt). Dermaßen geringe Druckverluste stellen sich z. B. dann ein, wenn die Länge des Leitungssystems und die Strömungsgeschwindigkeit im Leitungssystem möglichst gering und der Strömungsquerschnitt im Leitungssystem groß gehalten werden.

Wird das erfindungsgemäße Verfahren in einem Hordenreaktor bei mehreren Katalysatorfestbetten (gegebenenfalls bei allen (bzw. allen minus eins) im Hordenreaktor befindlichen) angewendet, erfolgt die Zufuhr von Eingangsgas II vor jedem Katalysatorfestbett bevorzugt mit einem unabhängigen Leitungssystem. Grundsätzlich können diese unabhängigen Leitungssysteme jedoch alle aus einer Eingangsgas II-Quelle versorgt (gespeist) werden.

In der Regel werden sie jedoch mit voneinander verschiedenen (eine unterschiedliche Zusammensetzung aufweisenden) Eingangsgasen II gespeist werden.

Als Fertigungsmaterial für das für das erfindungsgemäße Verfahren erforderliche E-quipment kommen insbesondere die in der deutschen Anmeldung Nr. 10 2005 061 626 empfohlenen Stähle in Betracht. Auf der produktberührten Seite insbesondere auch Edelstahl vom DIN Typ 1.4841.

Das erfindungsgemäße Verfahren ist insbesondere dann vorteilhaft, wenn es in einem als Hordenreaktor ausgestalteten Schachtreaktor durchgeführt und dabei auf alle im Schachtreaktor befindlichen Katalysatorfestbetten angewendet wird, gegebenenfalls ausgenommen das in Strömungsrichtung des Reaktionsgases erste Katalysatorfestbett im Schachtreaktor. Die letztere Ausnahme wird in der Regel dann gegeben sein, wenn das dem Schachtreaktor zugeführte Reaktionsgas bereits das den wenigstens einen zu dehydrierenden Kohlenwasserstoff, gegebenenfalls in ausreichender Menge molekularen Wasserstoff sowie bedarfsgerecht molekularen Sauerstoff enthält. Das letztere ist normalerweise dann der Fall, wenn das dem als Hordenreaktor ausgestalteten Schachtreaktor zugeführte Reaktionsgas aus einer der heterogen katalysierten partiellen Dehydrierung des wenigstens einen zu dehydrierenden Kohlenwasserstoff (z. B. Propan) zu wenigstens einem dehydrierten Kohlenwasserstoff (z. B. Propylen) nachgeschalteten (in der Regel ebenfalls heterogen katalysierten) Partialoxidation des wenigstens einen dehydrierten Kohlenwasserstoff (z. B. Propylen zu Acrolein und/oder Acrylsäure als Zielprodukt) in Begleitung von verbliebenem (noch) zu dehydrierendem Kohlenwasserstoff stammendes, nach Zielproduktabtrennung aus dem Produktgasgemisch der Partialoxidation verbliebenes, Oxygenat (z. B. nicht umgesetzten molekularen Sauerstoff) enthaltendes Restgas umfasst (unter einer vollständigen Oxidation (Verbrennung) eines dehydrierten und/oder zu dehydrierenden Kohlenwasserstoffs wird in dieser Schrift verstanden, dass der im Kohlenwasserstoff insgesamt enthaltene Kohlenstoff in Oxide des Kohlenstoffs (CO, $CO_2$) und der in ihm enthaltene Wasserstoff in $H_2O$ umgewandelt wird; alle davon verschiedenen Umsetzungen eines dehydrierten und/oder zu dehydrierenden Kohlenwasserstoffs unter reaktiver Einwirkung von molekularem Sauerstoff werden in dieser Schrift mit dem Begriff der Partialoxidation subsumiert; die zusätzliche reaktive Einwirkung von Ammoniak kennzeichnet die Ammoxidation, die ebenfalls unter dem Begriff der Partialoxidation subsumieren soll).

Beispielsweise kann das dem Schachtreaktor zugeführte Reaktionsgas ein Gemisch aus frischem zu dehydrierendem Kohlenwasserstoff (z. B. Frischpropan) und vorgenanntem Restgas (das in der Regel auch Wasserdampf enthält) sein (gegebenenfalls können aber zusätzlich $H_2$, $H_2O$ und/oder $O_2$ dem Reaktionsgas vorab dessen Eintritt in den als Hordenreaktor ausgeführten Schachtreaktor zudosiert werden; beispielsweise kann auch Dehydrierkreisgas gemäß der WO 03/076370 bzw. gemäß der DE-A 102 11 275 dem Reaktionsgas vorab dessen Eintritt in den Hordenreaktor zudosiert werden).

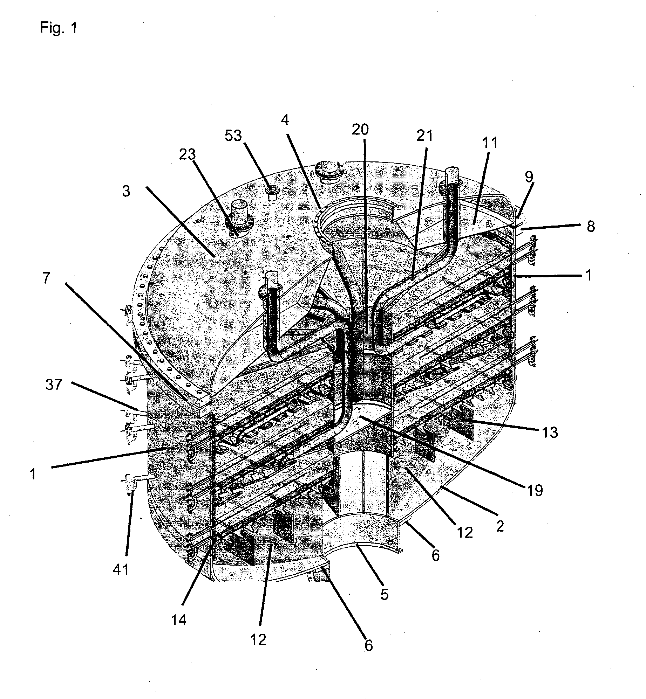

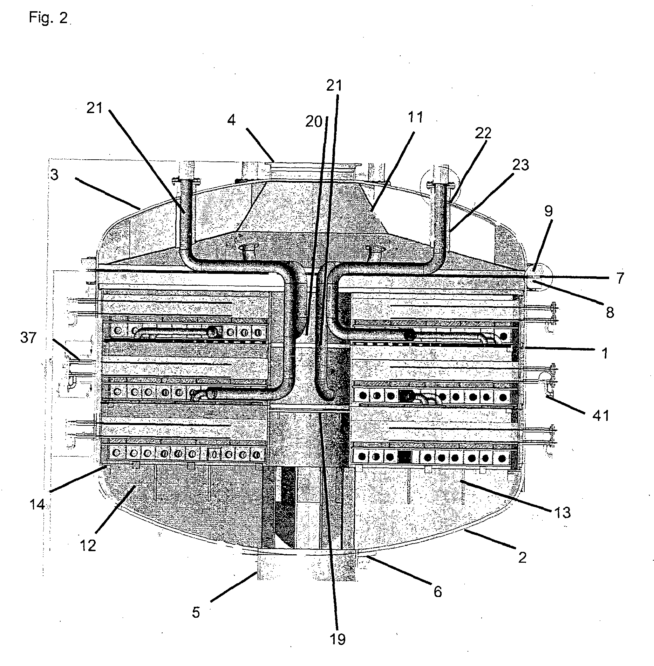

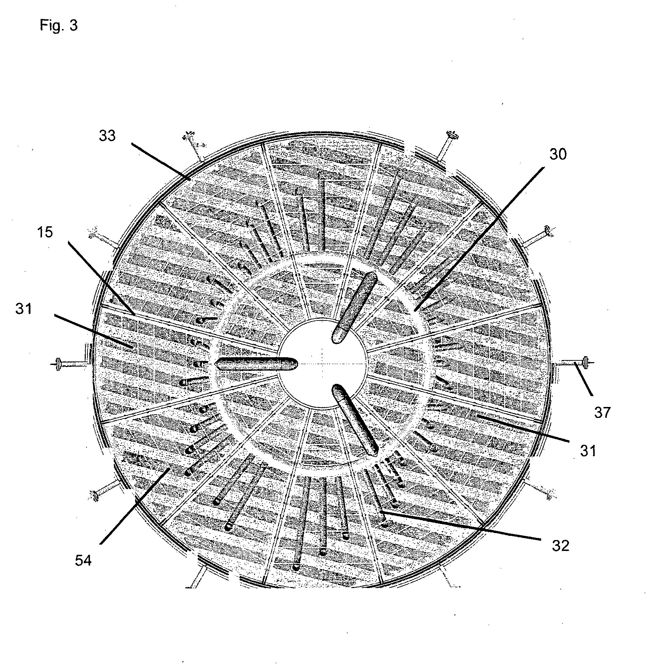

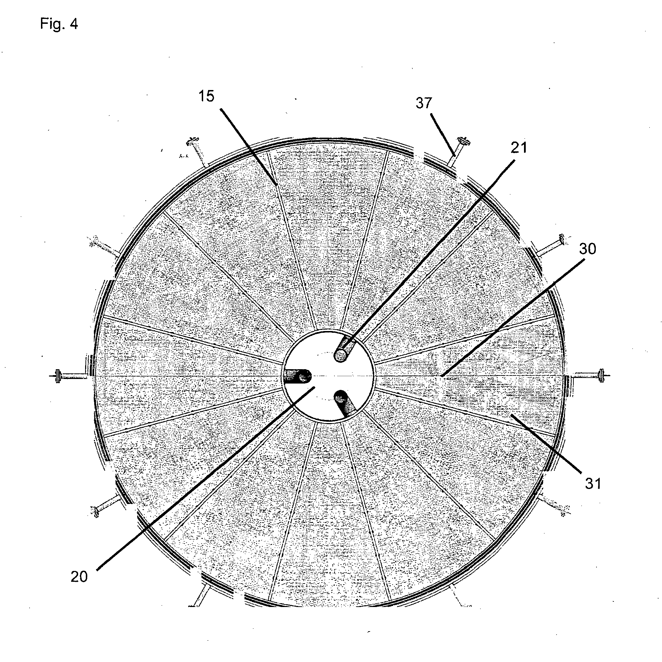

Das Design eines solchen als Hordenreaktor ausgeführten Schachtreaktor (vgl. Figuren 1 und 2) kann dabei (wie ganz generell für das erfindungsgemäße Verfahren) wie folgt gestaltet sein (die im Nachfolgenden verwendeten numerischen Adressen beziehen sich auf die Figuren 1 bis 17 dieser Anmeldung; die angegebenen Dimensionen beziehen sich im Zusammenhang beispielhaft auf eine konkrete Ausführungsform eines solchen Reaktors; selbstredend können entsprechende axiale Hordenreaktoren aber auch größer oder kleiner ausgeführt sein; auch kann die Anzahl der Katalysatorfestbetten erhöht oder geringer sein).

Nach außen (d. h., gegen die Umgebung) ist der im weiteren beispielhaft ausgeführte axiale Hordenreaktor durch den Mantel (1) eines geraden Kreiszylinders (verbindet man die Endpunkte paralleler Radien zweier in parallelen Ebenen liegender gleich großer Kreise miteinander durch Strecken, die senkrecht auf den beiden parallelen Kreisebenen (Grundfläche und Deckfläche (die in Strömungsrichtung des Reaktionsgases erste der beiden Kreisflächen) des Kreiszylinders) stehen, so entsteht ein gerader (nicht schiefer) Kreiszylinder (auch Rotationszylinder genannt; die Verbindungsstrecke der Kreismittelpunkte heißt Achse des Kreiszylinders)) abgegrenzt, der sowohl nach oben als auch nach unten durch je eine eine Klöpperform (gemäß DIN 28011) aufweisende gewölbte Haube (2,3) abgeschlossen ist (Mantel und Hauben bilden zusammen die Druckbehältereinhüllende). Unterhalb des unteren Klöpperbodens wird der Druckbehälter von einer Standzarge eingefasst, die ihrerseits z. B. von vier bis acht Stützen getragen wird. Die Manteldicke beträgt 30 mm. Der Außendurchmesser des Kreiszylindermantels beträgt 6070 mm. Der Abstand von Grundfläche und Deckfläche des Kreiszylinders beträgt 2640 mm. Die Höhe sowohl der oberen als auch der unteren Haube liegt bei 1350 mm (jeweils bestimmt vom Mittelpunkt der kreisförmigen Grundfläche bzw. Deckfläche bis zum jeweiligen Haubenmaximum). Die Wanddicke der oberen Haube (3) liegt bei einheitlichen 30 mm, die Wanddicke der unteren Haube (2) beginnt bei 30 mm und verdickt sich zum Gasaustrittsrohrstutzen hin stetig auf 45 mm.

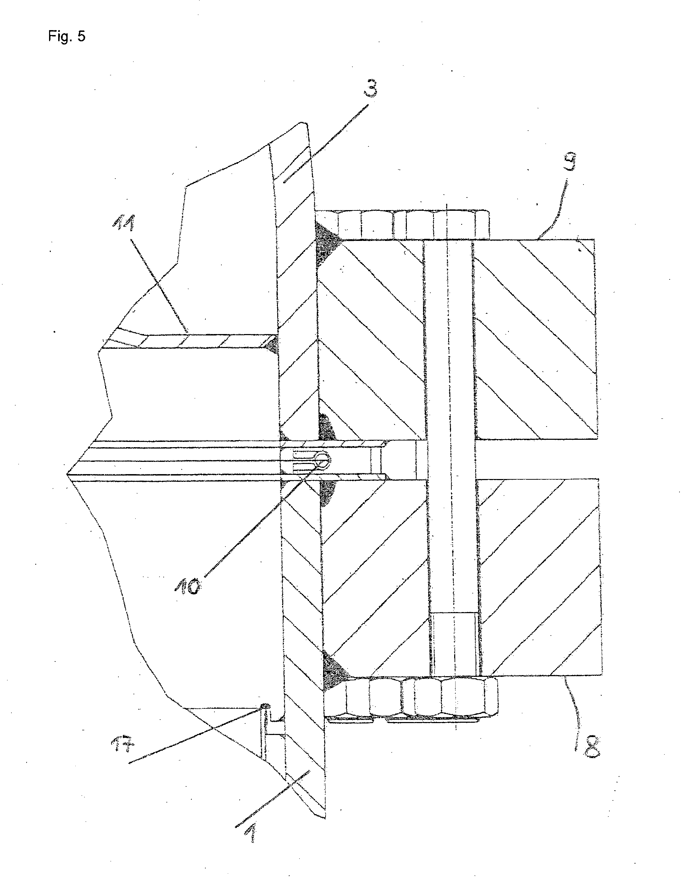

Die obere Haube läuft in einem zentrierten (zentralen) Gaseintrittsrohrstutzen (4) aus. Sein Innendurchmesser liegt bei 1200 mm. Die untere Haube läuft in entsprechender Weise in einem zentrierten (zentralen) Gasaustrittsrohrstutzen (5) aus. Sein Innendurchmesser liegt bei 1400 mm. Die Stutzenhöhe beträgt jeweils 240 mm (werden auf der Außenseite der Druckbehälterwand zur thermischen Isolierung entsprechende Isoliermaterialien aufgebracht (z.B. in einer Schichtdicke von 300 bis 500 mm), ist die Stutzenhöhe entsprechend größer, da der Stutzen anwendungstechnisch zweckmäßig aus der Isolierschicht herausragen sollte). Dort wo die untere Haube in den Gasaustrittsrohrstutzen (5) ausläuft, ist die Haube zusätzlich mit einem Verstärkungsring (6) ausgerüstet, der an der Haube und am Stutzen angeschweißt ist. Sein Außendurchmesser beträgt 1900 mm und seine Dicke beträgt 50 mm. Während die untere Reaktorhaube mit dem Kreiszylindermantel vorzugsweise durch Anschweißen verbunden ist, erfolgt das Verbinden der oberen Reaktorhaube mit dem Kreiszylindermantel vorzugsweise durch eine Flanschverbindung (7) (zwischen einem peripheren Flansch des Kreiszylindermantel (8) und einem entsprechenden peripheren Flansch an der Haube (9)) mit Schweißlippendichtung (10). Fig. 5 zeigt einen vergrößerten Ausschnitt der Flanschverbindung im Längsschnitt einschließlich Schweißlippendichtung (10) (letztere wird in typischer Weise aus Stahlblechen gefertigt, die eine Dicke von ca. 2 mm aufweisen; entsprechende Schweißlippendichtungen zeigt auch die WO 2004/067164). Die Gasleckage durch die angewandte Flanschverbindung sollte < $10^{-4}$ mbar·l/s betragen. Die Abnehmbarkeit der oberen Haube erleichtert die Befüllung mit bzw. die Entnahme von inerten Formkörpern und/oder Katalysator, z. B. im Fall eines Teilkatalysatorwechsels oder im Fall eines Vollkatalysatorwechsels.

Um die Verweilzeit eines über den Gaseintrittrohrstutzen (4) zugefügten Reaktionsgases, das den wenigstens einen zu dehydrierenden Kohlenwasserstoff und molekularen Sauerstoff enthält, im Innenraum der oberen Reaktorhaube (3) zu minimieren (und so unerwünschte Nebenreaktionen in einem bereits erhöhte Temperatur aufweisenden Reaktionsgas möglichst weitgehend zu vermeiden), ist das Haubeninnenvolumen mittels einer eingezogenen Zwischendecke (11) verkleinert, deren Gestalt z. B. dem Mantel (mit einer Manteldicke von 15 mm) eines geraden Kreiskegelstumpfes oder zweier aufeinandergesetzter gerader Kegelstümpfe entsprechen kann.

Die Innenwand der oberen Haube und die Kreiskegelstumpfmäntel werden zweckmäßig mit radial angeordneten Rippen versteift (vgl. WO 2004/067164).

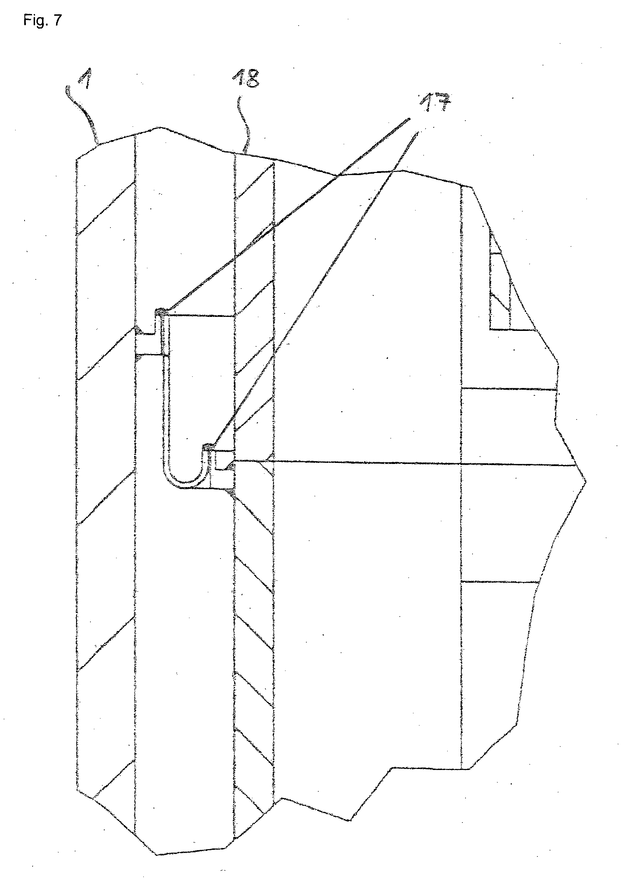

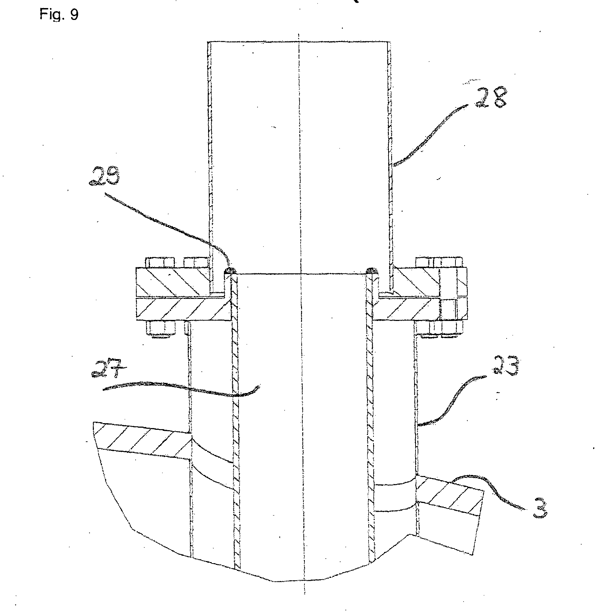

Der Deckkreis des Kreiskegelstumpfes entspricht dem Grundkreis des Gaseintrittsrohrstutzens (4). Selbstverständlich kann die Zwischendecke aber auch zur Deckfläche des Mantelkreiszylinders hin ausgewölbt sein, wie es z. B. in Fig. 5 und in Fig. 7 sowie in Fig. 9 der WO 2004/067164 gezeigt ist. Vorzugsweise sollte die eingezogene Zwischendecke (11) das für das Reaktionsgas zugängliche Haubeninnenvolumen, bezogen auf seinen Wert in Abwesenheit einer Zwischendecke, um wenigstens 50 Vol.-%, normalerweise jedoch um nicht mehr als 90 Vol.-%, verringern. Das Anbringen der Zwischendecke (11) erfolgt durch Anschweißen. Die Zwischendecke selbst weist anwendungstechnisch zweckmäßig im wesentlichen gleichmäßig an ihrem Umfang verteilt 3 bis 36 Durchtrittsöffnungen auf, deren Öffnungsquerschnitt 1 bis 8 cm$^2$ beträgt. Über diese Durchtrittsöffnungen kommuniziert der zwischen Zwischendecke und Reaktorhaube eingeschlossene Raum R mit dem verbliebenen Haubeninnenvolumen. Um zu gewährleisten, dass im wesentlichen kein Reaktionsgas über vorgenannte Durchtrittsöffnungen in den Raum R dringt, wird der Raum R kontinuierlich mit einem Inertgas (vorzugsweise molekularer Stickstoff, z. B. Industriestickstoff mit bis zu 1 Vol.-% $O_2$, Wasserdampf oder deren Gemische) gespült, das über 1 bis 12 Zuleitungen in die Haubendecke zugeführt (53) und über die Durchtrittsöffnungen abgeführt (dem Reaktionsgas zugefügt) wird. Dieser Inertgasstrom wird in der Regel insgesamt weniger als 1 Vol.-%, meist sogar weniger als 0,1 oder weniger als 0,01 Vol.-%, bezogen auf den Volumenstrom des Reaktionsgases (in Nm$^3$), betragen.

Die das Reaktionsgas zuführende Rohrleitung wird mit dem Gaseintrittsrohrstutzen (4) ebenso durch Anflanschen gasdicht verbunden, wie die den Produktgasstrom abführende Rohrleitung mit dem Gasaustrittsrohrstutzen (5). Anwendungstechnisch zweckmäßig wird in beiden Fällen eine Schweißlippendichtung angewendet. Alternativ kann die zuführende Rohrleitung auch unmittelbar an den Gaseintrittsrohrstutzen (4) angeschweißt werden.

Die vorgenannten (Rohr)Leitungen werden für einen sachgerechten und sicheren Betrieb bevorzugt mit Einrichtungen zur Kompensation von Längenausdehnungseffekten, wie sie z. B. aufgrund von Temperaturänderungen auftreten können, ausgerüstet, wobei mit Vorteil Kompensatoren eingesetzt werden, die sich durch laterale Wirkungsweise auszeichnen.

Diese in der Regel mehrschichtig ausgeführten Kompensatoren können aus dem gleichen Werkstoff wie die Rohrleitung selbst gefertigt sein. Besonders vorteilhaft sind jedoch Ausführungsformen mit (allgemein: gasdurchlässigem starrem Innenrohr und gasundurchlässiger elastischer Außenhülle (gasundurchlässigem elastischem Außenrohr)) einem das zu führende Gas berührenden inneren, vorzugsweise aus dem Edelstahl der DIN Werkstoffnummer 1.4893 gefertigten, Rohrteil, der zweckmäßig eine gasdurchlässige Dehnfuge aufweist, und einem außenliegenden, gasundurchlässigen, elastischen, gewellten Teil, der wenigstens teilweise aus einem insbesondere mechanisch und temperaturbelastbaren Werkstoff gefertigt ist, wie z. B. dem Werkstoff 1.4876 (Bezeichnung nach VdTÜV-Wb 434) bzw. 1.4958/1.4959 (Bezeichnung nach DIN 17459) bzw. INCOLOY 800 H bzw. 800 HT oder Nickelbasiswekstoff 2.4816 (alternative Bezeichnung Alloy 600) oder 2.4851 (alternative Bezeichnung Alloy 601).

Im übrigen können alle Teile des in Rede stehenden Hordenreaktors aus dem Edelstahl der DIN Werkstoffnummer 1.4893 oder aus einem anderen in der deutschen Anmeldung 10 2005 061 626 empfohlenen bzw. erwähnten Edelstahl gefertigt werden. Auf der produktberührten Seite insbesondere auch aus Edelstahl vom DIN Typ 1.4841.

Der Innenraum der unteren Haube wird durch das Aufsetzen von zwölf Lamellen(wänden) (12) auf der Haubeninnenwand über die gesamte Haubenhöhe in zwölf (alternativ kommen auch acht, sechs, oder vier, oder drei, oder sechzehn in Betracht) kongruente Sektoren (kammern) aufgeteilt. Auf der die Haubeninnenwand berührenden Seite ist die Form der einzelnen Lamelle (der jeweilige Lamellenrücken) der Haubenwölbung angepasst. Die Wanddicke der Lamellen beträgt einheitliche 30 mm. Neben dem Lamellenrücken weist die einzelne aufgesetzte Lamelle jeweils noch zwei Kanten K und C auf. Die Kanten K verlaufen parallel zur Achse des Mantelkreiszylinders und erstrecken sich mit einer Länge von 1400 mm von der Innenwand der unteren Haube bis zur Grundfläche des Mantelkreiszylinders, auf der die Kanten K senkrecht stehen. Die Kanten K bilden die dem Haubeninneren zugewandte Stirn der jeweiligen Lamelle. Die Kanten C berühren die Grundfläche des Mantelkreiszylinders und schließen die einzelne Lamelle nach oben ab. Wie die vorgenannte Grundfläche selbst, schließen sie mit der zugehörigen Kante K einen rechten Winkel ein, d. h., sie verlaufen auf einem Radius dieser kreisförmigen Grundfläche. Während diese Radien jedoch eine Länge von 3010 mm aufweisen, beträgt die Länge der Kanten C lediglich 2460 mm. Die Kante C steht nicht auf der Kante K über, sondern schließt dort, wo sie mit der Kante K zusammenstößt, bündig ab. Das heißt, die Kanten K von einander gegenüberstehenden Lamellen (ihre Kanten C liegen auf einem gemeinsamen Durchmesser der Grundfläche des Mantelkreiszylinders) berühren sich nicht, sondern weisen einen Abstand von jeweils 1100 mm auf.

Die Kanten K enden mit ihrem unteren Ende auf der Umrisslinie des Gasaustrittrohrstutzens.

Verlängert man die Lamellenkanten C zweier benachbarter Lamellen so lange, bis sie sich schneiden, so schließen diese Lamellenkanten jeweils einen Winkel von 30° ein. Zwei benachbarte Lamellen schließen so gemeinsam mit der Haubeninnenwand, auf der sie aufgesetzt sind, jeweils eine Kammer ein, die sowohl zur Grundfläche des Mantelkreiszylinders als auch zur zentralen Achse der unteren Haube (= Verlängerung der Symmetrieachse des Mantelkreiszylinders nach unten) hin offen ist. Zusätzlich können die Lamellenwände Durchtrittsöffnungen aufweisen, über die benachbarte Kammern zusätzlich miteinander kommunizieren können. Da die zwölf Lamellen auf der Innenwand der unteren Haube nur über die Schwerkraft aufsitzen und nicht über eine Schweißnaht mit der Innenwand der unteren Haube verbunden sind, werden die Positionierung und die Relativanordnung der zwölf Lamellen mit Hilfe von jeweils zwischen zwei benachbarten Lamellen angebrachten (angeschweißten) Verstrebungen (13) stabilisiert.

Dort wo die Grundfläche des Mantelkreiszylinders und die untere Haube aufeinanderstoßen, ist ein waagrecht umlaufender Auflagering (14) angebracht (an der Haubeninnenwand angeschweißt), der eine Auflagebreite von 60 mm aufweist. Die Dicke (Höhe) des Auflagerings beträgt 20 mm.

Dort wo die einzelnen Lamellen von unten auf den Auflagering stossen, weisen sie eine passgenaue Einkerbung auf, in die sich der Auflagering nahtlos einfügt, so dass die Kante C und die Auflagefläche des Auflagerings auf einer Höhe liegen und die Auflagefläche die Kante C auf deren Länge in Abwesenheit der Einkerbung ergänzt.

Zur Aufnahme der verschiedenen Katalysatorfestbetten in dem vorstehend beschriebenen Druckbehälter, wird in diesen eine entsprechende Anzahl von Kreisringzylindereinheiten eingelegt.

Unter einem Kreisring wird dabei das Gebiet in einer Ebene verstanden, das durch zwei konzentrische Kreisringe mit voneinander verschiedenem Radius begrenzt wird. Verbindet man von zwei in parallelen Ebenen liegenden kongruenten (deckungsgleichen) Kreisringen (dem Grundkreisring und dem Deckkreisring) die jeweiligen Endpunkte paralleler Radien auf den beiden äußeren Kreisen sowie die jeweiligen Endpunkte paralleler Radien auf den beiden inneren Kreisen durch Strecken, so entsteht ein Kreisringzylinder. Die Verbindungsstrecken der Endpunkte auf den beiden inneren Kreisen heißen innere Mantellinien des Kreisringzylinders (ihre Gesamtheit bildet einen inneren Mantel) und die Verbindungsstrecken der Endpunkte auf den beiden äußeren Kreisen heißen äußere Mantellinien des Kreisringzylinders (ihre Gesamtheit bildet einen äußeren Mantel). Stehen die Mantellinien senkrecht auf den beiden Kreisringen, so heißt der Kreisringzylinder gerade oder nicht schief. Die Verbindungsstrecke der Kreisringmittelpunkte heißt Achse des Kreisringzylinders. Wird ein gerader Kreisringzylinder von zwei zum Grundkreisring und Deckkreisring parallelen Ebenen, die den Abstand H voneinander haben, geschnitten, so entsteht zwischen den Ebenen eine Kreisringzylindereinheit der Höhe H, die ebenfalls einen Grundkreisring und einen Deckkreisring aufweist.

Die Wanddicke des äußeren Mantels der für den beschriebenen Druckbehälter verwendeten Kreisringzylindereinheiten beträgt 20 mm und die Wanddicke des inneren Mantels der für den beschriebenen Druckbehälter verwendeten Kreisringzylindereinheiten beträgt 30 mm. Ihre Höhe H beträgt 850 mm (Ausnahme: bei der in Strömungsrichtung des Reaktionsgases ersten Kreisringzylindereinheit beträgt die Höhe H 710 mm). Der Radius des inneren Kreises der vorgenannten Kreiszylindereinheiten (Abstand vom Kreisringmittelpunkt bis zum inneren Mantel der Kreisringzylindereinheiten) beträgt 600 mm, der Radius des äußeren Kreises der vorgenannten Kreisringzylindereinheiten (Abstand von Kreisringmittelpunkt bis zum äußeren Mantel der Kreisringzylindereinheiten) beträgt 2900 mm. Der Abstand zwischen dem äußeren Mantel der Kreisringzylindereinheiten und dem inneren Mantel der Kreisringzylindereinheiten beträgt somit 2270 mm.

Der vom inneren Mantel umhüllte Raum wird als Kreisringzylindereinheitinnenraum bezeichnet. Der zwischen dem äußeren und dem inneren Mantel befindliche Raum wird als Kreisringzylindereinheitzwischenraum bezeichnet.

Zwölf Trennwände $W_T$ (15), die eine Wanddicke von 30 mm aufweisen, unterteilen den Kreisringzylindereinheitzwischenraum in zwölf kongruente Sektorenräume.

Die einzelne Trennwand weist eine der Einheithöhe H entsprechende Höhe H auf. Die Länge T einer Trennwand entspricht dem Abstand zwischen dem äußeren Mantel der Kreisringzylindereinheit und dem inneren Mantel der Kreisringzylindereinheit. Diesem Sachverhalt entsprechend, ist die einzelne Trennwand sowohl am äußeren Mantel als auch am inneren Mantel über ihre Höhe H angeschweißt. Die Höhe H der Trennwand steht senkrecht auf Grundkreisring und Deckkreisring der Kreisringzylindereinheit. Die Länge T der Trennwand verläuft radial vom äußeren zum inneren Mantel und mündet, wenn man sie gedanklich verlängert, in der Achse der Kreisringzylindereinheit. Verlängert man zwei benachbarte Trennwände in der vorbeschriebenen Weise bis sie sich schneiden, so schließen sie einen Winkel von 30° ein.

Setzt man eine wie beschrieben segmentierte erste Kreisringzylindereinheit in den beschriebenen Druckbehälter ein, so wird sie in einfacher Weise auf dem Auflagering (14) abgestellt, und zwar so, dass einerseits zwischen dem Mantelkreiszylinder des Druckbehälters und dem äußeren Mantel der Kreisringzylindereinheit ein Abstand von 50 mm besteht, und andererseits die Trennwände der Kreisringzylindereinheit und die Lamellenwände in der unteren Haube des Druckbehälters aufeinander (bzw. übereinander, d. h., auf Deckung) stehen (d. h., die Achse des Mantelkreiszylinders und die Achse der Kreisringzylindereinheit fallen zusammen). Der Grundkreisring befindet sich in Strömungsrichtung des Reaktionsgases hinter dem Deckkreisring der eingesetzten segmentierten Kreisringzylindereinheit.

Zwischen benachbarten Wänden $W_T$ werden als Tragwerk jeweils zehn nichtgekrümmte Querwände (tragende Wände) Q (54) eingezogen, die eine Höhe von 200 mm und eine Wanddicke von 20 mm aufweisen. Die Anbindung der tragenden Wände an die Wände $W_T$ erfolgt durch Anschweißen. Die Länge der tragenden Wände nimmt mit zunehmendem Abstand derselben vom inneren Mantel der Kreisringzylindereinheit bzw. mit abnehmendem Abstand vom äußeren Mantel der Kreisringzylindereinheit in natürlicher Weise zu.

Untereinander sind die tragenden Wände Q innerhalb ein und desselben Sektorenraums anwendungstechnisch zweckmäßig äquidistant angeordnet.

Die tragenden Wände Q sind so eingezogen, dass der Abstand ihrer Unterkante zum Grundkreisring der segmentierten Kreisringzylindereinheit 40 mm beträgt.

Darüber hinaus weisen die Querwände Q Durchtrittsöffnungen O auf, deren Schwerpunkt in zweckmäßiger Weise auf der halben Höhe der Querwand liegt. Mit zunehmendem Abstand vom inneren Mantel der Kreisringzylindereinheit nimmt die Anzahl der Durchtrittsöffnungen O je Querwand zu. In verschiedenen Sektorenräumen befindliche Querwände Q, die denselben Abstand vom inneren Mantel der Kreisringzylindereinheit aufweisen, werden in zweckmäßiger Weise kongruent gestaltet. Vorzugsweise sind die Durchtrittsöffnungen O kreisrund.

Auf das so innerhalb eines Sektorenraums geschaffene Tragwerk wird passgenau ein Gitterrostsektor gesetzt, der das Katalysatorfestbett trägt.

In der Regel werden die Katalysatorformkörper nicht unmittelbar auf den Gitterrostsektor geschüttet (gegebenenfalls wird auf den Gitterrost noch ein Netzwerk aus Metall-(z. B. aus dem Edelstahl, aus dem der Druckbehälter gefertigt ist)draht mit geringer Maschenweite aufgebracht). Dies rührt daher, dass die Katalysatorformkörper üblicherweise eine geringere Ausdehnung als die Maschenweite des Gitterrosts aufweisen. Beim beschriebenen Reaktor beträgt die Maschenweite der verwendeten Gitterrostsektoren zweckmäßig 2 oder 3 bis 7 mm, z. B. 4 mm.

In entsprechender Weise werden zunächst in einer über alle Gittersektoren einheitlichen Schütthöhe von 50 mm inerte Kugeln aus Steatit C 220 der Fa. CeramTec mit einem Durchmesser von 5 mm aufgeschüttet, auf die eine Schüttung aus Katalysatorformkörpern folgt. Auf diese Inertschüttung wird eine weitere Inertschüttung der Schütthöhe 25 mm aus Kugeln desselben Steatit, aber mit einem Durchmesser von 2 bis 3 mm, aufgeschüttet, auf die eine Schüttung aus Katalysatorformkörpern folgt. Als Katalysatorformkörper werden z. B. Stränge des Durchmessers 1,5 mm und einer im Bereich 10 mm bis 15 mm gaußverteilten Länge mit Maximum bei ca. 12,5 mm verwendet (Elementstöchiometrie (Massenverhältnis der Aktivelemente einschließlich Träger) sowie Katalysatorvorläuferherstellung und Aktivierung zum aktiven Katalysator sind im übrigen wie in Beispiel 4 der DE-A 102 19 879).

Die Schütthöhe der Katalysatorformkörper beträgt einheitlich 300 mm (ihre Einheitlichkeit ist in einfacher Weise z. B. dadurch zu erzielen, dass man auf die fertiggestellte Schüttung (auf den Deckkreisring) ein Lochblech oder ein Gitternetz auflegt, und durch dessen Öffnungen ragende Katalysatorformkörper abträgt). Anschließend wird das Katalysatorfestbett mit einer Schüttung aus Ringen des Steatits C 220 der Fa. CeramTec abgeschlossen (bis zum Deckkreisring der segmentierten Kreisringzylindereinheit). Die Geometrie der Steatitringe ist 7 mm x 7 mm x 4 mm (Außendurchmesser x Höhe x Innendurchmesser) und die Schütthöhe beträgt einheitlich 200 mm (Ausnahme: bei der in Strömungsrichtung des Reaktionsgases ersten Kreisringzylindereinheit beträgt diese Schütthöhe nur 60 mm).

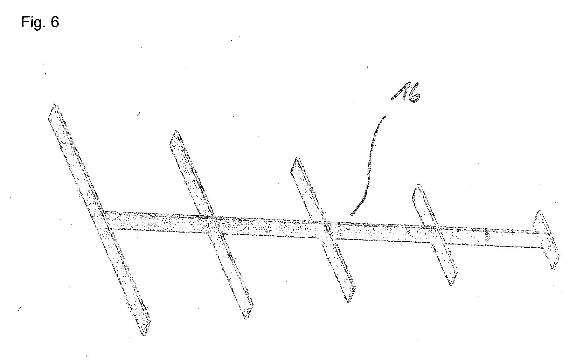

Einheitliche Schütthöhen der Inertschüttungen sind z. B. dadurch in einfacher Weise zu erzielen, dass man auf jeden Gittersektor eine im Inertbett verbleibende Schütthilfe (16) gemäß Figur 6 auflegt, die eine der Schütthöhe entsprechende Höhe aufweist, so dass längs derselben die Inertschüttung in einfacher Weise auf einheitliche Schütthöhe glattgestrichen werden kann. Die Wanddicke ihrer Elemente beträgt in typischer Weise 0,3 bis 2 cm. Entsprechende Schütthilfen können auch in der Aktivkörperschüttung verwendet werden.

Alternativ zu den Steatitringen können auch 7 mm x 7 mm Steatitvollzylinder (Außendurchmesser x Höhe) verwendet werden. Als weitere Alternative können auch Steatitkugeln mit einem Durchmesser von 5 mm bis 6 mm verwendet werden.

Auf diese in Strömungsrichtung letzte (unterste) mit einem Katalysatorfestbett beschickte Kreisringzylindereinheit werden zwei weitere solche mit einem solchen Katalysatorfestbett beschickte Kreisringzylindereinheit aufgesetzt. Die Trennwände $W_T$ aller Kreisringzylindereinheiten stehen dabei auf Deckung, d. h., in gleicher Weise aufeinander, wie ihre äußeren und inneren Mäntel.

Dort, wo die Kreisringzylindereinheiten aufeinanderstehen, bestehen in unvermeidbarer Weise geringfügige, gasdurchlässige Fugen. Der Raum (Spalt) zwischen dem Kreiszylindermantel (1) des Druckbehälters und den äußeren Mänteln der mit Katalysatorfestbett beschickten Kreisringzylindereinheiten wird zweckmäßig mit thermischem Isoliermaterial befüllt (z. B. Mineralwolle oder Glaswolle), das auf dem Auflagering (14) aufsitzt.

Auf der Höhe des Deckkreisrings der jeweiligen Kreisringzylindereinheit sind der Kreiszylindermantel (1) des Druckbehälters und der äußere Mantel der mit Katalysatorfestbett beschickten Kreisringzylindereinheit jeweils über eine Schweißlippendichtung (17) gasundurchlässig miteinander verbunden. Figur 7 zeigt eine Vergrößerung der Schweißlippendichtung (17) im Längsschnitt. Sie ist so beschaffen, dass sie thermische Kontraktionen und Expansionen (sowohl im Dehydrierbetrieb als auch im Rahmen der Katalysatorregenierung treten signifikante Temperaturänderungen auf) der jeweiligen Kreisringzylindereinheit aufzunehmen in der Lage ist, ohne aufzubrechen und die Dichtigkeit zu verlieren. Auf der Höhe des Grundkreisrings der jeweiligen Kreisringzylindereinheit besteht eine entsprechende Abdichtung nicht, weshalb von dort jeweils Reaktionsgas in den Raum (Spalt) zwischen Kreiszylinder (1) des Druckbehälters und dem äußeren Mantel (18) der jeweiligen Kreisringzylindereinheit gelangt und Druckausgleich bewerkstelligt.

Unterhalb des Deckkreisrings der in Strömungsrichtung des Reaktionsgases untersten (letzten) Kreisringzylindereinheit jedoch oberhalb des Grundkreisrings derselben Kreisringzylindereinheit wird in den Kreisringzylindereinheitinnenraum ein sich über den gesamten Kreisquerschnitt dieses Innenraums erstreckender und in entsprechender Weise kreisförmig gestalteter Boden (19) gasdicht an den inneren Mantel dieser Kreisringzylindereinheit angeschweißt. Dort wo zwei Kreisringzylinder aufeinanderstehen, wird oberhalb des Grundkreisrings der aus Strömungsrichtung des Reaktionsgases oberen Kreisringzylindereinheit im zugehörigen Kreiszylindereinheitinnenraum an den inneren Mantel eine am Umfang des Kreisringzylindereinheitinnenraums umlaufende Stahlblende (Stirnband) angeschweißt, die unterhalb der Schweißnaht über die zwischen den aufeinanderstehenden Kreisringzylindereinheiten bestehende Fuge glatt anliegend übersteht. Das Stirnband ist etwa 8 mm dick und 60 mm breit.

Im übrigen bilden die drei übereinander angeordneten Kreisringzylindereinheitinnenräume zusammen einen nicht schiefen kreiszylindrischen Gesamtinnenraum (20) aus, der nach unten durch den Boden (19) geschlossen ist und nach oben in die obere Haube mündet.

In diesem Gesamtinnenraum (20) werden die Rohre (21) geführt, durch die das für das erfindungsgemäße Verfahren benötigte Eingangsgas II strömt (Eingangsgas II-Zuführrohre). Der um die Eingangsgas II-Zuführrohre herum verbleibende Restraum des Gesamtinnenraums (20) wird mit Quarzsand befüllt, der eine Körnung von 0,3 bis 1,2 mm aufweist (alternativ können auch Oxidkeramiksplitt, -granulat, und/oder-kugeln (bzw. andere feinteilige Formkörper) verwendet werden; vorzugsweise ist die Befüllung staubfrei, was z. B. durch deren Waschen mit Wasser und nachfolgendes Trocknen (z. B. Feuertrocknen) bewirkt werden kann). Die Befüllung endet kurz (ca. 1 cm) unterhalb des Deckkreisrings der in Strömungsrichtung des Reaktionsgases ersten Kreisringzylindereinheit. Eine Zudosierung von Eingangsgas II wird (in Strömungsrichtung des Reaktionsgases) zwischen dem ersten und zweiten sowie zwischen dem zweiten und dritten Katalysatorfestbett vorgenommen.

Der Zutritt (22) der Rohre (21) in den Reaktor erfolgt über die obere Reaktorhaube (3) durch in selbiger befindliche und mit dieser fest verschweißte Eingangsstutzen (23).

Der Rohraußendurchmesser beträgt 168,3 mm und die Wandstärke kann 2 bis 7 mm betragen. Jeweils drei solche Rohre (Eingangsgas II-Zuführrohre) leisten die Versorgung mit Eingangsgas II zwischen zwei vorgenannten Katalysatorfestbetten. Innerhalb einer solchen Dreiergruppe von Eingangsgas II-Zuführrohren erfolgt die Speisung mit Eingangsgas II aus ein und derselben Quelle. In der Regel werden jedoch die Zusammensetzung des Eingangsgases II, das (in Strömungsrichtung) zwischen dem ersten und dem zweiten Katalysatorfestbett zugeführt wird, und die Zusammensetzung des Eingangsgases II, das zwischen dem zweiten und dem dritten Katalysatorfestbett zugeführt wird, voneinander verschieden sein und aus unterschiedlichen Quellen entstammen.

Die Zudosierung an Eingangsgas II zur jeweiligen Dreiergruppe an Eingangsgas II-Zuführrohren erfolgt jeweils Gesamtmengen geregelt.

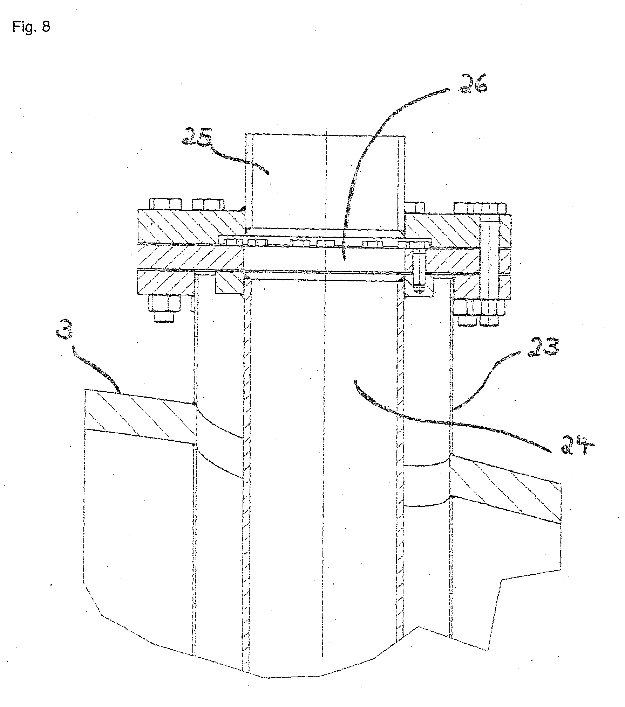

Die Abdichtung der Eintritte der Eingangsgas II-Zuführrohre in die obere Reaktorhaube zeigt die Figur 8 im Längsschnitt. Sie erfolgt über eine äußere und eine innere Flanschverbindung. Der Übergang eines Eingangsgas II zuführenden Rohres in den Eingangsstutzen (23) erfolgt dreigeteilt (dreifach gestückelt). Diese drei Teile sind der Rohrabschnitt im Eingangsstutzen (24), der Rohrabschnitt außerhalb des Eingangsstutzens (25) und der Rohrabschnitt in der Zwischenscheibe (26). Die innere Flanschverbindung bindet den unteren Rohrabschnitt von unten gasdicht an den Rohrabschnitt in der Zwischenscheibe an und die äußere Flanschverbindung dichtet einerseits den Eingangsstutzen gegen die Umgebung ab und bindet gleichzeitig den oberen Rohrabschnitt gasdicht von oben an den Rohrabschnitt in der Zwischenscheibe an. Als Dichtungsmaterial können Materialien auf der Basis von z. B. Glimmer oder gekammtem Graphit oder metallische Dichtungen verwendet werden. Die vorbeschriebene Konstruktionsweise ermöglicht bereits nach Lösen der äußeren Flanschverbindung das Abheben der oberen Reaktorhaube, ohne die Position der Rohre (21) im Reaktor verändern zu müssen.

Eine alternative Abdichtung der Eintritte der Eingangsgas II-Zuführrohre in die obere Reaktorhaube zeigt die Figur 9 im Längsschnitt.

Der Übergang eines Eingangsgas II zuführenden Rohres in den Eingangsstutzen (23) erfolgt zweigeteilt. Diese zwei Teile sind der Rohrabschnitt im Eingangsstutzen (27) und der Rohrabschnitt außerhalb des Eingangsstutzens (28). Der Innendurchmesser des letzteren ist 10 bis 30 mm größer als der Innendurchmesser des ersteren. Der gasdichte Übergang zwischen beiden erfolgt mittels einer Flanschverbindung. Dazu ist am oberen Ende des Stutzens ein peripherer Flansch angeschweißt, der bis zum im Stutzen geführten Rohrabschnitt ragt, der 10 bis 30 mm über diesen Flansch herausragt und mittels einer Schweißlippendichtung (29) am Flansch befestigt ist. Am unteren Ende des Rohrabschnitts außerhalb des Eingangsstutzens (28) ist ebenfalls ein peripherer Flansch angeschweißt. Als Dichtungsmaterial zwischen beiden Flanschen können Materialien auf der Basis von z. B. Glimmer, gekammtem Graphit oder eine metallische Dichtung verwendet werden.

Auf der Höhe der Durchtrittsöffnungen O der Querwände Q der in Strömungsrichtung des Reaktionsgases ersten Kreisringzylindereinheit und auf der Höhe der Durchtrittsöffnungen O der Querwände Q der in Strömungsrichtung des Reaktionsgases zweiten Kreisringzylindereinheit befinden sich im inneren Mantel der jeweiligen Kreisringzylindereinheit jeweils drei Durchtrittsöffnungen MO, deren Mittelpunkte auf den Ecken eines gleichseitigen Dreiecks liegen. Durch diese Öffnungen MO biegen die jeweils drei Eingangsgas II-Zuführrohre ab (sie sind in diese Öffnungen gasdicht eingeschweißt) und werden durch Durchtrittsöffnungen O der Querwände Q radial in Richtung des äußeren Mantels der jeweiligen Kreisringzylindereinheit geführt, bis sie auf eine in der jeweiligen Kreisringzylindereinheit angebrachte Eingangsgas II-verteilungskreisröhre KR (30) stossen, an die sie angeschweißt sind und die den selben äußeren und inneren Rohrdurchmesser aufweist, wie die ihr zugeführten Eingangsgas II-Zuführrohre. Die Verteilungskreisröhre KR ist auf der Höhe der Durchtrittsöffnungen O der jeweiligen Kreisringzylindereinheit durch viaduktartige Durchtrittsöffnungen in den jeweiligen Wänden $W_T$ geführt, jedoch nicht an den Viaduktbrücken angeschweißt.

Die Breite des Spalts zwischen Verteilungsröhre KR und Durchtrittsöffnung beträgt etwa 4 bis 15 mm.

Die Länge der Eingangsgas II-Zuführungsrohre, gerechnet von ihrem Eintritt in die obere Reaktorhaube bis zur jeweiligen Verteilungskreisröhre KR, liegt bei ≥ 3 und ≤ 6 m. Der Außendurchmesser des von den beiden Verteilungskreisröhren gezogenen Kreises beträgt 2860 mm. Der entsprechende Innendurchmesser liegt bei 2560 mm.

Während der Durchtritt der Eingangsgas II-Zuführungsrohre durch die Durchtrittsöffnungen MO abgedichtet erfolgt, trifft dies auf die entsprechenden Durchtritte durch die Durchtrittsöffnungen O nicht zu. Diese erfolgen vielmehr kommunizierend.

Die Spaltbreite beträgt etwa 2 bis 10 mm.

Von der Verteilungskreisröhre KR (30) führen durch Durchtrittsöffnungen O geführte Eingangsgas II-Verteilungsrohre radial in Richtung des äußeren sowie in Richtung des inneren Mantels der entsprechenden Kreisringzylindereinheit.

Der Außendurchmesser der Verteilungsrohre liegt im Bereich 80 bis 110 mm und kann z. B. 88,9 mm betragen. Die Wanddicke der Verteilungsrohre liegt im Bereich 2,1 bis 6,3 mm.

Zwischen den Querwänden Q der Kreisringzylindereinheit biegen die Verteilungsrohre (32) nach unten in Richtung des Grundkreisrings der Kreisringzylindereinheit ab.

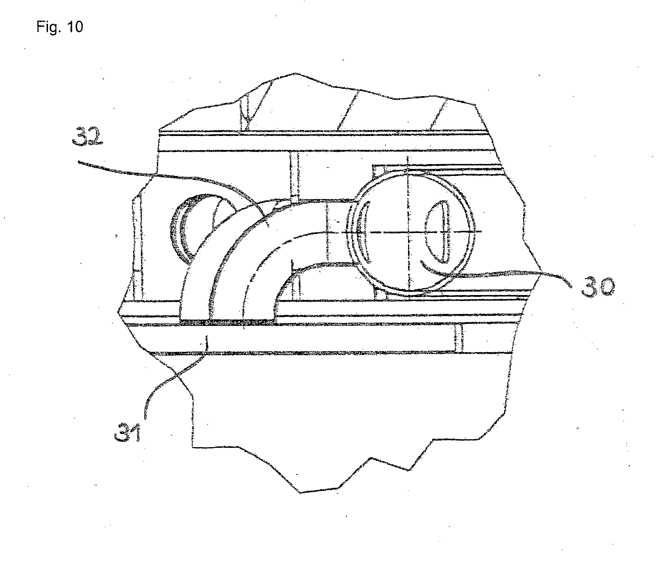

Unmittelbar unterhalb des Tragwerks der in Strömungsrichtung des Reaktionsgases ersten und der in Strömungsrichtung des Reaktionsgases zweiten (aber nicht unterhalb des Tragwerks der in Strömungsrichtung des Reaktionsgases dritten) Kreisringzylindereinheit befinden sich Austrittsöffnungen A aufweisende quaderförmige Zudosierungskästen (31), mit den Kanten a, b und c und drei paarweise kongruenten Rechtecken als ihre Begrenzungsflächen, in die die Verteilungsrohre gemäß gezeigtem Längsschnitt in Figur 10 münden (die Wanddicken der Zudosierungskästen betragen zweckmäßig 1 bis 3 mm, vorzugsweise 1,5 bis 2,5 und mit Vorteil 2 mm). In Zudosierungskästen (Verteilungskästen) mit kurzen Kanten a, mündet in der Regel nur ein Verteilungsrohr. In Zudosierungskästen (Verteilungskästen) mit langer Kante a münden bis zu drei Verteilungsrohre. Die Länge der Kanten c beträgt für alle Zudosierungskästen einheitlich 40 mm und die Länge der Kanten b beträgt für alle Zudosierungskästen einheitlich 150 mm. Im Unterschied dazu variieren die Längen der Kanten a für verschiedene Kästen. Eines der beiden paarweise kongruenten Rechtecke a x b liegt für alle Kästen jeweils in der Ebene des Grundkreisrings der Kreisringzylindereinheit.

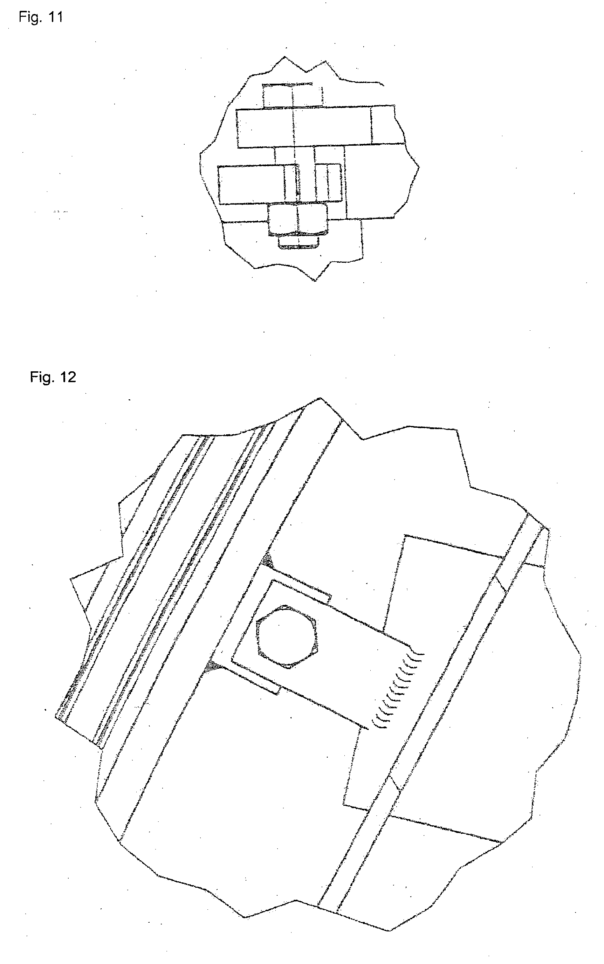

Die Kanten c stehen alle senkrecht auf der Ebene des Grundkreisrings und weisen in Richtung des zur Kreisringzylindereinheit gehörigen Deckkreisrings. Die Kanten a aller Kästen sind parallel ausgerichtet. Der Abstand zweier benachbarter Zudosierungskästen beträgt 130 mm. In dieser Abstandssequenz sind die Zudosierungskästen über den gesamten Querschnitt des Kreiszylindereinheitinnenraums verteilt angeordnet. Die Kanten a laufen wie Sekanten von einer Seite des äußeren Mantels der Kreisringzylindereinheit zur anderen Seite dieses äußeren Mantels. Stossen sie dabei auf den inneren Mantel der Kreisringzylindereinheit wird der Zudosierungskasten kurz vor dem inneren Mantel abgeschlossen und auf der gegenüberliegenden Umfangseite des inneren Mantels als (separater) neuer (frischer) Zudosierungskasten mit entsprechender Kantenlänge a fortgeführt. An der Innenfläche des äußeren Mantels sowie an der Außenfläche des inneren Mantels der Kreisringzylindereinheit befinden sich (vorzugsweise formschlüssig ausgeführte) Halterungen (33) an denen die Zudosierungskästen in der Mitte der Rechteckfläche b x c befestigt sind. Die Figuren 11 (Seitenansicht) und 12 (Draufsicht) zeigen ein Beispiel einer solchen formschlüssig ausgeführten Halterung.

Für den Fall einer Verwendung von Zudosierungspolygonen (vorzugsweise regelmäßig 12-eckig), die über den gesamten Winkelumfang von 360° gasdurchgängig sind, kommen beispielsweise auch die nachfolgenden Abmessungen in Betracht:

Länge Kante b: 160 mm;
Länge Kante c: 40 mm;
Wandstärke der Zudosierungskästen: 2 mm;
Abstand von radial aufeinanderfolgenden Zudosierungskästen (lichte Weite): 125 mm;
Innendurchmesser der Austrittsöffnungen A: 2,5 mm;
Abstand zweier auf der Perlenschnur benachbarter Austrittsöffnungen A (Mittelpunkt zu Mittelpunkt): einheitlich 60 mm;
Abstand der gedachten Schnur zu der Kante a, die dem Grundkreisring der Kreisringzylindereinheit zugewandt ist: 10 mm; und

Die Austrittsöffnungen A von einander radial gegenüberstehenden Rechteckflächen a x c von radial aufeinanderfolgenden Verteilerkästen stehen einander auf Lücke gegenüber.

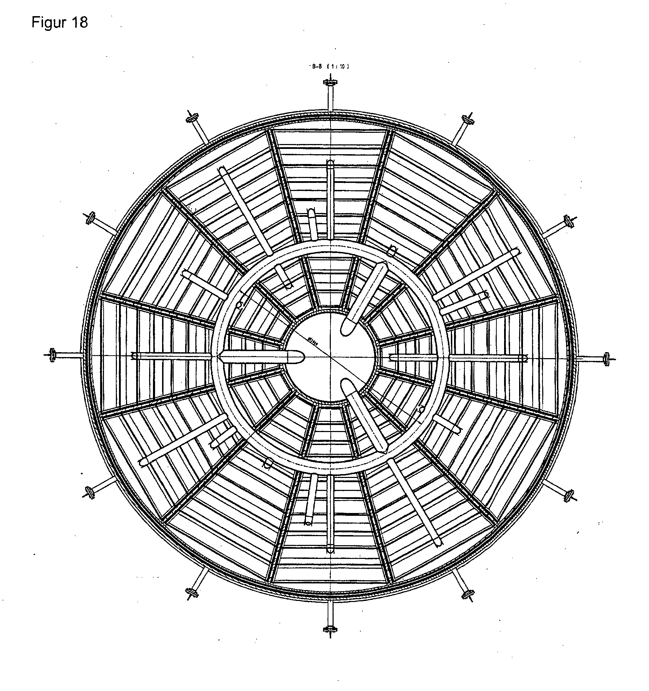

Die Versorgung der Zudosierungspolygone mit dem molekularen Sauerstoff enthaltenden Eingangsgas II erfolgt anwendungstechnisch zweckmäßig so, dass die Zudosierungsstellen für ein und dasselbe Polygon über den Umfang des Polygons gleichmäßig verteilt sind. Die Anzahl dieser Zudosierungsstellen für ein und dasselbe Polygon kann z.B. 2, oder 3, oder 4, oder 5, oder 6, oder 7, oder mehr betragen. Mit zunehmender Anzahl der Zudosierungsstellen vereinheitlicht sich der auf den individuellen Austrittsöffnungen A des Polygons stehende Druck. Figur 18 zeigt eine solche Zudosierungspolygonstruktur schematisch.

In einer alternativen Ausführungsform können die Zudosierungskästen über den Querschnitt des Kreisringzylindereinheitinnenraums anstelle sekantenförmig aber auch spinnennetzartig als konzentrische Polygone oder als konzentrische Kreise angeordnet sein. Die individuellen Polygone bzw. Kreise können dabei über den gesamten Winkelumfang von 360° durchgängig gefertigt (ein Zudosierungspolygon bzw. -kreis), oder in voneinander getrennte Zudosierungskästen unterteilt sein.

Die Anordnung der Zudosierungskästen ist in beiden Kreisringzylindereinheiten zweckmäßig deckungsgleich (kongruent) vorgenommen.

Die Austrittsöffnungen A befinden sich in den Rechteckflächen a x c der quaderförmigen Zudosierungskästen (Verteilungskästen). Sie sind alle kreisförmig und haben einen Innendurchmesser im Bereich von 2 bis 4 mm, mit Vorteil von 3 mm.

Die Austrittsöffnungen A befinden sich in beiden kongruenten Rechteckflächen a x c eines Zudosierungskastens (Verteilungskastens) wie auf einer Schnur hintereinander aufgereihte Perlen angeordnet, wobei die Schnur zu der Kante a, die dem Grundkreisring der Kreisringzylindereinheit zugewandt ist, einen Abstand von 5 mm aufweist und eine Parallele zu dieser Kante a bildet. Der Abstand von einer Austrittsöffnung A zur auf der "Perlenschnur" nächsten Austrittsöffnung A beträgt (Mittelpunkt zu Mittelpunkt gemessen) stets 100 mm (alternativ kann auch ein anderer einheitlicher Abstand im Bereich von 50 bis 100 mm angewendet werden). Die Gesamtzahl der Austrittsöffnungen A aller in einer Kreisringzylindereinheit angebrachten Zudosierungskästen (Verteilungskästen) beträgt beim beschriebenen Reaktor etwa 2000 (wird der Abstand zweier benachbarter Austrittsöffnungen A auf der Perlenschnur < 100 mm gewählt, kann die Gesamtzahl der Austrittsöffnungen A auch bis zu 10000 oder bis zu 5000 betragen). Die dem Grundkreisring der Kreisringzylindereinheit zugewandten Kanten a dürfen nicht aus dem Grundkreisring herausragen. Abschließend sei noch festzuhalten, dass dort, wo die die Zudosierungskästen (Verteilungskästen) auf Trennwände $W_T$ stossen, diese Trennwände $W_T$ entsprechende Durchtritte für die Zudosierungskästen (Verteilungskästen) aufweisen, wobei zwischen dem inneren Umfang der Durchtrittsöffnung und dem äußeren Umfang des Zudosierungskastens (Verteilungskastens) ein gasdurchgängiger Spalt von etwa 1 cm besteht. Die Austrittsöffnungen A von einander gegenüberstehenden Rechteckflächen a x c von benachbarten Rechteckkästen stehen einander auf Lücke gegenüber.

Grundsätzlich kann die Gesamtzahl der Austrittsöffnungen A, wie beschrieben, in den verschiedenen Kreisringzylindereinheiten identisch sein.

Sie können aber auch voneinander verschieden sein. D. h., diese Gesamtzahl kann in Strömungsrichtung des Reaktionsgases von Kreisringzylindereinheit zu Kreisringzylindereinheit zunehmend oder abnehmend sein.

Die Überwachung und Regelung einer erfindungsgemäßen heterogen katalysierten partiellen Dehydrierung wenigstens eines zu dehydrierenden Kohlenwasserstoffs im beschriebenen axialen Hordenreaktor erfolgt zweckmäßig über die Messung der in den verschiedenen katalytisch aktiven Schüttungen des jeweiligen Katalysatorfestbetts vorliegenden Temperaturen.

Die katalytisch aktive Schüttung aus Katalysatorformkörpern innerhalb eines Katalysatorfestbetts des vorstehend beschriebenen axialen Hordenreaktors weist in Strömungsrichtung des Reaktionsgases eine Schütthöhe von 300 mm (30 cm) auf (selbstredend kann der Hordenreaktor in völliger Entsprechung auch auf von 30 cm abweichende Schütthöhen (z.B. 40 cm) ausgelegt werden; 40 cm hohe Schütthöhen sind z.B. dann empfehlenswert, wenn als Katalysatorformkörper entsprechende Katalysatorstränge des Durchmessers 3 mm verwendet werden, die einen geringeren Druckverlust bedingen; die größere Schütthöhe zieht vorteilhaft eine Verkleinerung des Durchmessers des Kreisringzylindereinheiteninnenraums auf 5,47 m und eine damit einhergehende Katalysatorschüttungsausströmfläche von nur noch 21,6 m$^2$ nach sich).

In Strömungsrichtung des Reaktionsgases erfolgen auf nachfolgenden Schütthöhen Sh einer katalytisch aktiven Schüttung Temperaturmessungen:

0 cm;
7,5 cm;
15 cm;
22,5 cm; und
30 cm.

Weist die Schütthöhe der Schüttung aus den Katalysatorformkörpern einen von 300 mm verschiedenen Wert auf (z. B. 240 mm, oder 260 mm, oder 280 mm) erfolgen die Temperaturmessungen auf über diese Schütthöhe in entsprechender Weise äquidistant verteilten fünf Höhen.

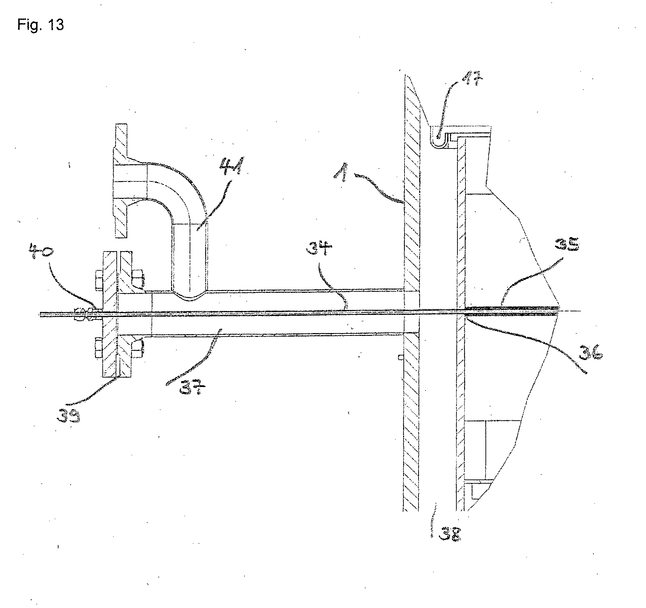

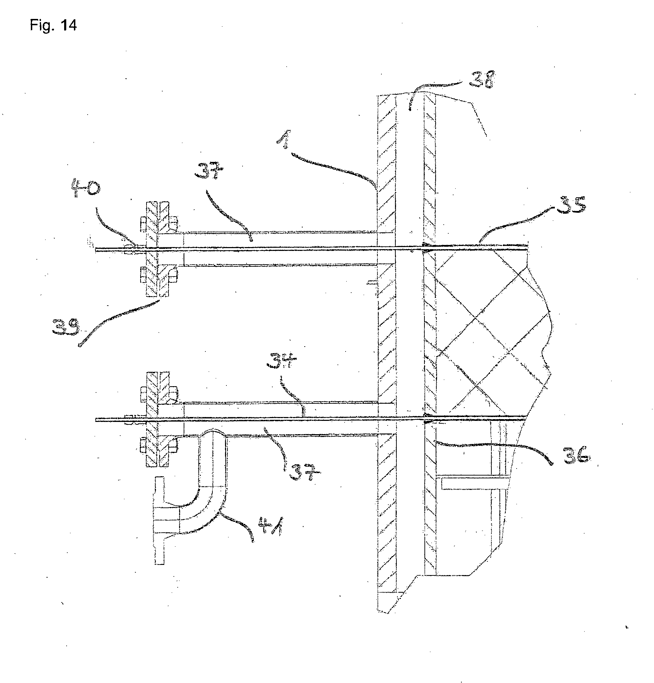

Zu diesem Zweck sind senkrecht zur Achse der entsprechenden Kreisringzylindereinheit (d. h. radial horizontal) durch den Mantel (1) des geraden Kreiszylinders sowie durch den äußeren Mantel der Kreisringzylindereinheit hindurch auf jeder der vorgenannten Höhen Sh drei Führungsfinger (34) zur Aufnahme je eines 3-fach Thermoelementes (das 3-fach Thermoelement greift simultan die Temperatur auf drei unterschiedlichen (in der Regel äquidistanten) Einführungstiefen in das Katalysatorfestbett die Temperatur ab) in die katalytisch aktive Schüttung hineingeführt (vgl. Figur 13). Der einzelne Führungsfinger ragt bis zum inneren Mantel der Kreisringzylindereinheit und ist gegenüber dem Katalysatorfestbett abgeschlossen. D. h., das Innere des jeweiligen Führungsfingers kommt nicht mit Reaktionsgas in Berührung. Der Innendurchmesser eines Führungsfingers liegt im Bereich 3 bis 5 mm und der Außendurchmesser eines Führungsfingers liegt im Bereich 6 bis 8 mm. Die drei auf ein und derselben Schütthöhe Sh eingebauten Führungsfinger (34) sind dabei so am Umfang des Mantels (1) verteilt, dass längs des Umfangs aufeinanderfolgende Führungsfinger einen Winkel von 120° einschließen, wenn man sie gedanklich so lange verlängert, bis sie sich schneiden. Bei in aufeinanderfolgenden Schütthöhen Sh befindlichen Führungsfingern stehen die in Strömungsrichtung nachfolgenden drei Führungsfinger zu den vorhergehenden drei Führungsfingern nicht auf Deckung sondern um 90° versetzt. Der Drehsinn der vorgenannten Versetzung (im Uhrzeigersinn oder entgegengesetzt zum Uhrzeigersinn) wird dabei über die katalytisch aktive Schüttung beibehalten, so dass die Führungsfinger bei Sh = 0 cm und bei Sh = 30 cm auf Deckung stehen, wie in Fig. 14 gezeigt.

Die auf den Schütthöhen Sh = 0 cm und Sh = 30 cm ermittelten Temperaturen werden herangezogen, um die Zudosierung des Eingangsgases II vorab desjenigen Katalysatorfestbetts, in welchem die Temperaturmessung erfolgt, einzustellen. Die Änderung des molaren Verhältnisses von zu dehydrierendem Kohlenwasserstoff zu dehydriertem Kohlenwasserstoff zwischen Eintritt des Reaktionsgases in den Gaseintrittsrohrstutzen (4) und Austritt des Reaktionsgases aus dem Gasaustrittsrohrstutzen (5) liefert den auf einmaligen Durchgang durch den Reaktor bezogenen resultierenden Dehydrierumsatz. Die Eindüsung der Eingangsgase II erfolgt so, dass der angestrebte Dehydrierumsatz erreicht wird.

Die detaillierte Ausgestaltung der Einführung eines Führungsfingers (34) zeigen die Figuren 13 und 14 im Längsschnitt.

Dabei wird ersichtlich, dass der Führungsfinger (34) innerhalb des Katalysatorfestbetts in einem (ebenfalls geschlossenen) Schutzfinger (35) geführt wird, der einen Innendurchmesser im Bereich von 4 bis 7 mm und einen Außendurchmesser im Bereich von 6 bis 9 mm aufweist. Der Spalt zwischen Führungsfingeraußenwand und Schutzfingerinnenwand ist möglichst gering. Gegebenenfalls wird ein verbliebener Spalt mit Hochtemperaturwärmeleitfähigkeitspaste gefüllt. Dieser Schutzfinger ist auf der Innenwand (36) des äußeren Mantels der Kreisringzylindereinheit aufsitzend gasdicht angeschweißt, so dass an dieser Stelle kein Reaktionsgas in den Spalt (38) zwischen Mantel (1) und äußerem Mantel der Kreisringzylindereinheit gelangen kann. Außerhalb des Mantels (1) ist der Führungsfinger auf der Symmetrieachse eines Spülrohres (37) geführt, dessen Innendurchmesser ca. 45 mm und dessen Außendurchmesser 50,8 mm beträgt. Die Länge eines Führungsfingers beträgt 2000 mm und die Länge des Spülrohres beträgt 400 mm. Das Spülrohr ist mit Hilfe eines Flansches (39) gasdicht verschlossen, in den der Führungsfinger über eine Klemmringdichtung (40) gasdicht eingeführt ist.

Auf der Aussenseite des Mantels (1) ist das Spülrohr gasdicht angeschweißt. Der Durchgang des Führungsfingers (34) durch den Mantel (1) erfolgt nicht gasdicht. Über einen am Spülrohr angeschweißten Stutzen (41) wird ein Inertgas (vorzugsweise molekularer Stickstoff, z. B. Industriestrickstoff mit bis zu 1 Vol.-% $O_2$, Wasserdampf oder deren Gemische) zugeführt, das am Durchgang des Führungsfingers (34) durch den Mantel (1) Zugang zum Spalt zwischen Mantel (1) und äußerem Mantel der Kreisringzylindereinheit hat (einem Teil der Spülrohre wird das Inertgas indirekt über den Spalt (38) zugeführt). Figur 13 zeigt außerdem die Schweißlippendichtung 17.

Diese Inertgasströme werden in ihrer Gesamtmenge in der Regel weniger als 1 Vol.-%, meist sogar weniger als 0,1 oder weniger als 0,01 Vol.-%, bezogen auf den Volumenstrom des Reaktionsgases (in Nm$^3$), betragen.

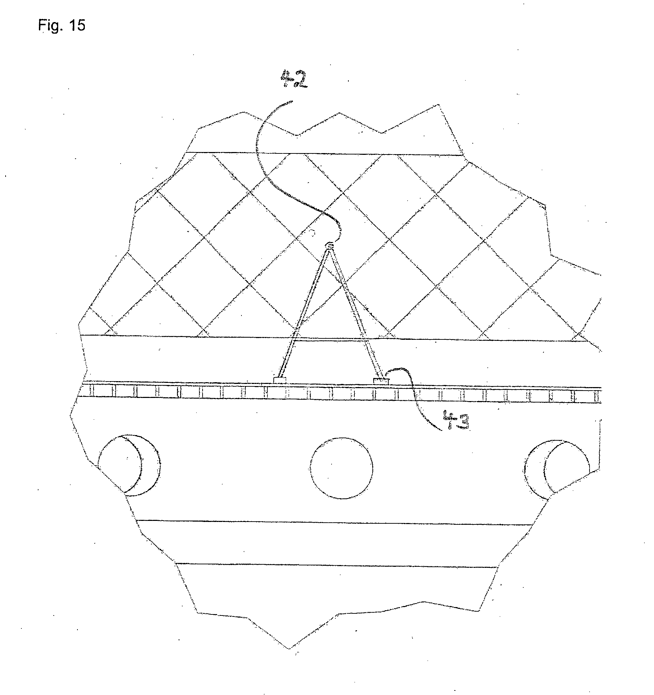

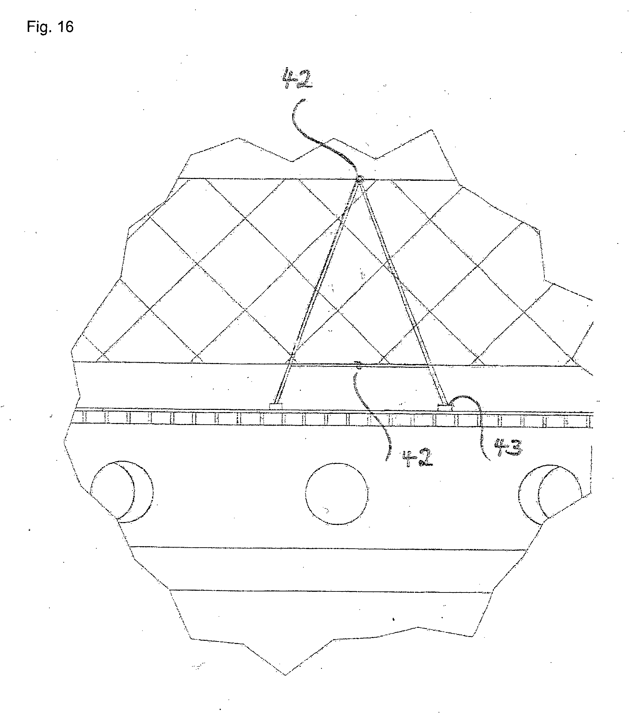

Die Figuren 15 und 16 zeigen zwei durch eine Querstrebe stabilisierte Zweibeine im Längsschnitt. Sie bestehen aus Röhrchen mit einem Außendurchmesser von 6 mm und einer Wanddicke von 1 mm. Die die beiden Schenkel bildenden Röhrchen sind in Auflagequader (43) eingelassen (30 mm x 10 mm x 20 mm (Breite x Höhe x Tiefe)).

Dort, wo die Schenkel zusammenlaufen sowie gegebenenfalls in der Mitte der Querstrebe, sind senkrecht zur Schnittebene kurze Hülsen (42) eingelassen.

Mehrere dieser Zweibeine werden vor der Aufschüttung des Katalysatorfestbetts auf den Gitterrostsektor gestellt. Die Schutzfinger (35) werden dann durch vorgenannte Hülsen (42) geführt, um die horizontale Führung der Führungsfinger für die Thermoelemente zu stabilisieren und so deren Durchhängen entgegenzuwirken.

Die auf Deckung stehenden Führungsfinder (Sh = 0 cm, Sh = 30 cm) können durch ein gemeinsames Zweibein gemäß Figur 16 gestützt werden.

Die Adiabasie des beschriebenen axialen Hordenreaktors kann in vorteilhafter Weise zusätzlich noch dadurch verbessert werden, dass man auf die Außenseite der Druckbehälterwand zur thermischen Isolierung entsprechende Isoliermaterialien (z. B. Foamglas oder Mineralwolle) aufbringt (z. B. in einer Schichtdicke von 300 bis 500 mm). Das Aufbringen kann z. B. durch Halterungen erfolgen, die an der Außenwand befestigt (z. B. angeschweißt) sind.

Die vorab der Beschreibung des Design des als axialen Hordenreaktor ausgeführten Schachtreaktor angesprochene heterogen katalysierte partielle Dehydrierung von Propan zu Propylen kann in diesem axialen Hordenreaktor z. B. wie folgt durchgeführt werden.

Über den Gaseintrittsrohrstutzen werden dem Hordenreaktor 116306 Nm$^3$/h eines Reaktionsgases zugeführt, das folgende Gehalte aufweist (die Inertgasströme an $N_2$ in die Reaktorhaube und in die Spülrohre (27) werden vernachlässigt, da ihre Menge insgesamt < 0,01 Vol.-%, bezogen auf die Reaktionsgasmenge (in Nm$^3$), beträgt):

| | |
|---|---|
| Propan | 34,44 Vol.-%, |
| Propylen | 0,2 Vol.-%, |
| $N_2$ | 52,8 Vol.-%, |
| $O_2$ | 3,04 Vol.-%, |
| $CO_2$ | 1,61 Vol.-%, |
| CO | 0,39 Vol.-%, |
| $H_2$ | 0,09 Vol.-%, |
| Ethan | 0,12 Vol.-%, |
| $H_2O$ | 7,22 Vol.-%, |
| iso-Buten | 0,01 Vol.-%, |
| Acrylsäure | 0,04 Vol.-%, |
| Acrolein | 0,02 Vol.-%, und |
| Essigsäure | 0,02 Vol.-%. |

Das Reaktionsgas weist eine Temperatur von 394 °C und einen Eingangsdruck von 3,10 bar auf.

Als Eingangsgas II 1 werden in Strömungsrichtung des Reaktionsgases zwischen dem ersten und dem zweiten Katalysatorfestbett über die Austrittsöffnungen A der entsprechenden Verteilungskästen 2930 Nm$^3$/h eines Gemischs aus Luft und Wasserdampf zudosiert, das folgende Gehalte aufweist:

| | |
|---|---|
| Wasser | 34,9 Vol.-%, |
| $N_2$ | 51,4 Vol.-%, und |
| $O_2$ | 13,7 Vol.-%. |

Die Temperatur des Eingangsgases II beträgt 450 °C und sein Druck beträgt 2,9 bar.

Als Eingangsgas II 2 werden in Strömungsrichtung des Reaktionsgases zwischen dem zweiten und dem dritten Katalysatorfestbett über die Austrittsöffnungen A der entsprechenden Verteilungskästen 2600 Nm$^3$/h eines Gemischs aus Luft und Wasserdampf zudosiert, das folgende Gehalte aufweist:

| | |
|---|---|
| Wasser | 54,33 Vol-%, |
| $N_2$ | 36,09 Vol.-%, und |
| $O_2$ | 9,58 Vol.-%. |

Die Temperatur des Eingangsgases II 2 beträgt 450 °C und sein Druck beträgt 2,8 bar. Über den Gasaustrittsrohrstutzen (5) verlässt ein Produktgasgemisch in einer Menge von 120754 Nm$^3$/h den Hordenreaktor, das folgende Gehalte aufweist:

| | |
|---|---|
| Propan | 24,95 Vol.-%, |
| Propylen | 5,77 Vol.-%, |
| $N_2$ | 49,865 Vol.-%, |
| $O_2$ | 0 Vol.-%, |
| $CO_2$ | 3,03 Vol.-%, |
| CO | 0,35 Vol.-%, |
| $H_2$ | 4,13 Vol.-%, |
| Methan | 0,01 Vol.-%, |

(fortgesetzt)

| | |
|---|---|
| Ethan | 0,11 Vol.-%, |
| Ethylen | 0,01 Vol.-%, |
| $H_2O$ | 11,98 Vol.-%, und |
| Acrylsäure | 0,01 Vol.-%. |

Die Temperatur des Produktgasgemischs beträgt 501 °C und der Druck des Produktgasgemischs beträgt 2,71 bar.

Als Eingangsgasstrom I 1 strömt aus dem in Strömungsrichtung des Reaktionsgases ersten Katalysatorfestbett in einer Menge von 120754 Nm$^3$/h (dem entspricht eine Strömungsgeschwindigkeit W1 von 1,1 m/s) ein Gasgemisch, das folgende Gehalte aufweist:

| | |
|---|---|
| Propan | 30,16 Vol.-%, |
| Propylen | 3,03 Vol.-%, |
| $N_2$ | 51,03 Vol.-%, |
| $O_2$ | 0 Vol.%, |
| $CO_2$ | 2,36 Vol.-%, |
| CO | 0,372 Vol.-%, |
| $H_2$ | 2,14 Vol.-%, |
| Methan | 0,001 Vol.-%, |
| Ethan | 0, 116 Vol.-%, |
| Ethylen | 0,004 Vol.-%, |
| $H_2O$ | 9,75 Vol.-%, und |
| Acrylsäure | 0,022 Vol.-%. |

Die Temperatur des Eingangsstroms I beträgt 496 °C und sein Druck beträgt 2,9 bar.

Als Eingangsgasstrom I 2 strömt aus dem in Strömungsrichtung des Reaktionsgases zweiten Katalysatorfestbett in einer Menge von 127180 Nm$^3$/h (dem entspricht eine Strömungsgeschwindigkeit von 1,33 m/s) ein Gasgemisch, das folgende Gehalte aufweist:

| | |
|---|---|
| Propan | 26,85 Vol.-%, |
| Propylen | 4,65 Vol.-%, |
| $N_2$ | 50,29 Vol.-%, |
| $O_2$ | 0 Vol.-%, |
| $CO_2$ | 2,75 Vol.-%, |
| CO | 0,359 Vol.-%, |
| $H_2$ | 3,34 Vol.-%, |
| Methan | 0,002 Vol.-%, |
| Ethan | 0,113 Vol.-%, |
| Ethylen | 0,005 Vol.-%, |
| $H_2O$ | 11,13 Vol.-%, und |
| Acrylsäure | 0,013 Vol.-%. |

Die Temperatur des Eingangsgasstroms I 2 beträgt 499 °C und sein Druck beträgt 2,8 bar.

Die Richtungen aller aus den Austrittsöffnungen A 1 (das sind die Austrittsöffnungen A in den Verteilungskästen für das Eingangsgas II 1 der in Strömungsrichtung des Reaktionsgases ersten Kreisringzylindereinheit) in fiktiver Abwesenheit des Eingangsgasstroms I 1 austretenden individuellen Eingangsgas II 1-ströme schließen mit der Strömungsrichtung das Eingangsgasstroms I 1 einen Winkel α von 90° ein.

Die Richtungen aller aus den Austrittsöffnungen A 2 (das sind die Austrittsöffnungen A in den Verteilungskästen für das Eingangsgas II 2 der in Strömungsrichtung des ersten Reaktionsgases zweiten Kreisringzylindereinheit) in fiktiver Abwesenheit des Eingangsstroms I 2 austretenden individuellen Eingangsgas II 2-ströme schließen mit der Strömungsrichtung des Eingangsstroms I 2 einen Winkel α von 90° ein.

Die Induktionszeit J 1 des Reaktionsgaseingangsgemischs 1 (wird in die in Strömungsrichtung des Reaktionsgases zweite Kreisringzylindereinheit geführt) beträgt 0,735 Sekunden.

Erhöht man die Temperatur des Reaktionsgaseingangsgemischs 1 auf 550 °C, fällt die Induktionszeit auf 0,15 Sekunden.

In entsprechender Weise wie eine Temperaturerhöhung verkürzt auch eine Druckerhöhung die Induktionszeit J.

Die Induktionszeit J 2 des Reaktionsgaseingangsgemischs 2 (wird in die in Strömungsrichtung des Reaktionsgases dritte Kreisringzylindereinheit geführt) beträgt 0,761 Sekunden.

Erhöht man die Temperatur des Reaktionsgaseingangsgemischs 2 auf 550 °C, fällt die Induktionszeit auf 0,15 Sekunden.

Die Entfernung D1 aller Austrittsöffnungen A1 vom relevanten Katalysatorfestbett beträgt 5 mm.

Die Entfernung D2 aller Austrittsöffnungen A2 vom relevanten Katalysatorfestbett beträgt 5 mm.

In alternativen Ausführungsformen können D 1 und D 2 aber jeweils auch auf jeden anderen Wert im Bereich von 5 mm bis 10 mm einheitlich eingestellt werden.

Bei einer Projektion aller Austrittsöffnungen A1 in Strömungsrichtung des Eingangsgasstroms I 1 in die Projektionsebene E1 senkrecht zur Strömungsrichtung des Eingangsgasstroms I beträgt die Anzahl ZA1 der in der vom Eingangsstrom I 1 erfassten Projektionsfläche in einem beliebigen $m^2$ befindlichen Austrittsöffnungsschwerpunkte $\geq$ 50.

Der Abstand von einem Austrittsöffnungsschwerpunkt zum nächstliegenden Austrittsöffnungsschwerpunkt beträgt dabei in der Projektionsebene nicht mehr als 100 mm.

Bei einer Projektion aller Austrittsöffnungen A2 in Strömungsrichtung des Eingangsstroms I 2 in die Projektionsebene E 2 senkrecht zur Strömungsrichtung des Eingangsstroms I 2 beträgt die Anzahl ZA2 der in der vom Eingangsgasstrom I 2 erfassten Projektionsfläche in einem beliebigen $m^2$ befindlichen Austrittsöffnungsschwerpunkte $\geq$ 50.

Der Abstand von einem Austrittsöffnungsschwerpunkt zum nächstliegenden Austrittsöffnungsschwerpunkt beträgt dabei in der Projektionsebene nicht mehr als 100 mm.

Beim Durchgang des Reaktionsgasgemischeingangsstroms 1 durch das in Strömungsrichtung des Reaktionsgases in der zweiten Kreisringzylindereinheit befindliche Katalysatorfestbett, werden 6,19 mol-% des im Reaktionsgasgemischeingangsstroms 1 enthaltenen Propan zu Propylen umgesetzt.

Beim Durchgang des Reaktionsgasgemischeingangsstroms 2 durch das in Strömungsrichtung des Reaktionsgases in der dritten Kreiszylindereinheit befindliche Katalysatorfestbett, werden 3,09 mol-% des im Reaktionsgasgemischeingangsstrom 2 enthaltenen Propan zu Propylen umgesetzt.

Das über den Gasaustrittsrohrstutzen (5) den Hordenreaktor verlassende Produktgasgemisch kann nun in an sich bekannter Weise verwendet werden, um das darin enthaltene Propylen in Begleitung des darin enthaltenen Propan in einer nachgeschalteten heterogen katalysierten Partialoxidation zu Acrolein und/oder Acrylsäure als Zielprodukt partiell zu oxidieren.

Dabei kann z. B. wie in den Schriften DE-A 10 2005 061 626, DE-A 10 2005 057 197, DE-A 10 2005 052 923, DE-A 10 2005 052 917, DE-A 10 2005 022 798, DE-A 10 2005 009 885, DE-A 10 2005 010 111, DE-A 10 2004 032 129, DE-A 10 2005 013 039, WO 03/076370, DE-A 102 11 275, WO 01/96270, DE-A 10 2005 056 377 und dem in diesen Schriften zitierten Stand der Technik vorgegangen werden. Insbesondere kann wie in der DE-A 10 2004 032 129 und wie in der DE-A 10 2005 013 039 oder wie in der DE-A 10 2005 022 798 beschrieben vorgegangen werden.

Aus dem Produktgasgemisch der Partialoxidation wird anschließend normalerweise Zielprodukt abgetrennt und aus dem dabei verbleibenden Restgas wenigstens eine Propan und gegebenenfalls nicht umgesetztes Propylen enthaltende Teilmenge zur Erzeugung des über den Gaseintrittsrohrstutzen des axialen Hordenreaktors zugeführten Reaktionsgases in die heterogen katalysierte partielle Dehydrierung rückgeführt.

In dem Vorstehenden entsprechender Weise wurden die dem über den Gaseintrittsrohrstutzen des beschriebenen axialen Hordenreaktors zugeführten 116309 $Nm^3$/h an Reaktionsgas erzeugt, durch Zusammenführen (z. B. wie in Figur 10 der WO 2004/067164) von 8063 $Nm^3$/h an Roh-Propan der Spezifikation

| | |
|---|---|
| Propan | $\geq$ 98,0 Vol.-%, |
| Propylen | $\leq$ 0,11 Vol.-%, |
| n-Butan | < 0,001 Vol.-%, |
| iso-Butan | < 0,05 Vol.-%, |
| Ethan | < 1,5 Vol.-%, |
| Ethylen | < 0,02 Vol.-%., und |
| sonstige $C_4$-Koh-lenwasserstoffe | < 0,001 Vol.-%, |

der Temperatur 20 °C sowie dem Druck 4 bar und 102961 $Nm^3$/h Restgas einer solchen Partialoxidation, das bei einer Temperatur von 104 °C und einem Druck von 3,3 bar folgende Gehalte aufweist:

| Propan | 31,18 Vol.-%, |
| Propylen | 0,215 Vol.-%, |
| $N_2$ | 59,69 Vol.-%, |
| $O_2$ | 3,44 Vol.-%, |
| $CO_2$ | 1,81 Vol.-%, |
| CO | 0,444 Vol.-%, |
| $H_2$ | 0,098 Vol.-%, |
| Ethan | 0,06 Vol.-%, |
| Ethylen | 0,003 Vol.-%, |
| $H_2O$ | 2,96 Vol.-%, |
| Acrylsäure | 0,04 Vol.-%, |
| Acrolein | 0,03 Vol.-%, und |
| Essigsäure | 0,03 Vol.-%. |

Zur Zusammenführung der beiden Gasströme werden diese z. B. in einem in die Rohrleitung eingebauten statischen Mischer miteinander intensiv vermischt.

Vorteilhaft ist es, wenn das vorgenannte Zusammenführen und der Zeitpunkt der Zufuhr in den Gaseintrittsrohrstutzen möglichst zeitnah erfolgen.

Beim Zusammenführen entsteht zunächst ein Gasgemisch mit P = 3,21 bar und T = 95,7 °C. Durch indirekten Wärmeaustausch mit dem den Gasaustrittsrohrstutzen (S) verlassenden Produktgasgemisch wird es auf die Temperatur 394 °C und den Eingangsdruck von 3,10 bar gebracht.

Die Abtrennung des Zielproduktes aus dem bei der heterogen katalysierten Gasphasen-Partialoxidation des Propylens zu Acrolein und/oder Acrylsäure anfallenden Produktgasgemischs kann in wenigstens einem Abtrennschritt in an sich bekannter Weise erfolgen. Dabei wird normalerweise so vorgegangen, dass man das wenigstens eine Zielprodukt in einem Grundabtrennschritt aus der Gasphase in die flüssige Phase überführt (vorab wird das Produktgasgemisch gegebenenfalls abgekühlt). Dies kann z. B. durch partielle oder vollständige sowie gegebenenfalls fraktionierende Kondensation des Zielprodukts (z. B. Acrolein und/oder Acrylsäure) und/oder durch Absorption des wenigstens einen Zielproduktes aus dem Produktgasgemisch in ein wässriges oder organisches Lösungsmittel erfolgen (d.h., fraktionierende Kondensation und/oder Absorption mit Wasser bzw. wässriger Lösung können auch überlagert angewendet werden). Fraktionierende Kondensation und/oder Absorption in Wasser bzw. in wässrige Lösungen sind als Grundabtrennschritt generell bevorzugt. Im Fall von Acrylsäure und/oder Acrolein als Zielprodukt kommen als geeignete Absorptionsmittel beispielsweise Wasser, wässrige Lösungen niederer Carbonsäuren sowie hydrophobe organische Lösungsmittel wie Gemische aus Diphenyl und Diphenylether (z. B. Diphyl®) oder Gemische aus Diphyl (75 bis 99,9 Gew.-%) und Dimehtylphthalat (0,1 bis 25 Gew.-%) in Betracht. Im Fall von Acrylsäure wird man das das Zielprodukt enthaltende Produktgasgemisch bevorzugt fraktionierend kondensieren. Beispielsweise kann die Grundabtrennung (insbesondere im Fall von Acrylsäure) wie in den nachfolgenden Schriften beschrieben erfolgen (vgl. z. B. EP-A 1 388 533, EP-A 1 388 532, DE-A 102 35 847, EP-A 792 867, WO 98/01415, EP-A 10 15 411, EP-A 10 15 410, WO 99/50219, WO 00/53560, WO 02/09839, DE-A 102 35 847, WO 03/041833, DE-A 102 23 058, DE-A 102 43 625, DE-A 103 36 386, EP-A 854 129, US-A 4,317,926, DE-A 198 37 520, DE-A 196 06 877, DE-A 190 50 1325, DE-A 102 47 240, DE-A 197 40 253, EP-A 695 736, EP-A 982 287, EP-A 10 41 062, EP-A 11 71 46, DE-A 43 08 087, DE-A 43 35 172, DE-A 44 36 243, DE-A 199 24 532, DE-A 103 32 758 sowie DE-A 199 24 533). Eine Acrylsäureabtrennung kann auch wie in der EP-A 982 287, der EP-A 982 289, der DE-A 103 36 386, der DE-A 101 15 277, der DE-A 196 06 877, der DE-A 197 40 252, der DE-A 196 27 847, der EP-A 920 408, der EP-A 10 68 174, der EP-A 10 66 239, der EP-A 10 66 240, der WO 00/53560, der WO 00/53561, der DE-A 100 53 086 und der EP-A 982 288 vorgenommen werden. Vorzugsweise wird wie in Fig. 7 der WO/0196271 bzw. wie in der DE-A 10 2004 032 129 und deren äquivalenten Schutzrechten beschrieben abgetrennt. Günstige Abtrennweisen sind auch die in den Schriften WO 2004/063138, WO 2004/035514, DE-A 102 43 625 und DE-A 102 35 847 beschriebenen Verfahren. Die Weiterverarbeitung einer dabei gewonnenen rohen Acrylsäure kann z. B. wie in den Schriften WO 01/77056, WO 03/041832, WO 02/055469, WO 03/078378 und WO 03/041833 beschrieben erfolgen.

Gemeinsames Merkmal der vorgenannten Trennverfahren ist, dass z. B. am Kopf der z. B. jeweiligen trennwirksame Einbauten enthaltenden Trennkolonne, in deren unteren Teil das das wenigstens ein Zielprodukt enthaltende Produktgasgemisch, normalerweise nach vorheriger direkter und/oder indirekter Kühlung desselben, zugeführt wird, normalerweise ein Restgasstrom verbleibt, der im wesentlichen diejenigen Bestandteile des Produktgasgemischs enthält, deren Siedepunkt bei Normaldruck (1 bar) ≤ -30°C beträgt (d. h., die schwer kondensierbaren oder auch leicht flüchtigen Bestandteile).

Im unteren Teil der Trennkolonne fallen normalerweise die schwerer flüchtigen Bestandteile des Produktgasgemischs, einschließlich des jeweiligen wenigstens einen Zielprodukts und der zum Zielprodukt ähnlich flüchtigen Nebenkomponenten, in kondensierter Phase an.

Die Restgasbestandteile sind normalerweise in erster Linie Propan, gegebenenfalls in der Partialoxidation nicht umgesetztes Propylen, molekularer Sauerstoff sowie häufig die in der Partialoxidation sonstigen mitverwendeten inerten Verdünnungsgase, wie z. B. Stickstoff und Kohlendioxid. Wasserdampf kann je nach angewandtem Trennverfahren im Restgas nur noch in Spuren oder in Mengen von bis zu 20 Vol.-% oder mehr enthalten sein. Mit diesem Restgas kann (und wird in der Regel) wie beschrieben verfahren werden.

An dieser Stelle sei nochmals festgehalten, dass die Grundabtrennung von Acrylsäure aus einem wie beschrieben erhaltenen, Acrylsäure als Zielprodukt enthaltenden, Partialoxidationsproduktgasgemisch bevorzugt so erfolgt, dass man das zuvor gegebenenfalls durch direkte und/oder indirekte Kühlung abgekühlte Produktgasgemisch in einer trennwirksame Einbauten enthaltenden Kolonne unter Seitenabzug einer rohen Acrylsäure (z. B. in sich selbst) aufsteigend fraktionierend kondensiert und/oder mit Wasser bzw. wässriger Lösung absorbiert, wie es die WO 2004/035514 und die DE-A 102 43 625 beispielhaft beschreiben. Die entnommene rohe Acrylsäure wird nachfolgend bevorzugt einer Suspensionskristallisation unterworfen und das dabei gebildete Acrylsäuresuspensionskristallisat bevorzugt mittels einer Waschkolonne von verbliebener Mutterlauge abgetrennt. Mit Vorteil wird dabei als Waschflüssigkeit die Schmelze von in der Waschkolonne vorab abgetrennten Acrylsäurekristallen verwendet. Ferner ist die Waschkolonne bevorzugt eine solche mit erzwungenem Transport des Kristallbetts. Besonders bevorzugt handelt es sich um eine hydraulische (z. B. um eine TNO Waschkolonne) oder um eine mechanische Waschkolonne. Im einzelnen kann der Beschreibung der WO 01/77056, der WO 03/041832 sowie der WO 03/041833 gefolgt werden. D. h., vorzugsweise wird verbliebene Mutterlauge in die fraktionierende Kondensation rückgeführt (vgl. auch EP-A 10 15 410). Der Nebenkomponentenauslass befindet sich normalerweise unterhalb des Seitenabzugs der rohen Acrylsäure als purge-Strom.

Unter Anwendung von lediglich einer Kristallisationsstufe ist so Acrylsäure mit einer Reinheit ≥ 99,8 Gew.-% erhältlich, die sich in hervorragender Weise zur Herstellung von Superabsorbern auf Basis von Poly-Na-Acrylat eignet.

Im übrigen gilt auch für das erfindungsgemäße Verfahren das in der DE-A 102 45 585 sowie in der DE-A 102 46 119 gesetzte Anforderungsprofil.

Erfindungsgemäße Verfahren sind generell auch solche, bei denen sich an das erfindungsgemäße Verfahren der heterogen katalysierten partiellen Dehydrierung wenigstens eine heterogen katalysierte partielle Oxidation des wenigstens einen dehydrierten Kohlenwasserstoffs anschließt.

Die Figuren 1 bis 17 zeigen folgendes:

| | |
|---|---|
| Figur 1: | Draufsicht auf einen Längsschnitt eines als Hordenreaktor ausgeführten erfindungsgemäß geeigneten Schachtreaktors |
| Figur 2: | Vorderansicht eines Längsschnitts eines als Hordenreaktor ausgeführten erfindungsgemäß geeigneten Schachtreaktors |
| Figur 3: | Draufsicht auf eine Kreisringzylindereinheit unter Ausblendung des Gitterrostes |
| Figur 4: | Draufsicht auf eine Kreisringzylindereinheit mit Gitterrost und auf diesem aufgebrachtes Netzwerk aus Metalldraht |
| Figur 5: | Flanschverbindung zwischen Kreiszylindermantel (1) und oberer Reaktorhaube |
| Figur 6: | Schütthilfe |
| Figur 7: | Schweißlippendichtung zwischen Kreiszylindermantel (1) und äußerem Mantel einer Kreisringzylindereinheit auf der Höhe des Deckkreisrings |
| Figur 8: | Dreifach gestückelter Übergang eines Eingangsgas II zuführenden Rohres in den Eingangsstutzen der oberen Reaktorhaube |
| Figur 9: | Zweigeteilter Übergang eines Eingangsgas II zuführenden Rohres in den Eingangsstutzen der oberen Reaktorhaube |
| Figur 10: | Einmündung von Verteilungsrohren in Zudosierungskästen |
| Figur 11: | Seitenansicht einer formschlüssig ausgeführten Halterung für einen Verteilungskasten |
| Figur 12: | Draufsicht der in Figur 11 in Seitenansicht gezeigten Halterung |
| Figur 13: | Einführung eines Thermoelements in ein Katalysatorfestbett |
| Figur 14: | Einführungen zweier im Katalysatorfestbett übereinander geführten Thermoelemente, die auf Deckung stehen |
| Figur 15: | Zweibein zur Stützung eines Thermoelementführungsfingers |
| Figur 16: | Zweibein zur Stützung der Führungsfinger zweier im Katalysatorfestbett übereinander geführter Thermoelemente die auf Deckung stehen |
| Figur 17: | Schachtreaktornachahmung |

Beispiel

Verwendet wurde eine quaderförmige (Schacht)Reaktornachahmung (44). Der Innenraum des Quaders wies bei einer Wandstärke von 3 mm (alle Elemente der Reaktornachahmung waren aus Edelstahl 1.4541 gefertigt) folgende Dimensionen auf. Die Grundfläche betrug 840 mm (Kante a) x 900 mm (Kante b). Die Höhe (Kante c) der quaderförmigen Reaktornachahmung betrug 1200 mm.

60 cm oberhalb der Grundfläche der Reaktornachahmung (die Maßangabe bezieht sich auf den Abstand von der Grundfläche bis zur Oberkante a* der Zudosierungskästen (Verteilungskästen)) waren im Innenraum der Reaktornachahmung mit ihrer Längstkante b* parallel zur Kante b drei identische, ebenfalls quaderförmige, Zudosierungskästen (45) mit dem rechteckigen Querschnitt a* x c* angebracht. Die Wanddicke dieser Zudosierungskästen betrug 2 mm. Die Kanten a* waren parallel zur Kante a und wiesen eine Länge von 15 cm auf. Die Kanten c* waren parallel zur Kante c und wiesen eine Länge von 4 cm auf. Der Abstand zwischen zwei Zudosierungskästen betrug 13 cm. Der mittlere der drei Verteilungskästen befand sich mit seiner Kante a* auf der Quaderbreite a = 840 mm zentriert angeordnet. Die Kante b* hatte eine Länge von 900 mm. Die Verteilungskästen waren mit ihren Begrenzungsflächen a* x c* jeweils an den Begrenzungsflächen a x c angeschweißt. Oben war die quaderförmige Reaktornachahmung offen (d.h. sie wies keine Decke auf), unten und an den vier Seiten geschlossen.

In den beiden Rechteckflächen c* x b* eines Zudosierungskastens befanden sich kreisrunde Austrittsöffnungen A (46) mit dem Durchmesser 3 mm wie auf einer Schnur hintereinander aufgereihte Perlen angeordnet, wobei die Schnur zur Kante b*, die der Grundfläche a x b zugewandt ist, einen Abstand von 5 mm aufwies und eine Parallele zu dieser Kante bildete.

Der Abstand von einer Austrittsöffnung A zur auf der "Perlenschnur" nächsten Austrittsöffnung A betrug (Mittelpunkt zu Mittelpunkt gemessen) 10 cm. Die Austrittsöffnungen A von einander gegenüber stehenden Rechteckflächen c* x b* von benachbarten Rechteckkästen standen einander auf Lücke gegenüber.

Auf den Zudosierungskästen (45) lag (über den Querschnitt a x b) ein Lochblech (47) auf, wobei die Blechdicke 3 mm betrug. Die Form der Löcher war gleichförmig oval. Ihre Querausdehnung betrug 3 mm und ihre Längsausdehnung betrug 1,5 cm. Das Öffnungsverhältnis des Lochblechs lag bei 30 % (Öffnungsverhältnis % = (Summe der durchlässigen Flächen/Gesamtfläche) x 100). Die Lochmittelpunkte waren gemäß einer quadratischen Teilung gleichförmig verteilt.

Auf dem Lochblech war eine Inertschüttung (48) der Schütthöhe 20 cm aus Steatitringen 7 mm x 7 mm x 4 mm (Außendurchmesser x Höhe x Innendurchmesser) aufgeschüttet. Auf diese Inertschüttung wurde ein gleichmäßiges Metallnetz aufgelegt, das aus längs- und querliegenden Metalldrähten aufgebaut war.

Der Abstand zwischen zwei nächstliegenden parallelen Metalldrähten betrug 2 mm. Auf dieses Metallnetz war ein Gitterrost (49) aufgelegt, der sich ebenso wie das Metallnetz über den gesamten Querschnitt a x b erstreckte. Die Höhe des Gitterrostes (Parallele zur Kante c) betrug 10 cm. Im übrigen wies der Gitterrost eine wabenförmige Struktur auf. Die einzelne Wabe wies einen quadratischen Querschnitt auf, mit einer Innenfläche von 3 cm x 3 cm. Die Wanddicken des Gitterrostes lagen bei 1 mm.

Unterhalb der Zudosierungskästen befand sich eine Zudosierungsleitung (50), die parallel zur Kante a verlief (der Abstand zur nächstliegenden Kante a betrug 10 cm) und durch eine entsprechende Öffnung der Wand b x c eingeführt und in selbige gasdicht eingeschweißt war. Die Zudosierungsleitung wies einen quadratischen Querschnitt von 10 cm x 10 cm auf. Ihre Länge erstreckte sich bis zum Ende der Kante a* des dritten Zudosierungskastens.

Unterhalb des jeweiligen Verteilungskastens wies die Zudosierungsleitung eine kreisrunde Öffnung von 8 cm Durchmesser auf, die mit einer entsprechenden (zentriert angebrachten) kreisrunden Öffnung in der der Grundfläche a x b zugewandten Fläche a* x b* auf Deckung stand und an diese angeschweißt war. Die Länge der Zudosierungsleitung betrug 800 m.

In diese Zudosierungsleitung wurden 40 m$^3$/h einer Mischung (simulierte das Eingangsgas II) aus 10 Vol.-% $CO_2$ und 90 Vol.% Luft zugeführt (T = 25°C, P = 1,1 bar).

Im Abstand von 10 cm war unterhalb der Zudosierungsleitung ein Lochblech (51) in die quaderförmige Reaktornachahmung eingeschweißt, die sich über die gesamte Querschnittsfläche a x b erstreckte. Die Löcher des Lochblechs (3 mm Blechdicke) hatten einen Durchmesser von 1 cm. Die Lochmittelpunkte waren gemäß einer quadratischen Teilung gleichförmig verteilt. Das Öffnungsverhältnis des Lochblechs betrug 6 %.

Unterhalb des vorgenannten Lochblechs führte ein kreisrunder Stutzen (52) (300 mm Innendurchmesser) in die quaderförmige Reaktornachahmung. Sein Abstand zur Grundfläche a x b betrug 5 cm. Sein Abstand zur nächstliegenden Fläche a x c betrug 10 cm.

Die Fläche b x c, durch die der Stutzen eingeführt war, lag der Fläche b x c gegenüber, durch die die Zudosierungsleitung eingeführt war (verlängerte man die Zudosierungsleitung und den Stutzen gedanklich in die jeweilige Strömungsrichtung, so lagen die Verlängerungslinien im wesentlichen übereinander.

Durch den Stutzen wurden 2500 m$^3$/h Luft (T = 25°C. P = 1,1 bar) in die quaderförmige Reaktornachahmung geführt (simulierte den Eingangsgasstrom I).

Mit Hilfe eines Ultramat 22P der Fa. Siemens (saugt Gasgemisch an und führt es durch eine Küvette, von deren Inhalt im Abgleich mit dem Verhalten des Inhalts einer Vergleichsküvette der $CO_2$-Gehalt UV-spektroskopisch ermittelt wird) wurde in den einzelnen quadratischen Waben des wabenförmig aufgebauten Gitterrostes der $CO_2$-Gehalt bestimmt.

Die rechnerisch mittlere $CO_2$-Konzentration betrug 0,16 Vol.-%.

Die maximale gemessene $CO_2$-Konzentration betrug 0,30 Vol.-%.

Die minimale gemessene $CO_2$-Konzentration betrug 0,02 Vol.%.

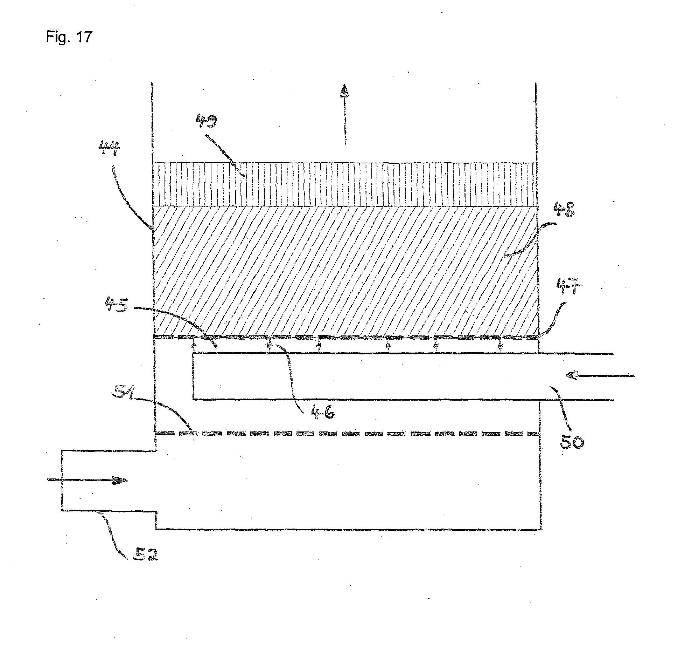

Einen Längsschnitt der quaderförmigen Reaktornachahmung zeigt Figur 17.

## Patentansprüche

1. Verfahren einer heterogen katalysierten partiellen Dehydrierung wenigstens eines zu dehydrierenden Kohlenwasserstoffs zu wenigstens einem dehydrierten Kohlenwasserstoff, bei dem man zum Zweck der heterogen katalysierten partiellen Dehydrierung des wenigstens einen zu dehydrierenden Kohlenwasserstoffs die Gesamtmenge eines molekularen Sauerstoff, molekularen Wasserstoff und den wenigstens einen zu dehydrierenden Kohlenwasserstoff enthaltenden Reaktionsgasgemischeingangsstroms mit der Maßgabe durch ein in einem Schacht mit vorgegebenem Querschnitt befindliches Katalysatorfestbett, das in Strömungsrichtung des Reaktionsgasgemischeingangsstroms zunächst eine Schüttung aus inerten Formkörpern und eine in Strömungsrichtung des Reaktionsgasgemischeingangsstroms sich an diese anschließende katalytisch aktive Schüttung mit wenigstens einem Katalysatorformkörper umfasst, die so beschaffen ist, dass sie im Reaktionsgasgemischeingangsstrom in Strömungsrichtung desselben wenigstens in ihrem Eingangsbereich für die Verbrennungsreaktion von molekularem Wasserstoff mit molekularem Sauerstoff zu Wasser und/oder für die Verbrennungsreaktion von im Reaktionsgasgemischeingangsstrom enthaltenem Kohlenwasserstoff mit molekularem Sauerstoff zu Kohlenoxiden und Wasser eine geringere Aktivierungsenergie bedingt, als für die Dehydrierung des wenigstens einen zu dehydrierenden Kohlenwasserstoffs zu dem wenigstens einen dehydrierten Kohlenwasserstoff, führt, dass eine Teilmenge des wenigstens einen zu dehydrierenden Kohlenwasserstoffs zu dem wenigstens einen dehydrierten Kohlenwasserstoff dehydriert wird, und dabei den Reaktionsgasgemischeingangsstrom im Schacht dadurch erzeugt, dass man einem im Schacht mit einem Volumenstrom V1 auf das Katalysatorfestbett zuströmenden, molekularen Wasserstoff und den wenigstens einen zu dehydrierenden Kohlenwasserstoff enthaltenden Eingangsgasstrom I vor dem Katalysatorfestbett ein molekularen Sauerstoff enthaltendes Eingangsgas II mit einem Gesamtvolumenstrom V2 zudosiert, **dadurch gekennzeichnet, dass** das Eingangsgas II in Form von aus einer Vielzahl von in Strömungsrichtung des Eingangsgasstroms I vor dem Katalysatorfestbett befindlichen Austrittsöffnungen A eines Leitungssystems ausströmenden Eingangsgas II-strömen so zudosiert wird, dass

   a) die Richtungen der Mehrzahl M aller aus den Austrittsöffnungen A in fiktiver Abwesenheit des Eingangsgasstroms I austretenden Eingangsgas II-ströme mit der Strömungsrichtung des Eingangsgasstroms I einen Winkel $\alpha$ von $90\pm60°$ einschließen;
   b) die Entfernung D der Mehrzahl M aller Austrittsöffnungen A vom Katalysatorfestbett, bezogen auf die Strömungsgeschwindigkeit W des Eingangsgasstroms I im Schacht, kleiner oder gleich wie die Induktionszeit J des Reaktionsgaseingangsgemischs multipliziert mit $2\cdot W$ ist;
   c) bei einer Projektion der Schwerpunkte der Mehrzahl M aller Austrittsöffnungen A in Strömungsrichtung des Eingangsgasstroms I in die Projektionsebene E senkrecht zur Strömungsrichtung des Eingangsgasstroms I innerhalb der Projektionsebene E für wenigstens 75 % der vom Eingangsgasstrom I erfassten Projektionsfläche die Anzahl ZA der in einem beliebigen $m^2$ befindlichen Austrittsöffnungsschwerpunkte $\geq 10$ beträgt;
   d) die aus den zu der Anzahl ZA von Austrittsöffnungsschwerpunkten gehörigen Austrittsöffnungen A austretenden individuellen Eingangsgas II-ströme um nicht mehr als 50 % von ihrem Zahlenmittelwert abweichen;
   e) innerhalb der Anzahl ZA von Austrittsöffnungen der Abstand d von einem Austrittsöffnungsschwerpunkt <u>zum nächstliegenden</u> Austrittsöffnungsschwerpunkt nicht mehr als $2\sqrt{1m^2/ZA}$ beträgt, und
   f) das Verhältnis V1 : V2 $\geq 8$ beträgt.

2. Verfahren nach Anspruch 1, **dadurch gekennzeichnet, dass** das Eingangsgas II folgende Gehalte aufweist:

   0 bis 80 Vol.-% Wasserdampf,
   10 bis 97 Vol.-% $N_2$, und
   3 bis 25 Vol.-% $O_2$.

3. Verfahren nach Anspruch 1 oder 2, **dadurch gekennzeichnet, dass** das Eingangsgas II folgende Gehalte aufweist:

15 bis 80 Vol.-% $H_2O$,
20 bis 85 Vol.-% $N_2$, und
5 bis 25 Vol.-% $O_2$.

**4.** Verfahren nach einem der Ansprüche 1 bis 3, **dadurch gekennzeichnet, dass** das Verhältnis V1 : V2 $\geq$ 15 beträgt.

**5.** Verfahren nach einem der Ansprüche 1 bis 4, **dadurch gekennzeichnet, dass** das Verhältnis V1 : V2 $\geq$ 20 beträgt.

**6.** Verfahren nach einem der Ansprüche 1 bis 5, **dadurch gekennzeichnet, dass** die Längstausdehnung L einer Austrittsöffnung A $\geq$ 0,1 mm bis $\leq$ 5 cm beträgt.

**7.** Verfahren nach einem der Ansprüche 1 bis 6, **dadurch gekennzeichnet, dass** die Längstausdehnung L einer Austrittsöffnung A $\geq$ 1 mm bis $\leq$ 5 mm beträgt.

**8.** Verfahren nach einem der Ansprüche 1 bis 7, **dadurch gekennzeichnet, dass** die Richtungen der Mehrzahl M aller aus den Austrittsöffnungen A in fiktiver Abwesenheit des Eingangsgasstroms I austretenden Eingangsgas II-ströme mit der Strömungsrichtung des Eingangsgasstroms I einen Winkel $\alpha$ von $90\pm30°$ einschließen.

**9.** Verfahren nach einem der Ansprüche 1 bis 8, **dadurch gekennzeichnet, dass** die Richtungen der Mehrzahl M aller aus den Austrittsöffnungen A in fiktiver Abwesenheit des Eingangsgasstroms I austretenden Eingangsgas II-ströme mit der Strömungsrichtung des Eingangsgasstroms I einen Winkel $\alpha$ von $90\pm10°$ einschließen.

**10.** Verfahren nach einem der Ansprüche 1 bis 9, **dadurch gekennzeichnet, dass** die Mehrzahl M aller Austrittsöffnungen A die Bedingung D $\leq$ 0,5·W·J erfüllt.

**11.** Verfahren nach einem der Ansprüche 1 bis 10, **dadurch gekennzeichnet, dass** die Mehrzahl M aller Austrittsöffnungen A die Bedingung D $\leq$ 0,2·W·J erfüllt.

**12.** Verfahren nach einem der Ansprüche 1 bis 11, **dadurch gekennzeichnet, dass** ZA $\geq$ 30 beträgt.

**13.** Verfahren nach einem der Ansprüche 1 ·bis 12, **dadurch gekennzeichnet, dass** ZA $\geq$ 50 beträgt.

**14.** Verfahren nach einem der Ansprüche 1 bis 13, **dadurch gekennzeichnet, dass** ZA $\geq$ 100 beträgt.

**15.** Verfahren nach einem der Ansprüche 1 bis 14, **dadurch gekennzeichnet, dass** der Abstand d nicht mehr als $1,5\sqrt{1m^2 / ZA}$ beträgt.

**16.** Verfahren nach einem der Ansprüche 1 bis 15, **dadurch gekennzeichnet, dass** der Abstand d nicht mehr als $\sqrt{1m^2 / ZA}$ beträgt.

**17.** Verfahren nach einem der Ansprüche 1 bis 16, **dadurch gekennzeichnet, dass** die aus den zu der Anzahl ZA von Austrittsöffnungsschwerpunkten gehörigen Austrittsöffnungen A austretenden individuellen Eingangsgas II-ströme um nicht mehr als 30 % von ihrem Zahlenmittelwert abweichen.

**18.** Verfahren nach einem der Ansprüche 1 bis 17, **dadurch gekennzeichnet, dass** die aus den zu der Anzahl ZA von Austrittsöffnungsschwerpunkten gehörigen Austrittsöffnungen A austretenden individuellen Eingangsgas II-ströme um nicht mehr als 10 % von ihrem Zahlenmittelwert abweichen.

**19.** Verfahren nach einem der Ansprüche 1 bis 18, **dadurch gekennzeichnet, dass** unter der Mehrzahl M aller Austrittsöffnungen A und der aus diesen austretenden individuellen Eingangsgas II-ströme diejenigen Austrittsöffnungen A und die aus ihnen austretenden individuellen Eingangsgas II-ströme verstanden werden, aus denen insgesamt gerade mehr als 70 % des Gesamtvolumenstroms V2 mit der Maßgabe austreten, dass die aus ihnen austretenden individuellen Eingangsgas II-ströme innerhalb der Gesamtmenge aller individuellen Eingangsgas II-ströme keinen umfassen, der kleiner ist, als der größte der nicht zu dieser Mehrzahl M gehörigen individuellen Eingangsgas II-ströme.

**20.** Verfahren nach einem der Ansprüche 1 bis 19, **dadurch gekennzeichnet, dass** unter der Mehrzahl M aller Austrittsöffnungen A und der aus diesen austretenden individuellen Eingangsgas II-ströme diejenigen Austrittsöffnungen A und die aus ihnen austretenden individuellen Eingangsgas II-ströme verstanden werden, aus denen insgesamt gerade mehr als 90 % des Gesamtvolumenstroms V2 mit der Maßgabe austreten, dass die aus ihnen austretenden individuellen Eingangsgas II-ströme innerhalb der Gesamtmenge aller individuellen Eingangsgas II-ströme keinen umfassen, der kleiner ist, als der größte der nicht zu dieser Mehrzahl M gehörigen individuellen Eingangsgas II-ströme.

**21.** Verfahren nach einem der Ansprüche 1 bis 20, **dadurch gekennzeichnet, dass** bei einer Projektion der Schwerpunkte M aller Austrittsöffnungen A in Strömungsrichtung des Eingangsgasstroms I in die Projektionsebene E senkrecht zur Strömungsrichtung des Eingangsgastroms I innerhalb der Projektionsebene E für wenigstens 85 % der vom Eingangsgasstrom I erfassten Projektionsfläche die Anzahl ZA, der in einem beliebigen $m^2$ befindlichen Austrittsöffnungsschwerpunkte $\geq$ 10 beträgt.

**22.** Verfahren nach einem der Ansprüche 1 bis 21, **dadurch gekennzeichnet, dass** bei einer Projektion der Schwerpunkte M aller Austrittsöffnungen A in Strömungsrichtung des Eingangsgasstroms I in die Projektionsebene E senkrecht zur Strömungsrichtung des Eingangsgastroms I innerhalb der Projektionsebene E für wenigstens 95 % der vom Eingangsgasstrom I erfassten Projektionsfläche die Anzahl ZA, der in einem beliebigen $m^2$ befindlichen Austrittsöffnungsschwerpunkte $\geq$ 10 beträgt.

**23.** Verfahren nach einem der Ansprüche 1 bis 22, **dadurch gekennzeichnet, dass** der Reaktionsgasgemischeingangsstrom mit der Maßgabe durch das Katalysatorfestbett geführt wird, dass wenigstens 2 mol-% des in ihm enthaltenen wenigstens einen zu dehydrierenden Kohlenwasserstoffs zu dem wenigstens einen dehydrierten Kohlenwasserstoff dehydriert werden.

**24.** Verfahren nach einem der Ansprüche 1 bis 23, **dadurch gekennzeichnet, dass** der Reaktionsgasgemischeingangsstrom mit der Maßgabe durch das Katalysatorfestbett geführt wird, dass wenigstens 5 mol-% des in ihm enthaltenen wenigstens einen zu dehydrierenden Kohlenwasserstoffs zu dem wenigstens einen dehydrierten Kohlenwasserstoff dehydriert werden.

**25.** Verfahren nach einem der Ansprüche 1 bis 24, **dadurch gekennzeichnet, dass** der Reaktionsgasgemischeingangsstrom wenigstens 5 Vol.-% des wenigstens einen zu dehydrierenden Kohlenwasserstoffs enthält.

**26.** Verfahren nach einem der Ansprüche 1 bis 25, **dadurch gekennzeichnet, dass** der Reaktionsgasgemischeingangsstrom wenigstens 10 Vol.-% des wenigstens einen zu dehydrierenden Kohlenwasserstoffs enthält.

**27.** Verfahren nach einem der Ansprüche 1 bis 26, **dadurch gekennzeichnet, dass** der molare Gehalt des Reaktionsgasgemischeingangsstroms an molekularem Sauerstoff nicht mehr als 50 mol-% der darin enthaltenen molaren Menge an molekularem Wasserstoff beträgt.

**28.** Verfahren nach einem der Ansprüche 1 bis 27, **dadurch gekennzeichnet, dass** beim Durchgang des Reaktionsgasgemischeingangsstroms durch das Katalysatorfestbett wenigstens 95 mol-% der Gesamtmenge des im Reaktionsgasgemischeingangsstrom enthaltenen molekularen Sauerstoffs zur Verbrennung des im Reaktionsgasgemischeingangsstrom enthaltenen molekularen Wasserstoffs verbraucht werden.

**29.** Verfahren nach einem der Ansprüche 1 bis 28, **dadurch gekennzeichnet, dass** die Temperatur des Reaktionsgasgemischeingangsstroms beim Eintritt in das Katalysatorfestbett 300 bis 700°C beträgt.

**30.** Verfahren nach einem der Ansprüche 1 bis 29, **dadurch gekennzeichnet, dass** die Belastung des Katalysatorfestbetts, bezogen auf die darin enthaltene Katalysatorgesamtmenge, mit dem wenigstens einen zu dehydrierenden Kohlenwasserstoff 100 bis 10000 Nl/l·h beträgt.

**31.** Verfahren nach einem der Ansprüche 1 bis 30, **dadurch gekennzeichnet, dass** die Induktionszeit J $\leq$ 2000 ms beträgt.

**32.** Verfahren nach einem der Ansprüche 1 bis 31, **dadurch gekennzeichnet, dass** die Induktionszeit J $\leq$ 100 ms beträgt.

**33.** Verfahren nach einem der Ansprüche 1 bis 32, **dadurch gekennzeichnet, dass** der Querschnitt der das Eingangsgas II führenden Leitungen dort, wo sich die Austrittsöffnungen A befinden, vieleckig ist.

34. Verfahren nach einem der Ansprüche 1 bis 33, **dadurch gekennzeichnet, dass** der Querschnitt der das Eingangsgas II führenden Leitungen dort, wo sich die Austrittsöffnungen A befinden, viereckig ist.

35. Verfahren nach einem der Ansprüche 1 bis 34, **dadurch gekennzeichnet, dass** die Austrittsöffnungen A rund sind.

36. Verfahren nach einem der Ansprüche 1 bis 35, **dadurch gekennzeichnet, dass** der Temperaturunterschied $\Delta T_{II}^I$ zwischen der Temperatur des Eingangsgasstroms I und der Temperatur des Eingangsgases II nicht größer als 300 °C ist.

37. Verfahren nach einem der Ansprüche 1 bis 36, **dadurch gekennzeichnet, dass** der wenigstens eine zu dehydrierende Kohlenwasserstoff Propan ist.

38. Verfahren nach einem der Ansprüche 1 bis 37, **dadurch gekennzeichnet, dass** der wenigstens eine dehydrierte Kohlenwasserstoff Propylen ist.

39. Verfahren nach einem der Ansprüche 1 bis 38, **dadurch gekennzeichnet, dass** es in einem als Hordenreaktor ausgestalteten Schachtreaktor durchgeführt wird.

40. Verfahren nach einem der Ansprüche 1 bis 39, **dadurch gekennzeichnet, dass** dem Verfahren der heterogen katalysierten partiellen Dehydrierung des wenigstens einen zu dehydrierenden Kohlenwasserstoffs ein Verfahren der heterogen katalysierten partiellen Oxidation des wenigstens einen dehydrierten Kohlenwasserstoffs nachgeschaltet ist.

41. Verfahren nach Anspruch 40, **dadurch gekennzeichnet, dass** das nachgeschaltete Verfahren der heterogen katalysierten partiellen Oxidation des wenigstens einen dehydrierten Kohlenwasserstoffs ein Verfahren der heterogen katalysierten partiellen Oxidation von Propylen zu Acrolein und/oder Acrylsäure ist.

42. Verfahren nach Anspruch 41, **dadurch gekennzeichnet, dass** die Abtrennung der Acrylsäure aus dem Produktgasgemisch der heterogen katalysierten partiellen Oxidation so erfolgt, dass man das zuvor gegebenenfalls durch direkte und/oder indirekte Kühlung abgekühlte Produktgasgemisch in einer trennwirksame Einbauten enthaltenden Kolonne unter Seitenabzug einer rohen Acrylsäure fraktionierend kondensiert und/oder einer Absorption mit Wasser, bzw. wässriger Lösung unterwirft.

43. Verfahren nach Anspruch 42, **dadurch gekennzeichnet, dass** sich ein Verfahren der Suspensionskristallisation der rohen Acrylsäure anschließt.

44. Verfahren nach Anspruch 43, **dadurch gekennzeichnet, dass** sich ein Verfahren des Waschens des gebildeten Acrylsäuresuspensionskristallisats in einer Waschkolonne anschließt.

45. Verfahren nach Anspruch 44, **dadurch gekennzeichnet, dass** sich ein Verfahren anschließt, bei dem gewaschenes Acrylsäuresuspensionskristallisat aufgeschmolzen und in Polymerisate einpolymerisiert wird.

**Claims**

1. A process for heterogeneously catalyzed partial dehydrogenation of at least one hydrocarbon to be dehydrogenated to at least one dehydrogenated hydrocarbon, in which, for the purpose of the heterogeneously catalyzed partial dehydrogenation of the at least one hydrocarbon to be dehydrogenated, the entirety of a reaction gas mixture input stream comprising molecular oxygen, molecular hydrogen and the at least one hydrocarbon to be dehydrogenated is conducted through a fixed catalyst bed which is disposed in a shaft with predefined cross section and, in flow direction of the reaction gas mixture input stream, comprises first a bed of inert shaped bodies and a catalytically active bed which follows it in flow direction of the reaction gas mixture input stream and comprises at least one shaped catalyst body which is such that it, in the reaction gas mixture input stream in flow direction thereof, at least in its entrance region, causes a lower activation energy for the combustion reaction of molecular hydrogen with molecular oxygen to water and/or for the combustion reaction of hydrocarbon present in the reaction gas mixture input stream with molecular oxygen to give carbon oxides and water than for the dehydrogenation of the at least one hydrocarbon to be dehydrogenated to the at least one dehydrogenated hydrocarbon, with the proviso that a portion of the at least one hydrocarbon to be dehydrogenated is dehydrogenated to the at least one dehydrogenated hydrocarbon, and the reaction gas mixture input stream is obtained in the shaft by metering an input gas II comprising molecular oxygen with a total volume flow rate V2 upstream of the fixed catalyst bed to an input gas stream I which comprises molecular hydrogen and the at least one hydrocarbon to be dehydrogenated and is flowing within the shaft toward the fixed catalyst bed with a volume flow rate V1, wherein the input gas II is metered in in the form of input gas II streams flowing out of a plurality of exit orifices A of a line system disposed upstream of the fixed catalyst bed in flow direction of the input gas stream I such that

    a) the directions of the plurality M of all input gas II streams exiting from the exit orifices A in the theoretical absence of the input gas stream I enclose an angle $\alpha$ of $90 \pm 60°$ with the flow direction of the input gas stream I;

    b) the distance D of the plurality M of all exit orifices A from the fixed catalyst bed, based on the flow rate W of the input gas stream I in the shaft, is less than or equal to the induction time J of the reaction gas input mixture multiplied by $2 \cdot W$;

    c) a projection of the centers of the plurality M of all exit orifices A in flow direction of the input gas stream I into the projection plane E at right angles to the flow direction of the input gas stream I within the projection plane E gives rise to a number ZA of the exit orifice centers present in any $m^2$ of $\geq 10$ for at least 75% of the projection area covered by the input gas stream I;

    d) the individual input gas II streams exiting from the exit orifices A corresponding to the number ZA of exit orifice centers deviate from their numerical mean by not more than 50%;

    e) among the number ZA of exit orifices, the distance d from one exit orifice center to the closest exit orifice center is not more than $2\sqrt{1m^2 / ZA}$, and

    f) the V1:V2 ratio is $\geq 8$.

2. The process according to claim 1, wherein the input gas II has the following contents:

    from 0 to 80% by volume of steam,
    from 10 to 97% by volume of $N_2$ and
    from 3 to 25% by volume of $O_2$.

3. The process according to claim 1 or 2, wherein the input gas II has the following contents:

    from 15 to 80% by volume of $H_2O$,
    from 20 to 85% by volume of $N_2$ and
    from 5 to 25% by volume of $O_2$.

4. The process according to any of claims 1 to 3, wherein the V1:V2 ratio is $\geq 15$.

5. The process according to any of claims 1 to 4, wherein the V1:V2 ratio is $> 20$.

6. The process according to any of claims 1 to 5, wherein the longest dimension L of one exit orifice A is from $\geq 0.1$ mm to $\leq 5$ cm.

7. The process according to any of claims 1 to 6, wherein the longest dimension L of one exit orifice A is from $\geq 1$ mm to $\leq 5$ mm.

8. The process according to any of claims 1 to 7, wherein the directions of the plurality M of all input gas II streams exiting from the exit orifices A in the theoretical absence of the input gas stream I enclose an angle $\alpha$ of $90 \pm 30°$ with the flow direction of the input gas stream I.

9. The process according to any of claims 1 to 8, wherein the directions of the plurality M of all input gas II streams exiting from the exit orifices A in the theoretical absence of the input gas stream I enclose an angle $\alpha$ of $90 \pm 10°$ with the flow direction of the input gas stream I.

10. The process according to any of claims 1 to 9, wherein the plurality M of all exit orifices A fulfills the condition $D \leq 0.5 \cdot W \cdot J$.

11. The process according to any of claims 1 to 10, wherein the plurality M of all exit orifices A fulfills the condition D ≤ 0.2·W·J.

12. The process according to any of claims 1 to 11, wherein ZA ≥ 30.

13. The process according to any of claims 1 to 12, wherein ZA ≥ 50.

14. The process according to any of claims 1 to 13, wherein ZA ≥ 100.

15. The process according to any of claims 1 to 14, wherein the distance d is not more than $1.5\sqrt{1m^2/ZA}$ .

16. The process according to any of claims 1 to 15, wherein the distance d is not more than $\sqrt{1m^2/ZA}$ .

17. The process according to any of claims 1 to 16, wherein the individual input gas II streams exiting from the exit orifices A corresponding to the number ZA of exit orifice centers deviate from their numerical mean by not more than 30%.

18. The process according to any of claims 1 to 17, wherein the individual input gas II streams exiting from the exit orifices A corresponding to the number ZA of exit orifice centers deviate from their numerical mean by not more than 10%.

19. The process according to any of claims 1 to 18, wherein the plurality M of all exit orifices A and of the individual input gas II streams exiting from them is understood to mean those exit orifices A and the individual input gas II streams exiting from them from which a total of just more than 70% of the total volume flow rate V2 exit, with the proviso that the individual input gas II streams exiting from them, among the entirety of all individual input gas II streams, comprise none which is smaller than the largest of the individual input gas II streams included in this plurality M.

20. The process according to any of claims 1 to 19, wherein the plurality M of all exit orifices A and of the individual input gas II streams exiting from them is understood to mean those exit orifices A and the individual input gas II streams exiting from them from which a total of just more than 90% of the total volume flow rate V2 exit, with the proviso that the individual input gas II streams exiting from them, among the entirety of all individual input gas II streams, comprise none which is smaller than the largest of the individual input gas II streams included in this plurality M.

21. The process according to any of claims 1 to 20, wherein a projection of the centers M of all exit orifices A in flow direction of the input gas stream I into the projection plane E at right angles to the flow direction of the input gas stream I within the projection plane E gives rise to a number ZA of the exit orifice centers present in any m$^2$ of ≥ 10 for at least 85% of the projection area covered by the input gas stream I.

22. The process according to any of claims 1 to 21, wherein a projection of the centers M of all exit orifices A in flow direction of the input gas stream I into the projection plane E at right angles to the flow direction of the input gas stream I within the projection plane E gives rise to a number ZA of the exit orifice centers present in any m$^2$ of ≥ 10 for at least 95% of the projection area covered by the input gas stream I.

23. The process according to any of claims 1 to 22, wherein the reaction gas mixture input stream is conducted through the fixed catalyst bed with the proviso that at least 2 mol% of the at least one hydrocarbon to be dehydrogenated present therein is dehydrogenated to the at least one dehydrogenated hydrocarbon.

24. The process according to any of claims 1 to 23, wherein the reaction gas mixture input stream is conducted through the fixed catalyst bed with the proviso that at least 5 mol% of the at least one hydrocarbon to be dehydrogenated present therein is dehydrogenated to the at least one dehydrogenated hydrocarbon.

25. The process according to any of claims 1 to 24, wherein the reaction gas mixture input stream comprises at least 5% by volume of the at least one hydrocarbon to be dehydrogenated.

26. The process according to any of claims 1 to 25, wherein the reaction gas mixture input stream comprises at least 10% by volume of the at least one hydrocarbon to be dehydrogenated.

27. The process according to any of claims 1 to 26, wherein the molar content in the reaction gas mixture input stream of molecular oxygen is not more than 50 mol% of the molar amount of molecular hydrogen present therein.

28. The process according to any of claims 1 to 27, wherein, as the reaction gas mixture input stream passes through the fixed catalyst bed, at least 95 mol% of the total amount of molecular oxygen present in the reaction gas mixture input stream is consumed for the combustion of the molecular hydrogen present in the reaction gas mixture input stream.

29. The process according to any of claims 1 to 28, wherein the temperature of the reaction gas mixture input stream on entry into the fixed catalyst bed is from 300 to 700°C.

30. The process according to any of claims 1 to 29, wherein the loading on the fixed catalyst bed, based on the total amount of catalyst present therein, with the at least one hydrocarbon to be dehydrogenated is from 100 to 10 000 1 (STP)/1·h.

31. The process according to any of claims 1 to 30, wherein the induction time J is $\leq$ 2000 ms.

32. The process according to any of claims 1 to 31, wherein the induction time J is $\leq$ 100 ms.

33. The process according to any of claims 1 to 32, wherein the cross section of the lines conducting the input gas II is polygonal where the exit orifices A are disposed.

34. The process according to any of claims 1 to 33, wherein the cross section of the lines conducting the input gas II is tetragonal where the exit orifices A are disposed.

35. The process according to any of claims 1 to 34, wherein the exit orifices A are round.

36. The process according to any of claims 1 to 35, wherein the temperature difference $\Delta T_{II}{}^{I}$ between the temperature of the input gas stream I and the temperature of the input gas II is not greater than 300°C.

37. The process according to any of claims 1 to 36, wherein the at least one hydrocarbon to be dehydrogenated is propane.

38. The process according to any of claims 1 to 37, wherein the at least one dehydrogenated hydrocarbon is propylene.

39. The process according to any of claims 1 to 38, which is performed in a shaft reactor configured as a tray reactor.

40. The process according to any of claims 1 to 39, wherein the process for heterogeneously catalyzed partial dehydrogenation of the at least one hydrocarbon to be dehydrogenated is followed by a process for heterogeneously catalyzed partial oxidation of the at least one dehydrogenated hydrocarbon.

41. The process according to claim 40, wherein the downstream process for heterogeneously catalyzed partial oxidation of the at least one dehydrogenated hydrocarbon is a process for heterogeneously catalyzed partial oxidation of propylene to acrolein and/or acrylic acid.

42. The process according to claim 41, wherein the acrylic acid is removed from the product gas mixture of the heterogeneously catalyzed partial oxidation in such a way that the product gas mixture which may optionally have been cooled beforehand by direct and/or indirect cooling is fractionally condensed in a column comprising separating internals with side draw removal of crude acrylic acid and/or subjected to an absorption with water or aqueous solution.

43. The process according to claim 42, which is followed by a process for suspension crystallization of the crude acrylic acid.

44. The process according to claim 43, which is followed by a process for washing the acrylic acid suspension crystals formed in a wash column.

45. The process according to claim 44, which is followed by a process in which washed acrylic acid suspension crystals are melted and polymerized to polymers.

**Revendications**

1. Procédé de déshydrogénation partielle sous catalyse hétérogène d'au moins un hydrocarbure à déshydrogéner en au moins un hydrocarbure déshydrogéné, selon lequel, dans le but de la déshydrogénation partielle sous catalyse hétérogène du ou des hydrocarbures à déshydrogéner, la totalité d'un courant d'entrée de mélange réactionnel gazeux contenant de l'oxygène moléculaire, de l'hydrogène moléculaire et le ou les hydrocarbures à déshydrogéner est passée au travers d'un lit catalytique fixe se trouvant dans un puits de section prédéterminée, qui comprend dans la direction d'écoulement du courant d'entrée de mélange réactionnel gazeux tout d'abord un garnissage de corps moulés inertes et un garnissage catalytiquement actif comprenant au moins un corps moulé catalytique suivant celui-ci dans la direction d'écoulement du courant d'entrée de mélange réactionnel gazeux, qui est conçu de manière à ce qu'il cause dans le courant d'entrée de mélange réactionnel gazeux, au moins dans sa zone d'entrée dans la direction d'écoulement de celui-ci, une énergie d'activation plus faible pour la réaction de combustion d'hydrogène moléculaire avec de l'oxygène moléculaire en eau et/ou pour la réaction de combustion de l'hydrocarbure contenu dans le courant d'entrée de mélange réactionnel gazeux avec de l'oxygène moléculaire en oxydes de carbone et en eau, que pour la déshydrogénation du ou des hydrocarbures à déshydrogéner en le ou les hydrocarbures déshydrogénés, à condition qu'une partie du ou des hydrocarbures à déshydrogéner soit déshydrogénée en le ou les hydrocarbures déshydrogénés, et le courant d'entrée de mélange réactionnel gazeux est formé dans le puits par ajout d'un gaz d'entrée II contenant de l'oxygène moléculaire en un débit volumique total V2 avant le lit catalytique fixe à un courant gazeux d'entrée I contenant de l'hydrogène moléculaire et le ou les hydrocarbures à déshydrogéner, s'écoulant dans le puits à un débit volumique V1 sur le lit catalytique fixe, **caractérisé en ce que** le gaz d'entrée II est ajouté sous la forme de courants de gaz d'entrée II sortant d'une pluralité d'ouvertures de sortie A d'un système de conduite se trouvant avant le lit catalytique fixe dans la direction d'écoulement du courant gazeux d'entrée I, de manière à ce que :

a) les directions de la pluralité M de tous les courants de gaz d'entrée II sortant des ouvertures de sortie A en l'absence fictive du courant gazeux d'entrée I forment avec la direction d'écoulement du courant gazeux d'entrée I un angle $\alpha$ de 90 $\pm$ 60° ;
b) la distance D entre la pluralité M de toutes les ouvertures de sortie A et le lit catalytique fixe, par rapport à la vitesse d'écoulement W du courant gazeux d'entrée I dans le puis, est inférieure ou égale à la période d'induction J du mélange d'entrée réactionnel gazeux multipliée par 2·W ;
c) lors d'une projection des centres de gravité de la pluralité M de toutes les ouvertures de sortie A dans la direction d'écoulement du courant gazeux d'entrée I dans le plan de projection E perpendiculaire à la direction d'écoulement du courant gazeux d'entrée I, dans le plan de projection E, pour au moins 75 % de la surface de projection comprise par le courant gazeux d'entrée I, le nombre ZA des centres de gravité d'ouvertures de sortie se trouvant dans un m$^2$ quelconque est $\geq$ 10 ;
d) les courants de gaz d'entrée II individuels sortant des ouvertures de sortie A appartenant au nombre ZA de centres de gravité d'ouvertures de sortie ne dévient pas de plus de 50 % de leur valeur moyenne en nombre ;
e) dans le nombre ZA d'ouvertures de sortie, la distance d entre un centre de gravité d'ouverture de sortie et le centre de gravité d'ouverture de sortie le plus proche n'est pas supérieure à $2\sqrt{1m^2/ZA}$ , et
f) le rapport V1:V2 est $\geq$ 8.

2. Procédé selon la revendication 1, **caractérisé en ce que** le gaz d'entrée II présente les teneurs suivantes :

0 à 80 % en volume de vapeur d'eau,
10 à 97 % en volume de N$_2$ et
3 à 25 % en volume d'O$_2$.

3. Procédé selon la revendication 1 ou 2, **caractérisé en ce que** le gaz d'entrée II présente les teneurs suivantes :

15 à 80 % en volume d'H$_2$O,
20 à 85 % en volume de N$_2$ et
5 à 25 % en volume d'O$_2$.

4. Procédé selon l'une quelconque des revendications 1 à 3, **caractérisé en ce que** le rapport V1:V2 est $\geq$ 15.

**5.** Procédé selon l'une quelconque des revendications 1 à 4, **caractérisé en ce que** le rapport V1:V2 est $\geq$ 20.

**6.** Procédé selon l'une quelconque des revendications 1 à 5, **caractérisé en ce que** la dimension longitudinale L d'une ouverture de sortie A est de $\geq$ 0,1 mm à $\leq$ 5 cm.

**7.** Procédé selon l'une quelconque des revendications 1 à 6, **caractérisé en ce que** la dimension longitudinale L d'une ouverture de sortie A est de $\geq$ 1 mm à $\leq$ 5 mm.

**8.** Procédé selon l'une quelconque des revendications 1 à 7, **caractérisé en ce que** les directions de la pluralité M de tous les courants de gaz d'entrée II sortant des ouvertures de sortie A en l'absence fictive du courant gazeux d'entrée I forment avec la direction d'écoulement du courant gazeux d'entrée I un angle $\alpha$ de 90 $\pm$ 30°.

**9.** Procédé selon l'une quelconque des revendications 1 à 8, **caractérisé en ce que** les directions de la pluralité M de tous les courants de gaz d'entrée II sortant des ouvertures de sortie A en l'absence fictive du courant gazeux d'entrée I forment avec la direction d'écoulement du courant gazeux d'entrée I un angle $\alpha$ de 90 $\pm$ 10°.

**10.** Procédé selon l'une quelconque des revendications 1 à 9, **caractérisé en ce que** la pluralité M de toutes les ouvertures de sortie A satisfait la condition D $\leq$ 0,5·W·J.

**11.** Procédé selon l'une quelconque des revendications 1 à 10, **caractérisé en ce que** la pluralité M de toutes les ouvertures de sortie A satisfait la condition D $\leq$ 0,2·W·J.

**12.** Procédé selon l'une quelconque des revendications 1 à 11, **caractérisé en ce que** ZA $\geq$ 30.

**13.** Procédé selon l'une quelconque des revendications 1 à 12, **caractérisé en ce que** ZA $\geq$ 50.

**14.** Procédé selon l'une quelconque des revendications 1 à 13, **caractérisé en ce que** ZA $\geq$ 100.

**15.** Procédé selon l'une quelconque des revendications 1 à 14, **caractérisé en ce que** la distance d n'est pas supérieure à $1,5\sqrt{1m^2/ZA}$ .

**16.** Procédé selon l'une quelconque des revendications 1 à 15, **caractérisé en ce que** la distance d n'est pas supérieure à $\sqrt{1m^2/ZA}$ .

**17.** Procédé selon l'une quelconque des revendications 1 à 16, **caractérisé en ce que** les courants de gaz d'entrée II individuels sortant des ouvertures de sortie A appartenant au nombre ZA de centres de gravité d'ouvertures de sortie ne dévient pas de plus de 30 % de leur valeur moyenne en nombre.

**18.** Procédé selon l'une quelconque des revendications 1 à 17, **caractérisé en ce que** les courants de gaz d'entrée II individuels sortant des ouvertures de sortie A appartenant au nombre ZA de centres de gravité d'ouvertures de sortie ne dévient pas de plus de 10 % de leur valeur moyenne en nombre.

**19.** Procédé selon l'une quelconque des revendications 1 à 18, **caractérisé en ce que** la pluralité M de toutes les ouvertures de sortie A et les courants de gaz d'entrée II individuels sortant de celles-ci se rapportent aux ouvertures de sortie A et aux courants de gaz d'entrée II individuels sortant de celles-ci à partir desquelles au total plus de 70 % du débit volumique total V2 sort, à condition que les courants de gaz d'entrée II individuels sortant de celles-ci ne comprennent pas dans la totalité de tous les courants de gaz d'entrée II individuels de courant qui est plus petit que le plus grand des courants de gaz d'entrée II individuels n'appartenant pas à cette pluralité M.

**20.** Procédé selon l'une quelconque des revendications 1 à 19, **caractérisé en ce que** la pluralité M de toutes les ouvertures de sortie A et les courants de gaz d'entrée II individuels sortant de celles-ci se rapportent aux ouvertures de sortie A et aux courants de gaz d'entrée II individuels sortant de celles-ci à partir desquelles au total plus de 90 % du débit volumique total V2 sort, à condition que les courants de gaz d'entrée II individuels sortant de celles-ci ne comprennent pas dans la totalité de tous les courants de gaz d'entrée II individuels de courant qui est plus petit que le plus grand des courants de gaz d'entrée II individuels n'appartenant pas à cette pluralité M.

**21.** Procédé selon l'une quelconque des revendications 1 à 20, **caractérisé en ce que** lors d'une projection des centres de gravité M de toutes les ouvertures de sortie A dans la direction d'écoulement du courant gazeux d'entrée I dans le plan de projection E perpendiculaire à la direction d'écoulement du courant gazeux d'entrée I, dans le plan de projection E, pour au moins 85 % de la surface de projection comprise par le courant gazeux d'entrée I, le nombre ZA des centres de gravité d'ouvertures de sortie se trouvant dans un m$^2$ quelconque est $\geq 10$.

**22.** Procédé selon l'une quelconque des revendications 1 à 21, **caractérisé en ce que** lors d'une projection des centres de gravité M de toutes les ouvertures de sortie A dans la direction d'écoulement du courant gazeux d'entrée I dans le plan de projection E perpendiculaire à la direction d'écoulement du courant gazeux d'entrée I, dans le plan de projection E, pour au moins 95 % de la surface de projection comprise par le courant gazeux d'entrée I, le nombre ZA des centres de gravité d'ouvertures de sortie se trouvant dans un m$^2$ quelconque est $\geq 10$.

**23.** Procédé selon l'une quelconque des revendications 1 à 22, **caractérisé en ce que** le courant d'entrée de mélange réactionnel gazeux est passé au travers du lit catalytique fixe à condition qu'au moins 2 % en moles du ou des hydrocarbures à déshydrogéner contenus dans celui-ci soient déshydrogénés en le ou les hydrocarbures déshydrogénés.

**24.** Procédé selon l'une quelconque des revendications 1 à 23, **caractérisé en ce que** le courant d'entrée de mélange réactionnel gazeux est passé au travers du lit catalytique fixe à condition qu'au moins 5 % en moles du ou des hydrocarbures à déshydrogéner contenus dans celui-ci soient déshydrogénés en le ou les hydrocarbures déshydrogénés.

**25.** Procédé selon l'une quelconque des revendications 1 à 24, **caractérisé en ce que** le courant d'entrée de mélange réactionnel gazeux contient au moins 5 % en volume du ou des hydrocarbures à déshydrogéner.

**26.** Procédé selon l'une quelconque des revendications 1 à 25, **caractérisé en ce que** le courant d'entrée de mélange réactionnel gazeux contient au moins 10 % en volume du ou des hydrocarbures à déshydrogéner.

**27.** Procédé selon l'une quelconque des revendications 1 à 26, **caractérisé en ce que** la teneur molaire du courant d'entrée de mélange réactionnel gazeux en oxygène moléculaire n'est pas supérieure à 50 % en moles de la quantité molaire d'hydrogène moléculaire contenue dans celui-ci.

**28.** Procédé selon l'une quelconque des revendications 1 à 27, **caractérisé en ce que**, lors du passage du courant d'entrée de mélange réactionnel gazeux au travers du lit catalytique fixe, au moins 95 % en moles de la totalité de l'oxygène moléculaire contenu dans le courant d'entrée de mélange réactionnel gazeux est consommé pour la combustion de l'hydrogène moléculaire contenu dans le courant d'entrée de mélange réactionnel gazeux.

**29.** Procédé selon l'une quelconque des revendications 1 à 28, **caractérisé en ce que** la température du courant d'entrée de mélange réactionnel gazeux lors de l'entrée dans le lit catalytique fixe est de 300 à 700 °C.

**30.** Procédé selon l'une quelconque des revendications 1 à 29, **caractérisé en ce que** le chargement du lit catalytique fixe, par rapport à la totalité de catalyseur contenu dans celui-ci, avec le ou les hydrocarbures à déshydrogéner est de 100 à 10 000 Nl/1·h.

**31.** Procédé selon l'une quelconque des revendications 1 à 30, **caractérisé en ce que** la période d'induction J est $\leq 2$ 000 ms.

**32.** Procédé selon l'une quelconque des revendications 1 à 31, **caractérisé en ce que** la période d'induction J est $\leq$ 100 ms.

**33.** Procédé selon l'une quelconque des revendications 1 à 32, **caractérisé en ce que** la section des conduites conduisant le gaz d'entrée II où se trouvent les ouvertures de sortie A est polygonale.

**34.** Procédé selon l'une quelconque des revendications 1 à 33, **caractérisé en ce que** la section des conduites conduisant le gaz d'entrée II où se trouvent les ouvertures de sortie A est tétragonale.

**35.** Procédé selon l'une quelconque des revendications 1 à 34, **caractérisé en ce que** les ouvertures de sortie A sont rondes.

**36.** Procédé selon l'une quelconque des revendications 1 à 35, **caractérisé en ce que** la différence de température $\Delta T_{II}^{\ I}$ entre la température du courant gazeux d'entrée I et la température du gaz d'entrée II n'est pas supérieure à 300 °C.

**37.** Procédé selon l'une quelconque des revendications 1 à 36, **caractérisé en ce que** le ou les hydrocarbures à déshydrogéner sont le propane.

**38.** Procédé selon l'une quelconque des revendications 1 à 37, **caractérisé en ce que** le ou les hydrocarbures déshydrogénés sont le propylène.

**39.** Procédé selon l'une quelconque des revendications 1 à 38, **caractérisé en ce qu'**il est réalisé dans un réacteur à puits configuré sous la forme d'un réacteur à plateaux.

**40.** Procédé selon l'une quelconque des revendications 1 à 39, **caractérisé en ce qu'**un procédé d'oxydation partielle sous catalyse hétérogène du ou des hydrocarbures déshydrogénés se trouve en aval du procédé de déshydrogénation partielle sous catalyse hétérogène du ou des hydrocarbures à déshydrogéner.

**41.** Procédé selon la revendication 40, **caractérisé en ce que** le procédé en aval d'oxydation partielle sous catalyse hétérogène du ou des hydrocarbures déshydrogénés est un procédé d'oxydation partielle sous catalyse hétérogène de propylène en acroléine et/ou acide acrylique.

**42.** Procédé selon la revendication 41, **caractérisé en ce que** la séparation de l'acide acrylique du mélange gazeux de produits de l'oxydation partielle sous catalyse hétérogène a lieu en soumettant le mélange gazeux de produits éventuellement refroidi auparavant par refroidissement direct et/ou indirect à une condensation fractionnée dans une colonne contenant des composants de séparation avec soutirage latéral d'un acide acrylique brut et/ou à une absorption avec de l'eau ou une solution aqueuse.

**43.** Procédé selon la revendication 42, **caractérisé en ce qu'**un procédé de cristallisation en suspension de l'acide acrylique brut s'ensuit.

**44.** Procédé selon la revendication 43, **caractérisé en ce qu'**un procédé de lavage des cristaux en suspension d'acide acrylique formés dans une colonne de lavage s'ensuit.

**45.** Procédé selon la revendication 44, **caractérisé en ce qu'**un procédé selon lequel les cristaux en suspension d'acide acrylique lavés sont fondus et polymérisés dans des polymères s'ensuit.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Figur 18

**EP 2 004 578 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005044916 A
- DE 19937107 A
- US 4788371 A
- US 4886928 A
- US 5430209 A
- US 5530171 A
- US 5527979 A
- US 5563314 A
- DE 102005061626 A
- DE 102005057197 A
- DE 102005052923 A
- DE 102005052917 A
- DE 102005022798 A
- DE 102005009885 A
- DE 102005010111 A
- DE 102004032129 A
- DE 102005013039 A
- WO 03076370 A
- DE 10211275 A
- WO 0196270 A
- US 2584391 A
- DE 102004024957 A
- WO 2004074222 A
- DE 102004003070 A
- EP 799169 A
- EP 731077 A
- DE 10131297 A
- WO 9946039 A
- EP 705136 A
- WO 9929420 A
- US 4220091 A
- US 5430220 A
- US 5877369 A
- EP 117146 A
- DE 19937196 A
- DE 19937105 A
- US 3670044 A
- US 6566573 A
- WO 9429021 A
- DE 102005044916
- DE 102005061626
- WO 2004067164 A
- DE 10219879 A
- DE 102005056377 A
- EP 1388533 A
- EP 1388532 A
- DE 10235847 A

- EP 792867 A
- WO 9801415 A
- EP 1015411 A
- EP 1015410 A
- WO 9950219 A
- WO 0053560 A
- WO 0209839 A
- WO 03041833 A
- DE 10223058 A
- DE 10243625 A
- DE 10336386 A
- EP 854129 A
- US 4317926 A
- DE 19837520 A
- DE 19606877 A
- DE 190501325 A
- DE 10247240 A
- DE 19740253 A
- EP 695736 A
- EP 982287 A
- EP 1041062 A
- DE 4308087 A
- DE 4335172 A
- DE 4436243 A
- DE 19924532 A
- DE 10332758 A
- DE 19924533 A
- EP 982289 A
- DE 10115277 A
- DE 19740252 A
- DE 19627847 A
- EP 920408 A
- EP 1068174 A
- EP 1066239 A
- EP 1066240 A
- WO 0053561 A
- DE 10053086 A
- EP 982288 A
- WO 0196271 A
- WO 2004063138 A
- WO 2004035514 A
- WO 0177056 A
- WO 03041832 A
- WO 02055469 A
- WO 03078378 A
- DE 10245585 A
- DE 10246119 A